# EUROPEAN PATENT APPLICATION

(11) **EP 4 685 276 A1**
(43) Date of publication of application: **28.01.2026**
(21) Application number: 24774983.1
(22) Date of filing: 22.03.2024
(51) Int. Cl.: C40B 40/06, A61K 31/7115, A61P 9/10, A61P 25/00, A61P 29/00, A61P 35/00, A61P 37/02, C12N 15/115, C12Q 1/6869, G01N 33/53, G01N 33/569, G01N 33/574

(54) **METHOD FOR PRODUCING SIX-LETTER DNA APTAMER**

(30) Priority: 23.03.2023 SG 10202300790X
(71) Applicant: Xenolis Pte. Ltd., Singapore 118259 (SG)
(72) Inventor: HIRAO, Michiko, Singapore 118259 (SG); MATSUNAGA, Ken-ichiro, Singapore 118259 (SG); TAN, Hui Pen, Singapore 118259 (SG); HIRAO, Ichiro, Singapore 118259 (SG)
(74) Representative: D Young & Co LLP
(86) International application number: PCT/JP2024/011193
(87) International publication number: WO 2024/195849

(57) **Abstract**

A problem to be addressed by the present invention is to provide a novel method for efficiently generating a nucleic acid aptamer having high affinity to a target substance. Provided is a single-stranded nucleic acid molecule library comprising a plurality of single-stranded nucleic acid molecules, wherein: the plurality of single-stranded nucleic acid molecules comprise a Ds/Pa'-containing single-stranded nucleic acid molecule, the Ds/Pa'-containing single-stranded nucleic acid molecule comprises a pair of primer-binding regions at the 5' end and 3' end thereof, and a variable region between the pair of primer-binding regions, the pair of primer-binding regions consist of base sequences which are common among the plurality of single-stranded nucleic acid molecules, and the variable region comprises an unnatural base Ds (7-(2-thienyl)-3H-imidazo[4,5-b]pyridine-3-yl) shown in the general formula (I) and an unnatural base Pa' shown in the following general formula (II).

## Description

### Technical Field

The present invention relates to a nucleic acid aptamer, a single-stranded nucleic acid molecule library, a method of producing a single-stranded nucleic acid molecule library, a method of producing a nucleic acid aptamer, a method of sequencing the base sequence of a single-stranded nucleic acid molecule containing an unnatural base, and the like.

### Background Art

A nucleic acid aptamer is a nucleic acid strand that binds specifically to a target, such as a small molecule, a protein, or a cell. A nucleic acid aptamer is usually generated from a nucleic acid library having a random sequence, through repetitions of selection and amplification, using SELEX (systematic evolution of ligands by exponential enrichment).

A nucleic acid aptamer generated using SELEX undergoes base sequencing, and then is chemically synthesized for mass production and modification. The nucleic acid aptamer can be provided at high purity, and thus, can be used as antibody substitute with excellent quality control.

In general, however, a nucleic acid is more hydrophilic than a protein-based antibody. Because of this, the hydrophobic interaction of a nucleic acid aptamer with a target protein can be weaker than the hydrophobic interaction of an antibody. Accordingly, a conventional nucleic acid aptamer has difficulty in providing a stronger binding force than an antibody, and has been considered to be insufficient for practical use.

To solve the above-described problem of a nucleic acid aptamer, the present inventors have developed ExSELEX (genetic alphabet Expansion for SELEX) as a new aptamer-generating method that enables the improvement of the hydrophobic interaction with a target protein by introducing the hydrophobic unnatural base 7-(2-thienyl)imidazo[4,5-b]pyridine (herein referred to as "Ds" or a "Ds base") as a fifth base into a nucleic acid aptamer (Non-Patent Literature 1). The Ds base is base-paired with 2-nitro-4-propynylpyrrole (2-nitro-4-propynylpyrrole and an analog thereof are herein referred to as a "Px" or a "Px base"). Accordingly, a Ds-Px base pair formed by base pairing of a Ds base and a Px base functions with high fidelity as a third base pair in PCR amplification (Non-Patent Literature 1 to 6). ExSELEX has enabled the affinity to a target protein to be improved on the basis of the Ds base in the base sequence constituting a nucleic acid aptamer.

### Citation List

### Patent Literature

Patent Literature 1: WO2007/066737
Patent Literature 2: WO2009/123216
Patent Literature 3: WO2013/073602

### Non-Patent Literature

Non-Patent Literature 1: Kimoto, M., et al., Nat. Biotechnol., 2013, 31: 453-457.
Non-Patent Literature 2: Kimoto, M., et al., Nucleic Acids Res., 2009, 37: e14.
Non-Patent Literature 3: Yamashige, R., et al., Nucleic Acids Res., 2012, 40:2793-2806.
Non-Patent Literature 4: Matsunaga, K., et al., J. Am. Chem. Soc., 2017, 139: 324-334.
Non-Patent Literature 5: Matsunaga, K., et al., Nucleic Acids Res., 2021, 49: 11407-11424.
Non-Patent Literature 6: Kimoto, M. and Hirao, I., Chem. Soc, Rev., 2020, 49: 7602-7626.

### Summary of Invention

### Technical Problem

An object of the present invention is to provide a novel method for efficiently generating a nucleic acid aptamer having high affinity to a target substance.

### Solution to Problem

The above-described ExSELEX has improved the affinity to a target protein on the basis of the Ds base in the base sequence constituting a nucleic acid aptamer. However, ExSELEX still remains low in the success rate of generation of a nucleic acid aptamer having high affinity at a nanomolar or lower level of a *K_{D}* value indicative of the affinity to a target protein.

The past research by the present inventors has discovered an AptD2 aptamer in the form of a six-letter DNA aptamer comprising the Px base in addition to the Ds base in the base sequence constituting a nucleic acid aptamer (WO2020/256639). The Px base in the AptD2 aptamer is a base incorporated as a result of a mutation in a PCR amplification process in ExSELEX.

To solve the above-described problems, the present inventors have investigated the significance of the Px base in the above-described AptD2 aptamer. As a result, the present inventors have discovered that, in the AptD2 aptamer, not only a Ds base but also a Px base is important in binding to a target protein.

On the basis of the above-described results, the present inventors have conceived the idea that using, as a starting material in ExSELEX, a DNA library composed of six kinds of bases consisting of four natural bases, a Ds base, and a Px base makes it possible to more efficiently generate a nucleic acid aptamer having higher affinity than a conventional ExSELEX based on five kinds of bases.

In ExSELEX, however, it is technically difficult to use, as a starting material, a DNA library comprising nucleic acid strands comprising a Px base. The reason for this is that, under basic conditions used for chemical synthesis of a nucleic acid strand, a nucleotide comprising a Px base is extremely unstable, and thus, it is difficult to construct a DNA library.

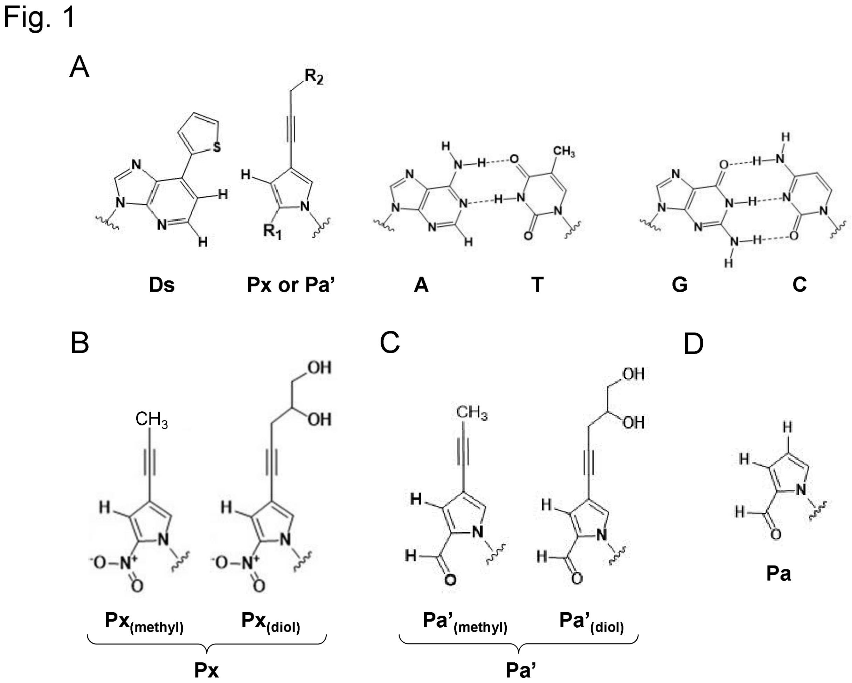

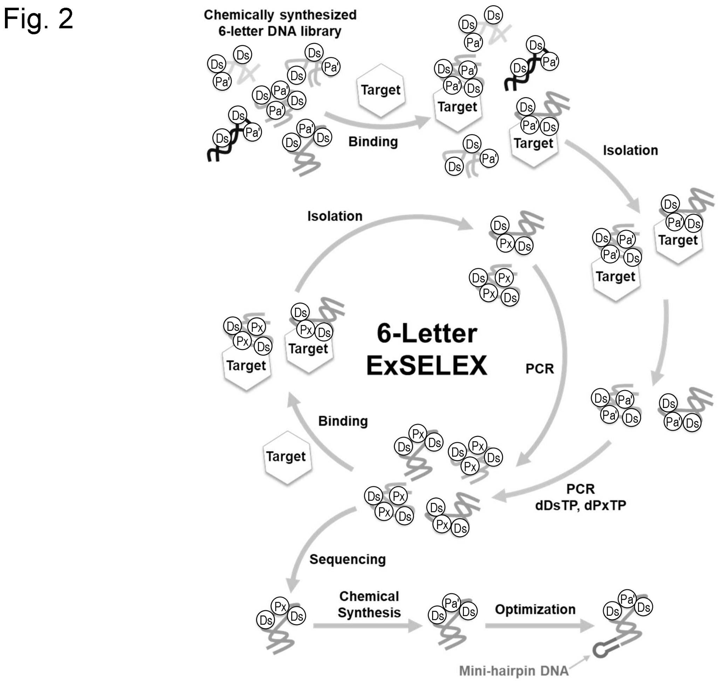

In view of this, the present inventors have conceived the idea of using, in place of a Px base, 4-propynylpyrrole-2-carbaldehyde that is an analog of the Px base, in a step of chemically synthesizing a DNA library (4-propynylpyrrole-2-carbaldehyde and an analog thereof are herein referred to as "Pa'" or a "Pa' base") (Figure 1). The Pa' base can form an unnatural base pair with the Ds base as the Px base can, but, unlike the Px base, is stable under basic conditions. Hence, in the chemical synthesis of a DNA library, the Pa' base was used, but on the other hand, in PCR amplification, the Px base, which has high fidelity in the formation of an unnatural base pair with the Ds base, was used (Figure 2).

The present inventors have applied a new six-letter ExSELEX based on selective use of the Px base or the Pa' base to various target proteins comprising IFN-y, Thrombin, CRP, IL-6, IL-8, and HMGB1, and have discovered that the ExSELEX can efficiently generate a nucleic acid aptamer having extremely high affinity to all the targets.

In addition, a nucleic acid aptamer that has been developed by the present inventors, using a conventional ExSELEX, and that binds to human Interferon gamma (IFN-y) has a problem in that the nucleic acid aptamer has a significantly low binding capacity to glycosylated IFN-y, but it has been revealed that the nucleic acid aptamer newly generated on the basis of six-letter ExSELEX according to the present invention can bind to the target whether the target is glycosylated or not. Many (50 to 70%) of the target proteins in a human body are glycosylated. Hence, the IFN-γ-targeting nucleic acid aptamer of the present invention has a great advantage in the capability to detect IFN-y sufficiently in a living body, compared with the conventional nucleic acid aptamer.

Furthermore, the present inventors have developed a novel method for determining the positions of the Ds base and the Px/Pa' bases in a six-letter nucleic acid aptamer generated on the basis of six-letter ExSELEX according to the present invention, and thus sequencing the bases, and have come to complete the present invention.

The present invention is based on the above-described novel discovery, and provides the following.
[1] A single-stranded nucleic acid molecule library comprising a plurality of single-stranded nucleic acid molecules, wherein:
   the plurality of single-stranded nucleic acid molecules comprises a Ds/Pa'-containing single-stranded nucleic acid molecule,
   the Ds/Pa'-containing single-stranded nucleic acid molecule comprises a pair of primer-binding regions at the 5' end and 3' end thereof, and a variable region between the pair of primer-binding regions,
   the pair of primer-binding regions consist of base sequences which are common among the plurality of single-stranded nucleic acid molecules, and
   the variable region comprises an unnatural base Ds shown in the following general formula (I) and an unnatural base Pa' shown in the following general formula (II): 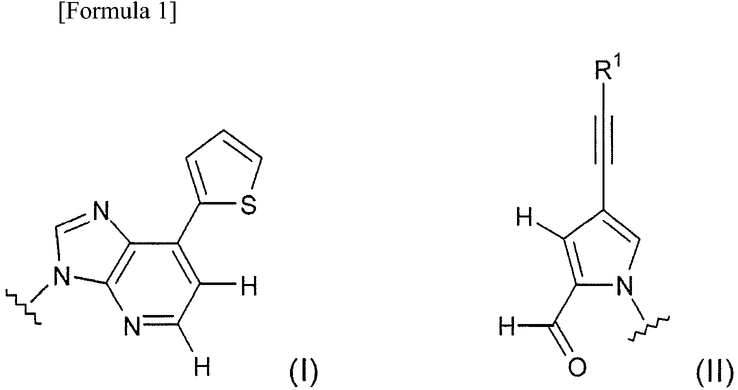
   wherein R¹ respectively and independently represents a hydrogen atom or an optionally substituted hydrocarbon group.
[1-2] A single-stranded nucleic acid molecule library comprising a plurality of single-stranded nucleic acid molecules, wherein:
   the plurality of single-stranded nucleic acid molecules comprises a variable region, and
   wherein the variable region comprises an unnatural base Ds shown in the above-described general formula (I) and an unnatural base Pa' shown in the above-described general formula (II).
[2] A single-stranded nucleic acid molecule library comprising a plurality of single-stranded nucleic acid molecules, wherein:
   the plurality of single-stranded nucleic acid molecules comprises a Ds/Px-containing single-stranded nucleic acid molecule,
   the Ds/Px-containing single-stranded nucleic acid molecule comprises a pair of primer-binding regions at the 5' end and 3' end thereof, and a variable region between the pair of primer-binding regions,
   the pair of primer-binding regions consist of base sequences which are common among the plurality of single-stranded nucleic acid molecules, and
   the variable region comprises an unnatural base Ds shown in the following general formula (I) and an unnatural base Px shown in the following general formula (III): 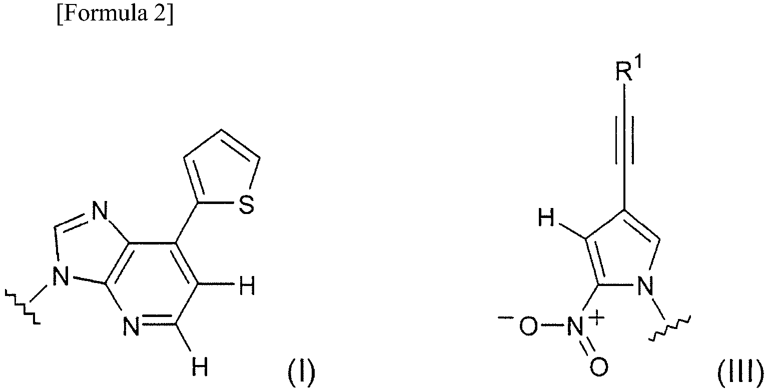
   wherein R¹ respectively and independently represents a hydrogen atom or an optionally substituted hydrocarbon group.
[2-2] A single-stranded nucleic acid molecule library comprising a plurality of single-stranded nucleic acid molecules, wherein:
   the plurality of single-stranded nucleic acid molecules comprises a variable region,
   wherein the variable region comprises an unnatural base Ds shown in the above-described general formula (I) and an unnatural base Px shown in the above-described general formula (III).
[3] The single-stranded nucleic acid molecule library of [1], [1-2], [2], or [2-2], wherein the R¹ represents any one of the following general formulas (IV) to (XXV): 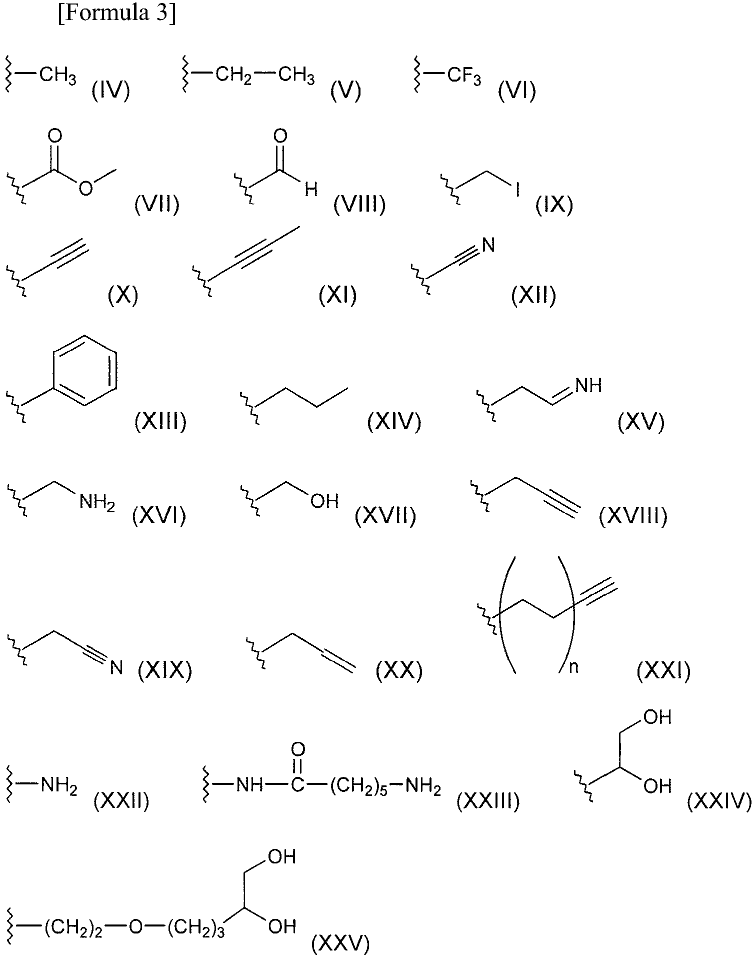 wherein the wave line represents a bond with alkyne, and n represents an integer of 1 or more.
[4] The single-stranded nucleic acid molecule library of any one of [1], [1-2], [2], [2-2], and [3], wherein the variable region comprises one to ten Ds.
[5] The single-stranded nucleic acid molecule library of any one of [1], [1-2], [2], [2-2], and [3], wherein the variable region comprises one to four Ds.
[6] The single-stranded nucleic acid molecule library of any one of [1], [1-2], [2], [2-2], and [3] to [5], wherein the variable region comprises one or more of the Pa' or Px on average.
[7] The single-stranded nucleic acid molecule library of any one of [1], [1-2], [2], [2-2], and [3], wherein the variable region comprises one to three of the Ds, and one to two of the Pa' or Px on average.
[8] The single-stranded nucleic acid molecule library of any one of [1], [1-2], [2], [2-2], and [3] to [7], wherein the variable region in the Ds/Pa'-containing single-stranded nucleic acid molecule or the Ds/Px-containing single-stranded nucleic acid molecule comprises: the Ds at one or more specified positions, and a random sequence consisting of the Pa' or Px and natural bases except the one or more specified positions.
[9] The single-stranded nucleic acid molecule library of any one of [1], [1-2], [2], [2-2], and [3] to [8], comprising a plurality of the Ds/Pa'-containing single-stranded nucleic acid molecules or the Ds/Px-containing single-stranded nucleic acid molecules which differ in the specified positions in the variable region.
[10] The single-stranded nucleic acid molecule library of any one of [1], [1-2], [2], [2-2], and [3] to [9], wherein the variable region is 10 to 300 bases in length.
[11] The single-stranded nucleic acid molecule library of [10], wherein the variable region is 14 to 100 bases in length.
[12] The single-stranded nucleic acid molecule library of any one of [1], [1-2], [2], [2-2], and [3] to [11], wherein each of the plurality of single-stranded nucleic acid molecules comprises a 5'-wing region consisting of natural bases between the primer-binding region at the 5' end and the variable region, and a 3'-wing region consisting of natural bases between the variable region and the primer-binding region at the 3' end.
[12-2] The single-stranded nucleic acid molecule library of any one of [1], [1-2], [2], [2-2], and [3] to [11], wherein each of the plurality of single-stranded nucleic acid molecules comprises: a 5'-wing region consisting of natural bases and located on the 5' end side of the variable region; and a 3'-wing region consisting of natural bases and located on the 3' end side of the variable region.
[13] The single-stranded nucleic acid molecule library of [12], wherein the 5'-wing region and/or the 3'-wing region consists of a random sequence composed of natural bases.
[14] The single-stranded nucleic acid molecule library of [13], wherein the random sequence composed of natural bases is 3 to 20 bases in length.
[15] The single-stranded nucleic acid molecule library of [12], wherein the 5'-wing region and the 3'-wing region comprise a pair of complementary sequences which are complementary to each other and are capable of forming a stem structure.
[16] The single-stranded nucleic acid molecule library of [15], wherein the complementary sequences comprise a non-Watson-Crick-type base pair, and/or an insertion sequence and/or a deletion of one or more bases.
[17] The single-stranded nucleic acid molecule library of [16], wherein the non-Watson-Crick-type base pair comprises a G-T base pair.
[18] The single-stranded nucleic acid molecule library of [16] or [17], wherein the insertion sequence forms a bulge structure or a bulge-out structure.
[19] The single-stranded nucleic acid molecule library of [18], wherein the bulge structure or bulge-out structure comprises an unnatural base.
[20] The single-stranded nucleic acid molecule library of [19], wherein the unnatural base is the Ds, the Pa', and/or the Px.
[21] The single-stranded nucleic acid molecule library of any one of [15] to [20], wherein the pair of complementary sequences are 3 to 20 bases in length.
[22] A method of producing a single-stranded nucleic acid molecule library, comprising:
   an amplification step of amplifying a single-stranded nucleic acid molecule using, as a template, the single-stranded nucleic acid molecule library of [1] or [1-2] or any one of [3] to [21] which recite [1] or [1-2], and
   using, as a substrate, natural nucleotides, Ds-containing nucleotides which contain the unnatural base Ds, and Px-containing nucleotides which contain the unnatural base Px.
[23] A method of producing a single-stranded nucleic acid molecule library, comprising:
   an amplification step of amplifying a single-stranded nucleic acid molecule using, as a template, the single-stranded nucleic acid molecule library of [2] or [2-2] or any one of [3] to [21] which recite [2] or [2-2], and
   using, as a substrate, natural nucleotides, Ds-containing nucleotides which contain the unnatural base Ds, and Pa'-containing nucleotides which contain the unnatural base Pa'.
[24] A method of producing a nucleic acid aptamer consisting of a single-stranded nucleic acid molecule, comprising:
   a complex formation step of mixing the single-stranded nucleic acid molecule library of [2] or [2-2] or any one of [3] to [21] which recite [2] or [2-2] with a target substance in a solution to allow for formation of a complex between a single-stranded nucleic acid molecule in the single-stranded nucleic acid molecule library and the target substance,
   a complex collection step of collecting the complex, and
   an amplification step of amplifying the single-stranded nucleic acid molecule comprised in the complex after the complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.
[24-2] A method of producing a nucleic acid aptamer consisting of a single-stranded nucleic acid molecule, comprising:
   a complex formation step of mixing the single-stranded nucleic acid molecule library of [2] or [2-2] or any one of [3] to [21] which recite [2] or [2-2] with a target substance in a solution to allow for formation of a complex between a single-stranded nucleic acid molecule in the single-stranded nucleic acid molecule library and the target substance, and
   a complex collection step of collecting the complex,
   wherein, if desired, the complex formation step and the complex collection step are repeated a plurality of times.
[25] The method of [24], further comprising a repeating step of repeating the complex formation step to the amplification step once or more than once, using the single-stranded nucleic acid molecule after the amplification step as a new, single-stranded nucleic acid molecule library.
[26] A method of producing a nucleic acid aptamer, comprising:
   a library synthesis step of synthesizing the single-stranded nucleic acid molecule library of [1] or [1-1] or any one of [3] to [21] which recite [1] or [1-1] by reacting natural nucleic acids, Ds-containing nucleic acids which contain the unnatural base Ds, and Pa'-containing nucleic acids which contain the unnatural base Pa',
   a first amplification step of amplifying a single-stranded nucleic acid molecule using, as a template, the single-stranded nucleic acid molecule library after the library synthesis step, and
   using, as a substrate, natural nucleotides, Ds-containing nucleotides which contain the unnatural base Ds, and Px-containing nucleotides which contain the unnatural base Px,
   a complex formation step of mixing the single-stranded nucleic acid molecule after the first amplification step with a target substance in a solution to allow for formation of a complex between the single-stranded nucleic acid molecule and the target substance,
   a complex collection step of collecting the complex, and
   a second amplification step of amplifying the single-stranded nucleic acid molecule comprised in the complex after the complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.
[27] The method of [26], further comprising a repeating step of repeating the complex formation step to the second amplification step once or more than once, for the single-stranded nucleic acid molecule after the second amplification step.
[28] A method of producing a nucleic acid aptamer, comprising:
   a library synthesis step of synthesizing the single-stranded nucleic acid molecule library of [1] or [1-1] or any one of [3] to [21] which recite [1] or [1-1] by reacting natural nucleic acids, Ds-containing nucleic acids which contain the unnatural base Ds, and Pa'-containing nucleic acids which contain the unnatural base Pa',
   a first complex formation step of mixing the single-stranded nucleic acid molecule library after the library synthesis step with a target substance in a solution to allow for formation of a complex between a single-stranded nucleic acid molecule in the single-stranded nucleic acid molecule library and the target substance,
   a first complex collection step of collecting the complex after the first complex formation step,
   a first amplification step of amplifying a single-stranded nucleic acid molecule using, as a template, the single-stranded nucleic acid molecule after the first complex collection step, and
   using, as a substrate, natural nucleotides, Ds-containing nucleotides which contain the unnatural base Ds, and Px-containing nucleotides which contain the unnatural base Px,
   a second complex formation step of mixing the single-stranded nucleic acid molecule after the first amplification step with the target substance in a solution to allow for formation of a complex between the single-stranded nucleic acid molecule and the target substance,
   a second complex collection step of collecting the complex after the second complex formation step, and
   a second amplification step of amplifying the single-stranded nucleic acid molecule comprised in the complex after the second complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.
[29] The method of [28], further comprising a repeating step of repeating the second complex formation step to the second amplification step once or more than once, for the single-stranded nucleic acid molecule after the second amplification step.
[30] A method of determining the base sequence of a single-stranded nucleic acid molecule containing an unnatural base,
   wherein the single-stranded nucleic acid molecule containing an unnatural base comprises a pair of primer-binding regions at the 5' end and 3' end thereof, and a central region between the pair of primer-binding regions,
   wherein the central region comprises an unnatural base Ds shown in the following general formula (I) and an unnatural base Px shown in the following general formula (III):
      wherein the method comprises:
      a first amplification step of amplifying the single-stranded nucleic acid molecule containing an unnatural base by a nucleic acid amplification method using natural nucleotides as a substrate and using a primer pair which binds to the base sequence of the pair of primer-binding regions, and
      a sequencing step of sequencing the base sequence of an amplification product composed only of natural nucleotides which is obtained after the first amplification step: 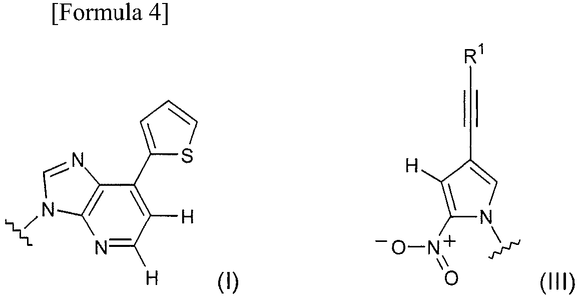
      wherein R¹ respectively and independently represents a hydrogen atom or an optionally substituted hydrocarbon group having one or more carbon atoms.
[30-2] A method of determining the base sequence of a single-stranded nucleic acid molecule containing an unnatural base,
   wherein the single-stranded nucleic acid molecule containing an unnatural base comprises a central region,
   the central region comprises an unnatural base Ds shown in the above-described general formula (I) and an unnatural base Px shown in the above-described general formula (III):
      wherein the method comprises:
      a linking step of linking a pair of nucleic acid sequences consisting of a pair of primer-binding regions with the 5' end and 3' end of the central region,
      a first amplification step of amplifying the single-stranded nucleic acid molecule containing an unnatural base by a nucleic acid amplification method using natural nucleotides as a substrate and using a primer pair which binds to the base sequence of the pair of primer-binding regions, and
      a sequencing step of sequencing the base sequence of an amplification product composed only of natural nucleotides which is obtained after the first amplification step.
[31] The method of [30], further comprising: a second amplification step of amplifying the single-stranded nucleic acid molecule containing an unnatural base by a nucleic acid amplification method using, as a substrate, natural nucleotides, Ds-containing nucleotides which contain the Ds, and Px-containing nucleotides which contain the Px, and using a primer pair which are labeled with different labeling molecules and which bind to the base sequences of the pair of primer-binding regions,
   a cleavage step of completely or partially cleaving the single-stranded nucleic acid molecule containing an unnatural base and the complementary strand thereof bound to the different labeling molecules which are amplified after the second amplification step at the position(s) of the Px, and
   a Px position determining step of determining the position(s) of the Px in the single-stranded nucleic acid molecule containing an unnatural base and the complementary strand thereof by determining the size of single-stranded nucleic acid fragments derived from the single-stranded nucleic acid molecule containing an unnatural base and the complementary strand thereof after the cleavage step based on detection of the labeling molecules.
[32] The method of [31], further comprising: a Ds/Px position determining step of determining the base positions where both of natural bases A and T are sequenced in the base sequence sequenced in the sequencing step as the positions of the Ds or the Px, and
   a Ds position identifying step of identifying, as the position(s) of the Ds, the position(s) other than the position(s) determined as the position(s) of the Px in the Px position determining step, among the positions of the Ds or the Px determined in the Ds/Px position determining step.
[33] The method of [30], further comprising: a Ds/Px position determining step of determining the base positions where both of natural bases A and T are sequenced in the base sequence sequenced in the sequencing step as the positions of the Ds or the Px,
   a synthesis step of synthesizing a plurality of single-stranded nucleic acid molecules which comprise the Ds and/or the Px at the positions of the Ds or the Px determined in the Ds/Px position determining step in multiple or all combinations, wherein the sequence of the single-stranded nucleic acid molecule except the position of the Ds or the Px consists of the base sequence sequenced in the sequencing step,
   a complex formation step of mixing the plurality of single-stranded nucleic acid molecules synthesized in the synthesis step with a target substance to allow for formation of a complex between the plurality of single-stranded nucleic acid molecules and the target substance,
   a measurement step of measuring complex formation efficiency after the complex formation step, and,
   a selection step of selecting the base sequence of the single-stranded nucleic acid molecule(s) which shows a higher or the highest complex formation efficiency among the plurality of single-stranded nucleic acid molecules, as the base sequence of the single-stranded nucleic acid molecule containing an unnatural base, after the measurement step.
[34] The method of any one of [30] to [33], wherein the single-stranded nucleic acid molecule containing an unnatural base is derived from the single-stranded nucleic acid molecule library of [2] or [2-2] or any one of [3] to [21] which recite [2] or [2-2], or is a single-stranded nucleic acid molecule produced by the method of any one of [24] to [29].
[35] The method of [31] or [32], wherein the different labeling molecules can be distinguished based on the wavelength of fluorescence thereof.
[36] The method of any one of [30] to [35], wherein the central region comprises one to ten Ds.
[37] The method of [36], wherein the central region comprises one to four Ds.
[38] The method of any one of [30] to [37], wherein the central region comprises one to three Ds and one to two Px.
[39] A nucleic acid aptamer selected from the group consisting of the following (1) to (6):
   (1) a nucleic acid aptamer which binds to Interferon gamma (IFN-y) selected from the group consisting of the below-described (1-i-a) to (1-iv-d) herein;
   (2) a nucleic acid aptamer which binds to Interferon gamma (IFN-y) selected from the group consisting of the below-described (2-i-a) to (2-vi-d) herein;
   (3) a nucleic acid aptamer which binds to C-reactive protein (CRP) selected from the group consisting of the below-described (3-i-a) to (3-v-b);
   (4) a nucleic acid aptamer which binds to Thrombin selected from the group consisting of the below-described (4-i-a) to (4-vi-d) herein;
   (5) a nucleic acid aptamer which binds to Interleukin-6 (IL-6) selected from the group consisting of the below-described (5-i-a) to (5-ii-d) herein;
   (6) a nucleic acid aptamer which binds to Interleukin-8 (IL-8) selected from the group consisting of the below-described (6-i-a) to (6-vii-d) herein;
   (7) a nucleic acid aptamer that binds to HMGB1 protein selected from the group consisting of the below-described (7-a) to (7-d) herein; and
   (8) a nucleic acid aptamer that binds to Interferon gamma (IFN-y) selected from the group consisting of the below-described (8-i-a) to (8-xii-b) herein.
[40] The nucleic acid aptamer of [39], wherein the pair of base sequences comprise a non-complementary base pair, and/or an insertion sequence and/or a deletion of one or more bases.
[41] The nucleic acid aptamer of [40], wherein the non-complementary base pair comprises a G-T base pair.
[42] The nucleic acid aptamer of [40] or [41], wherein the insertion sequence forms a bulge structure or a bulge-out structure.
[43] The nucleic acid aptamer of [42], wherein the bulge structure or bulge-out structure comprises an unnatural base.
[44] The nucleic acid aptamer of [43], wherein the unnatural base is the Ds, the Pa', and/or the Px.
[45] The nucleic acid aptamer of any one of [39] to [44], wherein the pair of base sequences consist of SEQ ID NOs: 51 and 52; SEQ ID NO: 53 (CTCCGCGTC) and SEQ ID NO: 54 (GACGCGGAG); SEQ ID NO: 55 (GTCCTCACG) and SEQ ID NO: 56 (CGTGAGGAC); SEQ ID NOs: 57 and 58 (wherein the base shown as n at position 9 in SEQ ID NO: 57 is the Ds); SEQ ID NOs: 59 and 60 (GGTCATGTGACAAGCAGC); SEQ ID NOs: 61 and 62; SEQ ID NOs: 63 and 64; SEQ ID NO: 65 (GCACGCTTT) and SEQ ID NO: 66 (AAAGCGTGC); SEQ ID NO: 67 (GCTGTCAC) and SEQ ID NO: 68 (GTGACAGC); SEQ ID NO: 69 (GCGTTG) and SEQ ID NO: 70 (CAACGC); SEQ ID NO: 71 (GCTGATCT) and SEQ ID NO: 72 (AGATCAGC); SEQ ID NOs: 73 and 74 (wherein the base shown as n at position 3 in SEQ ID NO: 74 is the Ds); SEQ ID NO: 75 and SEQ ID NO: 76 (ACCACGTGC); SEQ ID NOs: 77 and 78; SEQ ID NOs: 79 and 80; SEQ ID NOs: 81 and 82; SEQ ID NOs: 79 and 83 (wherein the base shown as n at position 7 in SEQ ID NO: 83 is the Ds); SEQ ID NOs: 84 and 85; SEQ ID NOs: 86 and 87; SEQ ID NO: 232 (GCGCTTT) and SEQ ID NO: 233 (AAAGCGC); SEQ ID NO: 234 (GCGTT) and SEQ ID NO: 235 (AACGC); SEQ ID NOs: 239 and 240; or SEQ ID NOs: 241 and 242.
[46] The nucleic acid aptamer of any one of [39] to [45], comprising a mini-hairpin sequence at the 5' end and/or 3' end thereof.
[47] The nucleic acid aptamer of any one of [39] to [46], wherein the Interferon gamma (IFN-γ) is glycosylated.
[48] The nucleic acid aptamer of any one of [39] to [46], wherein the R¹ represents any one of the following general formulas (IV) to (XXV): 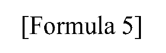 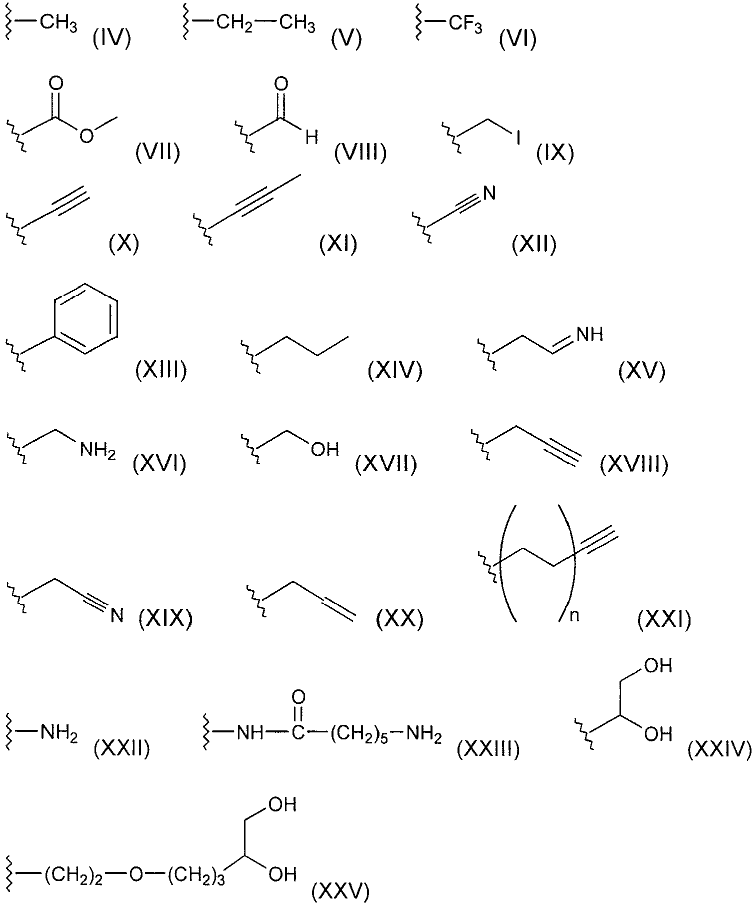 wherein the wave line represents a bond with alkyne, and n represents an integer of one or more.
[49] A kit for detecting Interferon gamma (IFN-γ), C-reactive protein, Thrombin, Interleukin-6, Interleukin-8, or HMGB 1, comprising the nucleic acid aptamer of any one of [39] to [48].
[50] A method for detecting Interferon gamma (IFN-γ), C-reactive protein, Thrombin, Interleukin-6, Interleukin-8, or HMGB1, comprising:
   a detection step of detecting Interferon gamma (IFN-γ), C-reactive protein, Thrombin, Interleukin-6, Interleukin-8, or HMGB1 with the nucleic acid aptamer of any one of [39] to [48].
[51] The kit of [49] or the method of [50], for detecting an inflammation or an inflammatory disease, or evaluating an inflammatory level thereof.
[52] The kit or method of [51], comprising the nucleic acid aptamer of the item (3) or comprising use of the nucleic acid aptamer of the item (3), wherein the inflammatory disease is an infection, collagen disease, cardiovascular disease, or tumor, or
   wherein the inflammation is associated with tissue damage, necrosis, or surgery.
[53] The kit of [49] comprising the nucleic acid aptamer of the item (1), (2), or (8), or the method of [50] comprising using the nucleic acid aptamer of the item (1), (2), or (8), for evaluating the activation level of T cells and/or NK cells in an immunotherapy, or for detecting an infection of Mycobacterium tuberculosis.
[54] A pharmaceutical composition comprising the nucleic acid aptamer of any one of [39] to [48].
[55] The pharmaceutical composition of [54] for anti-inflammation.
[56] The pharmaceutical composition of [54] for use in apheresis, comprising the nucleic acid aptamer of the item (1), (2), or (8) as an active ingredient.
[57] The pharmaceutical composition of [54] for suppressing or inhibiting blood clotting, comprising the nucleic acid aptamer of the item (4) as an active ingredient.
[58] The pharmaceutical composition of [54] for treating rheumatism, comprising the nucleic acid aptamer of the item (5) as an active ingredient.
[59] The pharmaceutical composition of [54] for treating cancer, comprising the nucleic acid aptamer of the item (6) as an active ingredient.
[60] A method of treating a disease, comprising a step of administering the nucleic acid aptamer of any one of [39] to [48] to a subject.
[61] The method of [60], which is for treating an inflammatory disease.
[62] The method of [60], comprising a step of removing Interferon gamma (IFN-y) from blood by apheresis using the nucleic acid aptamer of the item (1), (2), or (8).
[63] The method of [60], comprising administering the nucleic acid aptamer of the item (4) to the subject for suppressing or inhibiting blood clotting.
[64] The method of [60] for treating rheumatism, comprising administering the nucleic acid aptamer of the item (5) to the subject.
[65] The method of [60] for treating cancer, comprising administering the nucleic acid aptamer of the item (6) to the subject.
[66] The method of [60] for treating an inflammatory disease, an autoimmune disease, cancer, a cardiovascular disease, or a neurological disease, comprising administering the nucleic acid aptamer of the item (7) to the subject.

The present specification encompasses the disclosure of Singapore Patent Application No. 10202300790X that serves as the basis of the priority of the present application. In this regard, SEQ ID NOs: 1 to 87 herein are the same as SEQ ID NOs: 1 to 87 in Singapore Patent Application No. 10202300790X.

### Advantageous Effects of Invention

The present invention provides a novel method for efficiently generating a nucleic acid aptamer having high affinity to a target protein.

### Brief Description of Drawing

[Figure 1] Figure 1 shows: a Ds base that is a hydrophobic unnatural base; a Px base and a Pa' base that are to base-pair with a Ds base; and four kinds of natural DNA bases. Figure 1A shows the structure of each base pair. Figure 1B shows a Px_{(methyl)} base and a Px_{(diol)} base. Figure 1C shows a Pa'_{(methyl)} base and a Pa'_{(diol)} base. Figure 1D shows a Pa base.
[Figure 2] Figure 2 shows the overview of six-letter ExSELEX.
[Figure 3] Figure 3 shows the results obtained by analyzing the affinity of an AptD2 aptamer and a modified AptD2 aptamer to DEN2-NS1, using EMSA, in which the modified AptD2 aptamer is obtained by substituting the Px_{(diol)} base of the AptD2 aptamer with any of the different bases.
[Figure 4] Figure 4 schematically shows the overview of six-letter ExSELEX intended for an XL1 library or an XL2 library. Figure 4A schematically shows the overview of six-letter ExSELEX intended for an XL1 library. Figure 4B shows the constitution of an XL2 library. Here, in the drawings, the Pa' base shows a Pa'_{(methyl)} base, and the Px base shows a Px_{(methyl)} base (the same applies to the below-mentioned drawings).
[Figure 5] Figure 5 schematically shows a method of determining the position of an unnatural base in an aptamer selected in accordance with six-letter ExSELEX.
[Figure 6] Figure 6 shows the results obtained by analyzing IFN-γ-targeting nucleic acid aptamers obtained in six-letter ExSELEX intended for an XL1 library. Figure 6A shows the sequences of the higher-level families larger in the number of reads. Figure 6B shows the results obtained by analyzing the positions of unnatural bases in a nucleic acid aptamer that belongs to Family 1 in Figure 6A. Figure 6C shows the positions determined of the unnatural bases.
[Figure 7] Figure 7 shows the results obtained by analyzing the affinity of seven kinds of nucleic acid aptamers (AptIFN-γ-1, AptIFN-γ-2, AptIFN-γ-3, AptIFN-γ-4, AptIFN-γ-5, AptIFN-γ-6, and Bio-AptIFN-γ-3) to human IFN-y, using EMSA.
[Figure 8] Figure 8 shows the results obtained by analyzing the affinity of five kinds of nucleic acid aptamers (AptIFN-γ-1, AptIFN-γ-3, and Bio-AptIFN-γ-3; AptDs-IFN-γ and Bio-AptDs-IFN-γ as comparative controls) to non-glycosylated IFN-γ and glycosylated IFN-y, using EMSA.
[Figure 9] Figure 9 shows the results obtained by analyzing IFN-γ-targeting nucleic acid aptamers obtained in six-letter ExSELEX using XL2 library. Figure 9A shows the sequences of the higher-level families larger in the number of reads. Figure 9B shows the results obtained by analyzing the affinity to glycosylated human IFN-y, using EMSA. Figure 9C shows the results obtained by analyzing the positions of unnatural bases in a nucleic acid aptamer that belongs to Family 1 in Figure 9A. Figure 9D shows the positions determined of the unnatural bases.
[Figure 10] Figure 10 shows the results obtained by analyzing the affinity of 10 kinds of nucleic acid aptamers (XL2-01a, XL2-01b, XL2-01c, XL2-01d, XL2-01e, XL2-01h, XL2-01i, XL2-01j, XL2-01k, and XL2-011) to non-glycosylated IFN-γ and glycosylated IFN-y, using EMSA. Figure 10A shows the results of XL2-01a to XL2-01e. Figure 10B shows the results of XL2-01h to XL2-01l.
[Figure 11] Figure 11 shows the results obtained by analyzing the affinity of 3 kinds of nucleic acid aptamers (Bio-XL2-01a, Bio-XL2-01i, and Bio-AptDs-IFN-γ) to glycosylated IFN-y, using ELONA.
[Figure 12] Figure 12 shows the results obtained by analyzing CRP-targeting nucleic acid aptamers obtained in six-letter ExSELEX. Figure 12A shows the sequences of the higher-level families larger in the number of reads. Figure 12B shows the results obtained by analyzing the affinity of chemically synthesized CRP-targeting nucleic acid aptamers to CRP, using EMSA.
[Figure 13] Figure 13 shows the sequences of the higher-level families larger in the number of reads in six-letter ExSELEX targeted at Thrombin.
[Figure 14] Figure 14 shows the results obtained by analyzing the affinity of 9 kinds of nucleic acid aptamers (Thr101, Thr102, Thr104, Thr106, Thr201, Thr202, Thr203, Thr204, and Thr205) to human Thrombin, using EMSA.
[Figure 15] Figure 15 shows the results obtained by analyzing the affinity of 7 kinds of nucleic acid aptamers (Thr104, Bio-Thr104b, Thr204, Bio-Thr204, Thr205, Bio-Thr205, and BioMH-RE31) to human Thrombin using EMSA.
[Figure 16] Figure 16 shows the results obtained by analyzing the affinity of 4 kinds of nucleic acid aptamers (Bio-Thr104b, Bio-Thr204, Bio-Thr205, and BioMH-RE31) to human Thrombin using ELONA.
[Figure 17] Figure 17 shows the results of six-letter ExSELEX targeted at IL-6. Figure 17A shows the sequence of the higher-level families larger in the number of reads. Figure 17B shows the results obtained by analyzing the affinity of IL-6-targeting nucleic acid aptamers to IL-6, using SPR.
[Figure 18] Figure 18 shows the sequences of the higher-level families larger in the number of reads in six-letter ExSELEX targeted at IL-8.
[Figure 19] Figure 19 shows the results obtained by analyzing the affinity of six kinds of nucleic acid aptamers (IL8-102DD, IL8-102PD, IL8-223DD, IL8-223PD, IL8-201DDP, and IL8-201DPD) to human IL-8, using EMSA.
[Figure 20] Figure 20 shows the results obtained by analyzing the affinity of six kinds of nucleic acid aptamers (IL8-102PD, IL8-223PD, Bio-IL8-102PD, Bio-IL8-223PD, and Bio-IL8-223PDb) to human IL-8 using EMSA.
[Figure 21] Figure 21 shows the results obtained by analyzing the affinity of the nucleic acid aptamers Bio-HMGB1-301-DD and Bio-HMGB1-301-AA to human HMGB1 protein, using EMSA.
[Figure 22] Figure 22 shows the results obtained by analyzing the affinity of the nucleic acid aptamers produced in Example 5 to glycosylated IFN-γ and non-glycosylated IFN-y, using EMSA.
[Figure 23] Figure 23 shows the results obtained by analyzing the affinity of the nucleic acid aptamers produced in Example 5 to glycosylated IFN-γ and non-glycosylated IFN-y, using EMSA.
[Figure 24] Figure 24 shows the results obtained by analyzing the affinity of the nucleic acid aptamers produced in Example 5 to glycosylated IFN-γ and non-glycosylated IFN-y, using ELISA.
[Figure 25] Figure 25 shows the results obtained by analyzing the affinity of the nucleic acid aptamers produced in Example 5 to glycosylated IFN-γ and non-glycosylated IFN-y, using EMSA.
[Figure 26] Figure 26 shows the results obtained by analyzing the affinity of the nucleic acid aptamers produced in Example 5 to glycosylated IFN-γ and non-glycosylated IFN-y, using EMSA.
[Figure 27] Figure 27 shows the results obtained by analyzing the affinity of the nucleic acid aptamers produced in Example 5 to Thrombin, using EMSA.

### Description of Embodiments

### <Definitions of terms>

The definitions of the general terms used herein will be described below.

As used herein, a "nucleic acid aptamer" is an aptamer composed of a nucleic acid molecule, and refers to a ligand molecule that binds firmly and specifically to a target substance owing to the conformation formed on the basis of a secondary structure, or furthermore a tertiary structure, formed by single-stranded nucleic acid molecules via hydrogen bonding or the like. In a case where a nucleic acid aptamer has the capability to specifically inhibit or suppress the function, for example, the physiological activity, of a target substance, the nucleic acid aptamer can be an agent for inhibiting the function of the target substance. As used herein, "inhibiting the function of a target substance" refers to inhibiting or suppressing the function, for example, the catalytic function or the gene expression regulating function, of a target substance. As used herein, a nucleic acid constituting a nucleic acid aptamer is not particularly limited to any kind, and may be, for example, a DNA aptamer composed of DNA alone, an RNA aptamer composed of RNA alone, or a nucleic acid aptamer composed of a combination of DNA and RNA.

As used herein, a "target substance" refers to a substance that can be an object to which a nucleic acid aptamer binds. The target substance is not particularly limited to any kind, subject to being a biological material to which a nucleic acid aptamer can bind. Examples include: proteins, such as peptides (oligopeptides and polypeptides); nucleic acids; lipids; sugars (comprising a sugar chain); and low-molecular-weight compounds. A substance as a target to which a nucleic acid aptamer of the present invention binds is a protein in principle, and thus, a target substance is herein referred to as a "target protein" in some cases.

As used herein, a "nucleic acid" or a "nucleic acid molecule" refers to a polymer in which the constituent units are nucleosides in principle, and linked via an internucleoside bond.

As used herein, a "natural nucleoside" refers to a nucleoside present in nature. Examples include: a ribonucleoside consisting of a ribose and a base, such as adenine, cytosine, guanine, or uracil; and a deoxyribonucleoside consisting of a deoxyribose and the base, such as adenine, cytosine, guanine, or thymine. As used herein, a "natural nucleotide" is a nucleotide composed of a natural nucleoside.

As used herein, an "unnatural nucleoside" refers to an arbitrary nucleoside other than a natural nucleoside, and comprises a modified nucleoside and a nucleoside mimic.

As used herein, a "modified nucleoside" means a nucleoside having a modified portion, such as a modified base and/or a modified sugar portion.

As used herein, a "nucleotide" refers to a molecule having a phosphate group covalently bound to the sugar portion of a nucleoside. In a nucleotide comprising pentofuranosyl sugar, a phosphate group is usually linked to the hydroxyl group at position 2', position 3', or position 5' of the sugar. Amidite or phosphoramidite to be used as a raw material for nucleic acid synthesis is encompassed in a nucleotide.

As used herein, an "oligonucleotide" refers to a linear oligomer formed by several to tens of nucleotides linked with each other, in which the hydroxyl group of the sugar portion and the phosphate group are linked via covalent bonding between any nucleotides flanked with each other. In addition, a "polynucleotide" refers to a linear polymer formed by linking, via the covalent bonding, more nucleotides - tens or more of, preferably hundreds or more of, nucleotides - than in an oligonucleotide. Inside a natural oligonucleotide or polynucleotide structure, the phosphate group generally forms an internucleoside bond.

As used herein, a "natural base" refers to any of adenine, cytosine, guanine, thymine, uracil, and a modified base thereof that are present in nature. Note that, in the base sequences herein, a purine base selected from adenine and guanine is represented by "R", and a pyrimidine base selected from cytosine, thymine, and uracil is represented by "Y".

As used herein, an "unnatural base" or an "unnatural base" refers to an arbitrary nucleobase other than a natural base. An unnatural base or an unnatural base can substitute for a natural base constituting a natural nucleoside. In this regard, an unnatural base or an unnatural base herein may be a base that is capable of forming an unnatural base pair, or that is not capable of forming an unnatural base pair.

As used herein, an "unnatural base pair" refers to a pair of unnatural bases capable of forming base pairing, such as adenine and thymine, adenine and uracil, or guanine and cytosine, which are natural bases. Base pairing formed by an unnatural base pair can comprise a hydrogen bond found in base pairing between natural bases, a stacking effect via hydrophobic interaction, and the like. In some cases, a base-pairable unnatural base pair can be accurately replicated and transcribed owing to the complementarity between the unnatural bases. A nucleic acid strand comprising an unnatural base can also be amplified using a nucleic acid amplification method, such as PCR.

Specific examples of the above-described unnatural base include: Ds (7-(2-thienyl)-3H-imidazo[4,5-b]pyridine-3-yl; an unnatural base shown in the below-described general formula (I); herein referred to as an "unnatural base Ds" or simply as a "Ds base" or "Ds"); Pa (2-formyl-1*H*-pyrrole-1-yl; herein referred to as "Pa"); Pa' (an unnatural base shown in the below-described general formula (II); herein referred to as an "unnatural base Pa'" or simply as a "Pa' base" or "Pa"'); Px (an unnatural base shown in the below-described general formula (III); herein referred to as an "unnatural base Px" or simply a "Px base" or "Px"); Pn (2-nitropyrrole-1-yl; herein referred to as "Pn"); P (2-amino-imidazo[1,2-a]-1,3,5-triazin-4(8*H*)-one; herein referred to as "P"); Z (6-amino-5-nitro-2(1*H*)-pyridone; herein referred to as "Z"); 5SICS (6-methylisoquinoline-1(2*H*)-thione; herein referred to as "5SICS"); NaM (3-methoxynaphthalen-2-yl; herein referred to as "NaM"); and MMO2 (2-methoxy-4-methylphenyl; herein referred to as "MMO2"). Among these unnatural bases, examples of a complementary unnatural base of Ds include Pa, Px, Pa', and Pn. Examples of a complementary unnatural base of P include Z. Examples of a complementary unnatural base of 5SICS include NaM and MMO2.

The unnatural base Ds is herein shown in the following general formula (I). In addition, the unnatural base Pa' is herein shown in the following general formula (II). Furthermore, the unnatural base Px is herein shown in the following general formula (III): 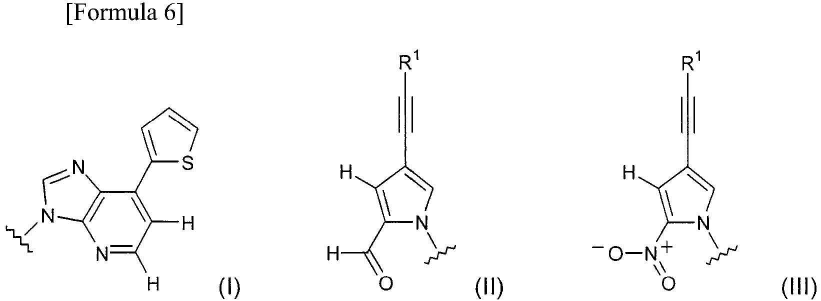 wherein, R¹ represents a hydrogen atom or an optionally substituted hydrocarbon group.

In the unnatural bases shown in the above-described general formula (I), general formula (II), and general formula (III), the wave line shows a sugar portion in a nucleoside or a nucleotide. In a case where the nucleic acid aptamer is a DNA aptamer, the sugar portion in a nucleoside or a nucleotide is a deoxyribose portion, and the unnatural base binds to the carbon atom at position 1' in the deoxyribose. In a case where the nucleic acid aptamer is an RNA aptamer, the sugar portion in a nucleoside or a nucleotide is a ribose portion, and the unnatural base binds to the carbon atom at position 1' in the ribose.

In the above-described general formula (II) and general formula (III), R¹ represents a hydrogen atom or an optionally substituted hydrocarbon group, or may be, for example, an optionally substituted alkyl group. As used herein, an "alkyl" or "alkyl group" refers to a saturated hydrocarbon having one or more carbon atoms. Specific examples of the alkyl group include methyl, ethyl, propyl, and butyl. In addition, examples of substitution of a hydrogen group in an alkyl group include substitution with hydroxyl, carboxyl, cyano, nitro, halogen (for example, Cl, F, Br, or I), thiol, alkoxyl group, ester, thioether, thio ester, nitro, amine, or the like. The number of substitutions of a hydrogen group in an alkyl group is not limited, and may be, for example, 1 or 2 or more.

Further examples of R¹ include a structure represented by any one formula shown in the following formula (IV) to formula (XXV).

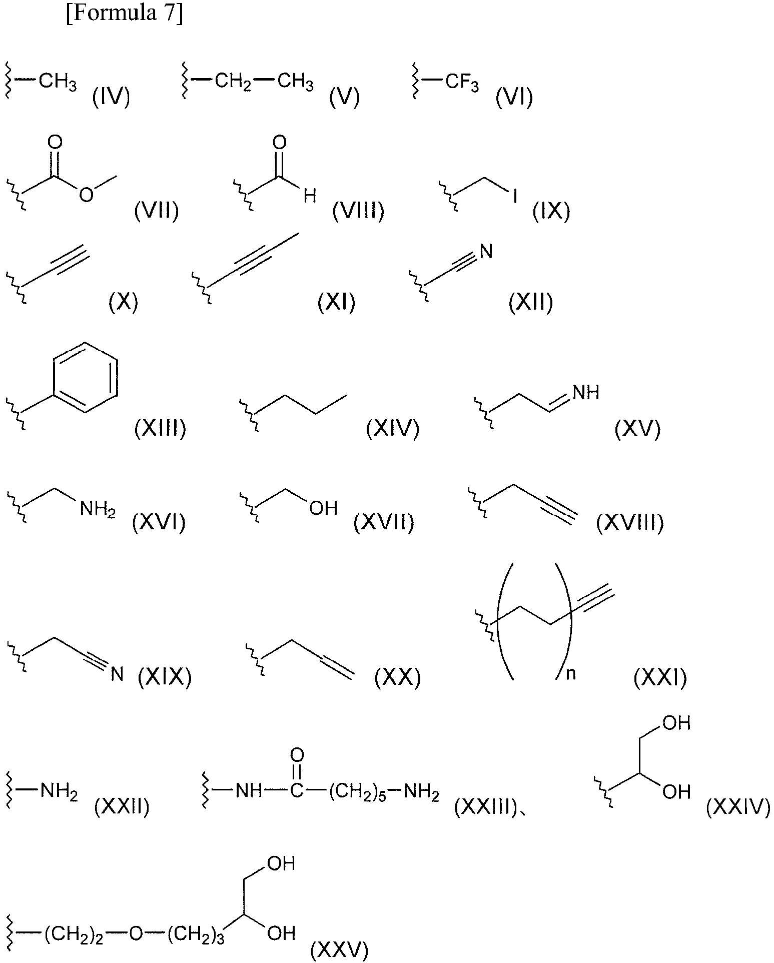

In this regard, in the above-described formula (IV) to formula (XXV), the wave line represents a bond to alkyne in the general formula (II) or the general formula (III), and n represents an integer of 1 or more.

Herein, a Pa' base in which the R¹ group is a methyl group shown in the above-described formula (IV) is referred to as "Pa'_{(methyl)}". In addition, a Px base in which the R¹ group is a methyl group shown in the above-described formula (IV) is referred to as "Px_{(methyl)}". Furthermore, a Pa' base in which the R¹ group is a diol group shown in the above-described formula (XXIV) is referred to as "Pa'_{(diol)}", and a Px base in which R¹ group is a diol group shown in the above-described formula (XXIV) is referred to as "Px_{(diol)}". Hereinafter, the Pa' base may be any above-described Pa' base, and is preferably a Pa'_{(methyl)} base or a Pa'_{(diol)} base, more preferably a Pa'_{(methyl)} base. In addition, the Px base may be any above-described Px base, and is preferably a Px_{(methyl)} base or a Px_{(diol)} base, more preferably a Px_{(methyl)} base.

When the substrate for replication or transcription does not comprise an unnatural nucleoside having a complementary unnatural base for an unnatural base, the unnatural base is base-paired alternatively with a natural base close to the complementary unnatural base in structure and/or nature, in some cases. In this case, the unnatural nucleoside in a nucleic acid molecule used as a template result in being substituted with the natural nucleoside after replication or transcription. For example, the above-described Ds, Px, Pa', or the like can be substituted with A or T.

As used herein, a "modified base" means a chemically-modified base. Examples of the modified base include modified pyrimidines (for example, 5-hydroxycytosine, 5-fluorouracil, 4-thiouracil, 5-(3-indole-2-ethyl)uracil, and 5-(4-hydroxyphenyl-2-ethyl)uracil), modified purines (for example, 6-methyladenine and 6-thioguanosine), and other heterocyclic bases.

As used herein, a "stem structure" or a "stem region" means a double-stranded structure formed by part of the constituent bases, in which part, for example, consecutive two or more bases are completely or partly base-paired with each other. The length of the stem structure is, for example, 1 bp or more, 2 bp or more, 3 bp or more, 4 bp or more, or 5 bp or more, and/or 30 bp or less, 25 bp or less, 20 bp or less, 15 bp or less, 10 bp or less, 9 bp or less, or 8 bp or less. Examples of the stem structure include a structure formed by base-pairing of two base sequences of SEQ ID NOs: 51 and 52; SEQ ID NOs: 53 and 54; SEQ ID NOs: 55 and 56; SEQ ID NOs: 57 and 58 (the base shown as n at position 9 in SEQ ID NO: 57 is Ds); SEQ ID NOs: 59 and 60; SEQ ID NOs: 61 and 62; SEQ ID NOs: 63 and 64; SEQ ID NOs: 65 and 66; SEQ ID NOs: 67 and 68; SEQ ID NOs: 69 and 70; SEQ ID NOs: 71 and 72; SEQ ID NOs: 73 and 74 (the base shown as n at position 3 in SEQ ID NO: 74 is Ds); SEQ ID NO: 75 and SEQ ID NO: 76; SEQ ID NOs: 77 and 78; SEQ ID NOs: 79 and 80; SEQ ID NOs: 81 and 82; SEQ ID NOs: 79 and 83 (the base shown as n at position 7 in SEQ ID NO: 83 is Ds); SEQ ID NOs: 84 and 85; SEQ ID NOs: 86 and 87; SEQ ID NOs: 232 and 233; SEQ ID NOs: 234 and 235; SEQ ID NOs: 239 and 240; or SEQ ID NOs: 241 and 242.

As used herein, a "mini-hairpin structure" or a "mini-hairpin" means a structure in which the below-described three DNA nucleic acid regions - the first nucleic acid region, the second nucleic acid region, and the third nucleic acid region - are linked in this order from the 5' end side to the 3' end side. The "first nucleic acid region" is a nucleic acid region consisting of two to five arbitrary nucleotides. A base in this nucleic acid region is preferably, but not limited to, guanine or cytosine. The "second nucleic acid region" is a nucleic acid region consisting of the base sequence 5'-gna-3' or 5'-gnna-3'. Each n in the sequence independently consists of any of a natural base, the base analog, and the modified base. The "third nucleic acid region" is a nucleic acid region having a base sequence complementary to the first nucleic acid region. Accordingly, the base sequence of the third nucleic acid region depends on the base sequence of the first nucleic acid region. In addition, the first nucleic acid region and the third nucleic acid region form a base pair in a molecule. As a result, the first nucleic acid region and the third nucleic acid region are completely base-paired with each other to constitute a stem portion, and in addition, the second nucleic acid region present between the first nucleic acid region and the third nucleic acid region constitutes a loop portion. One example of the mini-hairpin sequence includes 5'-CGCGTAGCG-3' (SEQ ID NO: 133; the base T may be biotinylated). The mini-hairpin structure enhances degradation resistance to nuclease, and/or can result in an increased thermal stability of a DNA aptamer through increasing the Tm value of the DNA aptamer.

As used herein, an "internal loop structure" refers to a loop structure that is within a stem structure, and generated in a case where one or more bases not forming a base pair are present at the positions corresponding between both of two strands forming the stem structure.

As used herein, a "bulge structure" refers to a protrusion structure within a stem structure, in which the protrusion structure is generated only at one of the positions corresponding between two strands forming the stem structure, at which position one or more bases not forming a base pair are present.

As used herein, the "loop structure" means a loop-shaped structure not base-paired inside a nucleic acid, in which the structure is generated owing to the formation of a stem structure, and located between two strands constituting the stem structure.

As used herein, a "hairpin structure" or a "stem-loop structure" means a structure consisting of one stem structure and one loop structure (one set of a stem structure and a loop structure).

As used herein, the term "complementary" means the relationship which enables nucleobases to form what is called a Watson-Crick base pair (natural base pair) or a non-Watson-Crick base pair (Hoogsteen base pair or the like) via hydrogen bonding. In the present invention, a base sequence is acceptable, subject to having a complementarity of at least 80%, preferably at least 90% (for example, 95%, 96%, 97%, 98%, or 99% or more). The complementarity of a base sequence can be determined using the BLAST program or the like.

As used herein, a "nucleic acid primer" (simply abbreviated as a "primer" in some cases) is a nucleic acid molecule that specifically binds to part of a nucleic acid sequence, and provides a starting point of nucleic acid synthesis reaction by a polymerase enzyme. A nucleic acid primer is usually single-stranded, and composed of a base sequence complementary to part of a target nucleic acid sequence to be used as a template in polymerase reaction. A nucleic acid strand that can be used as a primer is, for example, 6 to 100 bases, 10 to 70 bases, 12 to 50 bases, 15 to 40 bases, 18 to 30 bases, or 20 to 25 bases in length.

As used herein, a "plurality" refers to any integer of 2 or more, for example, 2 to 10¹⁰, 2 to 10⁹, 2 to 10⁸, 2 to 10⁷, 2 to 10⁶, 2 to 10⁵, 2 to 10⁴, 2 to 1,000, 2 to 900, 2 to 800, 2 to 700, 2 to 600, 2 to 500, 2 to 400, 2 to 300, 2 to 200, 2 to 100, 2 to 90, 2 to 80, 2 to 70, 2 to 60, 2 to 50, 2 to 40, 2 to 30, 2 to 20, 2 to 10,2 to 9, 2 to 8, 2 to 7, 2 to 6, 2 to 5, 2 to 4, 2 to 3, or 2.

### <Ds/Pa'-containing single-stranded nucleic acid molecule library>

In one aspect of the present invention, a single-stranded nucleic acid molecule library comprising a plurality of single-stranded nucleic acid molecules is provided. Herein, a single-stranded nucleic acid molecule library "comprising a plurality of single-stranded nucleic acid molecules" means that the number of the kinds of the single-stranded nucleic acid molecules constituting a single-stranded nucleic acid molecule library is 2 or more on the basis of the base sequence. In the present aspect, the plurality of single-stranded nucleic acid molecules comprises a Ds/Pa'-containing single-stranded nucleic acid molecule. Hence, the single-stranded nucleic acid molecule library of the present aspect is referred to as a "Ds/Pa'-containing single-stranded nucleic acid molecule library".

As used herein, the "Ds/Pa'-containing single-stranded nucleic acid molecule" is a single-stranded nucleic acid molecule comprising the above-described Ds base and Pa' base, one or more each, in addition to a natural base. The Ds/Pa'-containing single-stranded nucleic acid molecule consists of a variable region, or comprises a variable region as an essential region. In addition, the Ds/Pa'-containing single-stranded nucleic acid molecule may comprise, in addition to the variable region, a pair of primer-binding regions at the 5' end and 3' end thereof. For example, the Ds/Pa'-containing single-stranded nucleic acid molecule comprises a primer-binding region on the 5' end side, a variable region, and a primer-binding region on the 3' end side from the 5' end side in this order.

As used herein, the "primer-binding region" is a region to which a primer binds in a single-stranded nucleic acid molecule, in which the primer is used in a nucleic acid amplification reaction for amplifying a single-stranded nucleic acid molecule constituting a single-stranded nucleic acid molecule library. The base sequence constituting the primer-binding region is preferably a sequence common among a plurality of single-stranded nucleic acid molecules constituting a single-stranded nucleic acid molecule library. In principle, the primer-binding region is located at each of the 5' end and 3' end of a single-stranded nucleic acid molecule, and thus, a pair of primer-binding regions at the 5' end and 3' end are referred to as a "pair of primer-binding regions". The base sequences constituting a pair of primer-binding regions and the length of each primer-binding region are not particularly limited, and may be designed on the basis of, for example, amplification conditions for a nucleic acid amplification method. For example, the length of each primer-binding region may be 2 to 150 bases, 6 to 100 bases, 10 to 70 bases, 12 to 50 bases, 15 to 40 bases, 18 to 30 bases, or 20 to 25 bases. A primer-binding region composed of only two bases is known as a minimal primer, and can be utilized also in the present invention. In this regard, a single-stranded nucleic acid molecule library according to the present invention optionally does not comprise a primer-binding region. An aptamer comprising no primer-binding region is known also as a "Primer free DNA aptamer" or the like. Before an amplification step, a primer-binding sequence is linked to both ends of an aptamer sequence (using T4 RNA Ligase 1, thermotolerant RNA ligase, or the like), and before a complex formation step, the primer-binding sequence is removed by a cleavage enzyme, such as a restriction enzyme.

As used herein, a "variable region" refers to a region consisting of a base sequence mutually different among a plurality of single-stranded nucleic acid molecules constituting a single-stranded nucleic acid molecule library. In the present aspect, the variable region comprises a natural base(s) (for example, the A base, the G base, the T base/the U base, and/or the C base), an unnatural base Ds shown in the above-described general formula (I), and an unnatural base Pa' shown in the above-described general formula (II). All or part of the natural bases, the Ds base, and/or the Pa' base comprises, in at least a part thereof, a sequence located at mutually different positions, for example, a sequence located randomly.

The number of the Ds bases and the number of the Pa' bases in a variable region are not particularly limited, subject to being one or more each. For example, the number of the Ds bases and the number of the Pa' bases are each independently 1 to 15, 2 to 12, or 3 to 10, may each be 9 or less, 8 or less, 7 or less, 6 or less, or 5 or less, and are each preferably 1 to 4, 1 to 3, or 1 to 2.

In addition, in a case where a base is located randomly in a variable region, a base introduced into each position is determined probabilistically, and thus, the number of bases of each kind is expressed as an average value. In this case, the number of the Ds bases and the number of the Pa' bases are each not particularly limited, subject to being at least 0.1 to 1 on average. For example, the number of the Ds bases and the number of the Pa' bases are each independently 0.1 to 15, 0.5 to 12, or 1 to 10 on average, may each be 9 or less, 8 or less, 7 or less, 6 or less, or 5 or less on average, and are each preferably 1 to 4, 1 to 3, or 1 to 2 on average.

In one embodiment, the variable region comprises one to three Ds bases, and comprises one to two Pa' bases on average. In addition, in another embodiment, the variable region comprises one to three Pa' bases, and comprises one to two Ds bases on average.

In the variable region, the Ds base and/or the Pa' base may be located at a specified position(s), or may be located at random position(s). For example, both the Ds base and the Pa' base may be located at specified positions, or may be located at random positions. In addition, the Ds base may be located at a specified position, and the Pa' base may be located at a random position. Alternatively, the Pa' base may be located at a specified position, and the Ds base may be located at a random position. Locating the Ds base and/or the Pa' base at a specified position(s) gives an advantage in that the position of an unnatural base can be easily determined in the below-described method of sequencing a base sequence.

In one embodiment, a variable region in the Ds/Pa'-containing single-stranded nucleic acid molecule comprises a random sequence consisting of the Ds base at one or more specified positions and besides, the Pa' base and a natural base(s) (for example, the A base, the G base, the T base/the U base, and/or the C base) except at the one or more specified positions. Alternatively, a variable region in the Ds/Pa'-containing single-stranded nucleic acid molecule comprises a random sequence consisting of the Pa' base at one or more specified positions and besides, the Ds base and a natural base(s) (for example, the A base, the G base, the T base/the U base, and/or the C base) except the one or more specified positions.

In a further embodiment, among a plurality of Ds/Pa'-containing single-stranded nucleic acid molecules constituting a single-stranded nucleic acid molecule library of the present aspect, a specified position at which the Ds base or the Pa' base is located in a variable region is mutually different. In the below-described Examples, a group of single-stranded nucleic acid molecules among which a base at this specified position is the same, and in which a random base is introduced at a position other than the specified position is referred to as a sublibrary.

### <Ds/Px-containing single-stranded nucleic acid molecule library>

In one aspect of the present invention, a single-stranded nucleic acid molecule library comprising a plurality of single-stranded nucleic acid molecules is provided. In the present aspect, the plurality of single-stranded nucleic acid molecules comprise a Ds/Px-containing single-stranded nucleic acid molecule. Hence, the single-stranded nucleic acid molecule library of the present aspect is referred to as a "Ds/Px-containing single-stranded nucleic acid molecule library".

As used herein, the "Ds/Px-containing single-stranded nucleic acid molecule" is a single-stranded nucleic acid molecule comprising the above-described Ds base and Px base, one or more each, in addition to a natural base. The Ds/Px-containing single-stranded nucleic acid molecule comprises a variable region as an essential region. In addition, the Ds/Px-containing single-stranded nucleic acid molecule may comprise, in addition to the variable region, a pair of primer-binding regions at the 5' end and 3' end thereof. For example, the Ds/Px-containing single-stranded nucleic acid molecule comprises a primer-binding region on the 5' end side, a variable region, and a primer-binding region on the 3' end side from the 5' end side in this order.

In the present aspect, the variable region comprises a natural base(s) (for example, the A base, the G base, the T base/the U base, and/or the C base), an unnatural base Ds shown in the above-described general formula (I), and an unnatural base Px shown in the above-described general formula (III). All or part of the natural base(s), the Ds base, and/or the Px base comprise(s), in at least part thereof, a sequence located at mutually different positions, for example, a sequence located randomly.

In the present aspect, the number of the Ds bases and the number of the Px bases in a variable region are not particularly limited, subject to being one or more each. For example, the number of the Ds bases and the number of the Px bases are each independently 1 to 15, 2 to 12, or 3 to 10, may each be 9 or less, 8 or less, 7 or less, 6 or less, or 5 or less, and are each preferably 1 to 4, 1 to 3, or 1 to 2.

In addition, in a case where a base is located randomly in a variable region, the number of the Ds bases and the number of the Px bases are each not particularly limited, subject to being at least 0.1 to 1 on average. For example, the number of the Ds bases and the number of the Px bases are each independently 0.1 to 15, 0.5 to 12, or 1 to 10 on average, may each be 9 or less, 8 or less, 7 or less, 6 or less, or 5 or less on average, and are each preferably 1 to 4, 1 to 3, or 1 to 2 on average.

In one embodiment, the variable region comprises one to three Ds bases, and comprises one to two Px bases on average. In addition, in another embodiment, the variable region comprises one to three Px bases, and comprises one to two Ds bases on average.

In the variable region, the Ds base and/or the Px base may be located at a specified position(s), or may be located at random position(s). For example, both the Ds base and the Px base may be located at specified positions, or may be located at random positions. In addition, the Ds base may be located at a specified position, and the Px base may be located at a random position. Alternatively, the Px base may be located at a specified position, and the Ds base may be located at a random position. Locating the Ds base and/or the Px base at a specified position(s) gives an advantage in that the position of an unnatural base can be easily determined in the below-described method of sequencing a base sequence.

In one embodiment, a variable region in the Ds/Px-containing single-stranded nucleic acid molecule comprises a random sequence consisting of the Ds base at one or more specified positions (for example, position(s) 1 to 10, 2 to 9, 3 to 8, 4 to 7, or 5 to 6) and besides, the Px base and a natural base(s) (for example, the A base, the G base, the T base/the U base, and/or the C base) except at the one or more specified positions. Alternatively, a variable region in the Ds/Px-containing single-stranded nucleic acid molecule comprises a random sequence consisting of the Px base at one or more specified positions (for example, position(s) 1 to 10, 2 to 9, 3 to 8, 4 to 7, or 5 to 6) and besides, the Ds base and a natural base(s) (for example, the A base, the G base, the T base/the U base, and/or the C base) except at the one or more specified positions.

In a further embodiment, among a plurality of Ds/Px-containing single-stranded nucleic acid molecules constituting a single-stranded nucleic acid molecule library of the present aspect, a specified position at which the Ds base or the Px base is located in a variable region is mutually different.

In a Ds/Pa'-containing single-stranded nucleic acid molecule library or Ds/Px-containing single-stranded nucleic acid molecule library according to the present invention, the length of the variable region is not limited, and may be, for example, 5 bases or more, 10 bases or more, 12 bases or more, 14 bases or more, 16 bases or more, 18 bases or more, 20 bases or more, 30 bases or more, 40 bases or more, 50 bases or more, or 60 bases or more, and/or 500 bases or less, 400 bases or less, 300 bases or less, 200 bases or less, 100 bases or less, 90 bases or less, or 80 bases or less in length.

In one embodiment, in a Ds/Pa'-containing single-stranded nucleic acid molecule library or Ds/Px-containing single-stranded nucleic acid molecule library according to the present invention, each of a plurality of single-stranded nucleic acid molecules comprises a 5'-wing region and/or a 3'-wing region. As used herein, the "5'-wing region" is a region consisting of natural bases and located on the 5' end side of a variable region, or a region consisting of natural bases and located between a primer-binding region located at the 5' end and a variable region. In addition, the "3'-wing region" as used herein is a region consisting of natural bases and located on the 3' end side of a variable region, or a region consisting of natural bases and located between a variable region and a primer-binding region located at the 3' end.

The length of the 5'-wing region and/or the 3'-wing region is not particularly limited, and may be, for example, 2 bases or more, 4 bases or more, 6 bases or more, 8 bases or more, 10 bases or more, 12 bases or more, or 15 bases or more, and/or 80 bases or less, 60 bases or less, 40 bases or less, 30 bases or less, 25 bases or less, 20 bases or less, or 18 bases or less.

In one embodiment, the 5'-wing region and/or the 3'-wing region consist(s) of a random sequence composed of natural bases. The length of the random sequence may be, for example, 1 to 30 bases, 2 to 25 bases, 3 to 20 bases, 5 to 15 bases, or 8 to 12 bases.

In one embodiment, the 5'-wing region and/or the 3'-wing region comprise a pair of complementary sequences which are complementary to each other and are capable of forming a stem structure. The length of the stem structure may be, for example, 1 bp or more, 2 bp or more, 3 bp or more, 4 bp or more, or 5 bp or more, and/or 30 bp or less, 25 bp or less, 20 bp or less, 15 bp or less, 10 bp or less, or 8 bp or less. Examples of the range include 3 to 20 bp (3 to 20 bases in length).

In a further embodiment, the complementary sequence capable of forming a stem structure comprise a non-Watson-Crick-type base pair, and/or an insertion sequence and/or a deletion of one or more bases. The non-Watson-Crick-type base pair may be an arbitrary base pair other than the Watson-Crick-type base pair, or may be, for example, a G-T base pair. In addition, the insertion sequence can form a bulge structure or a bulge-out structure. The bulge structure or the bulge-out structure may be composed of natural bases, or may comprise an unnatural base (for example, a Ds base, a Pa' base, and/or a Px base).

### <Method of producing single-stranded nucleic acid molecule library>

In one aspect of the present invention, a method of producing a single-stranded nucleic acid molecule library is provided. A method of producing a single-stranded nucleic acid molecule library according to the present aspect comprises an amplification step as an essential step, and can produce a Ds/Px-containing single-stranded nucleic acid molecule library from a Ds/Pa'-containing single-stranded nucleic acid molecule library (Embodiment A), and produce a Ds/Pa'-containing single-stranded nucleic acid molecule library from a Ds/Px-containing single-stranded nucleic acid molecule library (Embodiment B).

In Embodiment A described above, an amplification step in the production method according to the present aspect can yield a Ds/Px-containing single-stranded nucleic acid molecule library in the form of an amplification product through amplifying a single-stranded nucleic acid molecule by a nucleic acid amplification method, using, as a template, any Ds/Pa'-containing single-stranded nucleic acid molecule library described in <Ds/Pa'-containing single-stranded nucleic acid molecule library> above, and using, as a substrate, natural nucleotides, Ds-containing nucleotides, and Px-containing nucleotides.

In addition, in Embodiment B, an amplification step in the production method according to the present aspect can yield a Ds/Pa'-containing single-stranded nucleic acid molecule library in the form of an amplification product through amplifying a single-stranded nucleic acid molecule by a nucleic acid amplification method, using, as a template, any Ds/Px-containing single-stranded nucleic acid molecule library described in <Ds/Px-containing single-stranded nucleic acid molecule library> above, and using, as a substrate, natural nucleotides, Ds-containing nucleotides, and Pa'-containing nucleotides.

A natural nucleotide used in the production method according to the present aspect is a nucleotide comprising adenine, cytosine, guanine, thymine, and/or uracil, which are natural bases. For example, a combination of deoxyribonucleotides comprising adenine, cytosine, guanine, and thymine (the combination is referred to as "dNTP" in some cases) can be used.

As used herein, the "Ds-containing nucleotide", "Px-containing nucleotide", or "Pa'-containing nucleotide" refers to a nucleotide comprising a sugar portion, such as deoxyribose or ribose, and Ds, Px, or Pa' respectively as a nucleobase.

In the present invention, an arbitrary known nucleic acid amplification method can be used as a specific amplification method to be used for the amplification step. As used herein, the "nucleic acid amplification method" refers to a method of amplifying a nucleic acid with a nucleic acid polymerase, using a primer, if desired. Examples include PCR, NASBA (Nucleic Acid Sequence-Based Amplification), ICAN (Isothermal and Chimeric primer-initiated Amplification of Nucleic acids) (an isothermal gene amplification method), LAMP (Loop-Mediated Isothermal Amplification) (registered trademark), and RCA (Rolling-Cycle Amplification). A nucleic acid amplification method to be used in the present invention is not limited, and is preferably PCR in which a thermotolerant DNA polymerase is usually used. Each nucleic acid amplification method is known in the art, and may be performed with reference to conditions described in various protocols. Examples of a collection of protocols include: Green, MR and Sambrook, J, (2012), Domingues L. (2017) PCR: Methods and Protocols, *Methods in Molecular Biology,* Humana Press; and Park DJ, (2010) PCR Protocols, *Methods in Molecular Biology,* Third Edition, Humana Press. In addition, kits for nucleic acid amplification are commercially available from life science manufacturers, and such kits can be utilized. In this case, conditions and the like for a nucleic acid amplification method may be in accordance with an attached protocol or a protocol recommended by each manufacturer. In the amplification step, several amplification cycles (for example,1 to 40 cycles, 2 to 30 cycles, 3 to 20 cycles, 4 to 15 cycles, or 5 to 10 cycles) may be performed using, for example, PCR.

The production method of the present aspect makes it possible that a Ds/Px-containing single-stranded nucleic acid molecule library to be used for ExSELEX is produced from a chemically synthesized Ds/Pa'-containing single-stranded nucleic acid molecule library. In addition, the production method makes it possible that a Ds/Pa'-containing single-stranded nucleic acid molecule library stable even under basic conditions is produced from a Ds/Px-containing single-stranded nucleic acid molecule library concentrated in ExSELEX.

### <Method of producing nucleic acid aptamer>

In one aspect of the present invention, a method of producing a nucleic acid aptamer is provided. A specific step in the method of producing a nucleic acid aptamer according to the present aspect differs among the following three embodiments: an embodiment optionally not comprising a library synthesis step (Embodiment 1); an embodiment comprising a library synthesis step, wherein, from a Ds/Pa'-containing single-stranded nucleic acid molecule library obtained in the library synthesis step, a Ds/Px-containing single-stranded nucleic acid molecule library is amplified without undergoing complex formation with a target substance (Embodiment 2); and an embodiment comprising a library synthesis step, wherein a Ds/Pa'-containing single-stranded nucleic acid molecule library obtained in the library synthesis step is used for complex formation with a target substance, and then, a Ds/Px-containing single-stranded nucleic acid molecule library is amplified (Embodiment 3). Thus, each embodiment will be described below.

### <Method of producing nucleic acid aptamer: Embodiment 1>

The production method of the present embodiment comprises a complex formation step, a complex collection step, and an amplification step as essential steps, or comprises a complex formation step and a complex collection step as essential steps. In the embodiment comprising no amplification step, a Ds/Px-containing single-stranded nucleic acid molecule after the complex collection step is not amplified, and, for example, the complex formation step and the complex collection step are repeated a plurality of times, whereby a final aptamer of one or more molecules can be obtained. Even such a one-molecule aptamer can be sequenced, using a one-molecule sequencing technology, such as nanopore sequencing. The constitution of each step will be specifically described below.

### (Complex formation step)

The complex formation step is a step of mixing any Ds/Px-containing single-stranded nucleic acid molecule library described in <Ds/Px-containing single-stranded nucleic acid molecule library> above with a target substance in a solution to allow for formation of a complex of the Ds/Px-containing single-stranded nucleic acid molecule in the Ds/Px-containing single-stranded nucleic acid molecule library and the target substance.

The present step can be performed, for example, by mixing the target substance and the Ds/Px-containing single-stranded nucleic acid molecule library in the solution. The mixing ratio of the single-stranded nucleic acid molecule library to the target substance is, for example, 100:1 to 1:100, preferably 1:1. The present step is not particularly limited to any condition, and can be performed, for example, by incubation in a temperature range (at a temperature of 15 to 60°C or 25 to 40°C) comprising ordinary temperature for 1 second to several days, several seconds to several hours, for example, 10 seconds to 6 hours, or 5 minutes to 3 hours.

A solution to be used in the present step is not particularly limited to any kind or nature, subject to being a solution that can form a complex of a nucleic acid and a target substance. The solution is preferably water or an aqueous solution. In the case of an aqueous solution, the pH range thereof may be from 5.0 to 9.0, preferably from 6.0 to 8.0, more preferably from 6.5 to 7.6. In addition, the salt concentration, as a final concentration, may be in the range of from 20 to 500 mM, preferably from 50 to 300 mM, more preferably from 90 to 180 mM. The aqueous solution may be, for example, a phosphate buffer, citric acid-phosphate buffer, tris-hydrochloric acid buffer, or a pH buffer solution, such as a HEPES buffer. In addition, the solution may further comprise a reducing agent or a surfactant, if desired. A complex formed in the present step may be washed before the subsequent complex collection step.

### (Complex collection step)

The complex collection step is a step of collecting a complex of a Ds/Px-containing single-stranded nucleic acid molecule and a target substance, in which the complex is formed in the complex formation step. A collection method may be a method that can separate a complex from an unbound Ds/Px-containing single-stranded nucleic acid molecule. The complex may be collected, for example, by sedimentation, aggregation, precipitation, filtration, centrifugation, electrophoresis, or magnetic force. For example, after electrophoresis performed using acrylamide gel, an area corresponding to a complex can be cut out from a band shifted to the higher molecular weight side than a target substance unbound to a nucleic acid and/or a nucleic acid unbound to a target substance. In addition, a nucleic acid strand bound to a target substance may be collected using a binding means, such as an antibody against the target substance.

### (Amplification step)

The amplification step is a step of amplifying a single-stranded nucleic acid molecule comprised in the complex after the complex collection step by a nucleic acid amplification method, using natural nucleotides, Ds-containing nucleotide, and Px-containing nucleotides as a substrate. The present step produces, in the form of an amplification product, a nucleic acid aptamer consisting of a Ds/Px-containing single-stranded nucleic acid molecule. As an amplification method to be used for the amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed.

In a further embodiment, the production method of the present aspect further comprises a repeating step of repeating the complex formation step to the amplification step once or more than once, using the Ds/Px-containing single-stranded nucleic acid molecule after the amplification step as a new Ds/Px-containing single-stranded nucleic acid molecule library. The larger the number of repetitions, the higher the ratio of a single-stranded nucleic acid molecule having higher ability for binding to a target substance. Thus, the upper limit of the number of repetitions is not particularly limited. The number of repetitions may be, for example, 1 to 10, 2 to 9, 3 to 8 or more, 4 to 7, or 5 to 6.

### <Method of producing nucleic acid aptamer: Embodiment 2>

The production method of the present embodiment comprises a library synthesis step, a first amplification step, a complex formation step, a complex collection step, and a second amplification step as essential steps. The constitution of each step will be specifically described below.

### (Library synthesis step)

The library synthesis step is a step of synthesizing any Ds/Pa'-containing single-stranded nucleic acid molecule library described in <Ds/Pa'-containing single-stranded nucleic acid molecule library> above by reacting natural nucleic acids, Ds-containing nucleic acids, and Pa'-containing nucleic acids. As used herein, the "Ds-containing nucleic acid" is a nucleic acid such as a nucleoside or nucleotide containing a Ds base (for example, amidite or phosphoramidite). In addition, the "Pa'-containing nucleic acid" is a nucleic acid such as a nucleoside or nucleotide containing a Pa' base (for example, amidite or phosphoramidite).

A synthesis method in the present step is not particularly limited. A method known in the art may be used. For example, a Ds/Pa'-containing single-stranded nucleic acid molecule library can be chemically synthesized by synthesizing a Ds base and a Pa' base in accordance with a method described in any literature above, then synthesizing a Ds-containing nucleic acid and a Pa'-containing nucleic acid, and further synthesizing a nucleic acid strand in accordance with a known solid phase synthesis method. For a method for chemically synthesizing a nucleic acid, see, for example, Current Protocols in Nucleic Acid Chemistry, Volume 1, Section 3. A Ds/Pa'-containing single-stranded nucleic acid molecule library after the chemical synthesis is preferably purified using a method known in the art, before use. Examples of a purifying method include a gel purification method, affinity column purification method, and HPLC method. In addition, a Ds-containing nucleic acid and a Pa'-containing nucleic acid can be enzymatically synthesized using Ds base- and Pa' base-containing nucleotides as a substrate, and using a known enzymatic DNA synthesis (EDS) technology.

### (First amplification step)

The first amplification step is a step of amplifying a single-stranded nucleic acid molecule using, as a template, the Ds/Pa'-containing single-stranded nucleic acid molecule library after the library synthesis step, and using, as a substrate, natural nucleotides, Ds-containing nucleotides, and Px-containing nucleotides. The present step yields a Ds/Px-containing single-stranded nucleic acid molecule in the form of an amplification product. As an amplification method to be used for the first amplification step, an arbitrary known nucleic acid amplification method can be used. Using, for example, a PCR method, several amplification cycles (for example,1 to 40 cycles, 2 to 30 cycles, 3 to 20 cycles, 4 to 15 cycles, or 5 to 10 cycles) may be performed.

### (Complex formation step)

The complex formation step is a step of mixing the Ds/Px-containing single-stranded nucleic acid molecule after the first amplification step with a target substance in a solution to allow for formation of a complex between the Ds/Px-containing single-stranded nucleic acid molecule and the target substance. The present step can be performed in accordance with the method described in the above-described complex formation step.

### (Complex collection step)

The complex collection step is a step of collecting the complex after the complex formation step. The present step can be performed in accordance with the method described in the above-described complex collection step.

### (Second amplification step)

The second amplification step is a step of amplifying a single-stranded nucleic acid molecule comprised in the complex after the complex collection step by a nucleic acid amplification method using natural nucleotides, Ds-containing nucleotide, and Px-containing nucleotides as a substrate. The present step produces a nucleic acid aptamer consisting of a Ds/Px-containing single-stranded nucleic acid molecule. As an amplification method to be used for the second amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed.

In a further embodiment, the production method of the present aspect further comprises a repeating step of repeating the complex formation step to the second amplification step once or more than once, using the Ds/Px-containing single-stranded nucleic acid molecule after the second amplification step. The larger the number of repetitions, the higher the ratio of a single-stranded nucleic acid molecule in a library, in which the single-stranded nucleic acid molecule has higher ability for binding to a target substance. Thus, the upper limit of the number of repetitions is not particularly limited. The number of repetitions may be, for example, 1 to 10, 2 to 9, 3 to 8 or more, 4 to 7, or 5 to 6.

### <Method of producing nucleic acid aptamer: Embodiment 3>

In the present embodiment, the production method of the present aspect comprises a library synthesis step, a first complex formation step, a first complex collection step, a first amplification step, a second complex formation step, a second complex collection step, and a second amplification step as essential steps.

The constitution of the library synthesis step in the present embodiment is in accordance with the library synthesis step in Embodiment 2, and thus, detailed description is omitted here. The first complex collection step is a step of collecting the complex after the first complex formation step, and can be performed in accordance with the method described in the above-described complex collection step. The second complex formation step is a step of mixing the Ds/Px-containing single-stranded nucleic acid molecule after the first amplification step with a target substance in a solution to allow for formation of a complex between the Ds/Px-containing single-stranded nucleic acid molecule and the target substance. The second complex formation step can be performed in accordance with the method described in the above-described complex formation step. The second complex collection step is a step of collecting the complex after the second complex formation step, and can be performed in accordance with the method described in the above-described complex collection step. The constitution of each step other the above-described steps will be specifically described below.

### (First complex formation step)

The first complex formation step is a step of mixing the Ds/Pa'-containing single-stranded nucleic acid molecule library after the library synthesis step with a target substance in a solution to allow for formation of a complex of the Ds/Pa'-containing single-stranded nucleic acid molecule in the Ds/Pa'-containing single-stranded nucleic acid molecule library and the target substance. The present step can be performed in accordance with the method described in the above-described complex formation step.

### (First amplification step)

The first amplification step is a step of amplifying a single-stranded nucleic acid molecule using, as a template, the Ds/Pa'-containing single-stranded nucleic acid molecule after the first complex collection step, and using, as a substrate, natural nucleotides, Ds-containing nucleotides, and Px-containing nucleotides. The present step yields a Ds/Px-containing single-stranded nucleic acid molecule in the form of an amplification product. As an amplification method to be used for the first amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed.

### (Second amplification step)

The second amplification step is a step of amplifying a single-stranded nucleic acid molecule comprised in the complex after the second complex collection step by a nucleic acid amplification method using natural nucleotides, Ds-containing nucleotide, and Px-containing nucleotides as a substrate. The present step produces a nucleic acid aptamer consisting of a Ds/Px-containing single-stranded nucleic acid molecule. As an amplification method to be used for the second amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed.

In a further embodiment, the production method of the present aspect further comprises a repeating step of repeating the second complex formation step to the second amplification step once or more than once, using the Ds/Px-containing single-stranded nucleic acid molecule after the second amplification step. The larger the number of repetitions, the higher the ratio of a single-stranded nucleic acid molecule in a library, in which the single-stranded nucleic acid molecule has higher ability for binding to a target substance. Thus, the upper limit of the number of repetitions is not particularly limited. The number of repetitions may be, for example, 1 to 10, 2 to 9, 3 to 8 or more, 4 to 7, or 5 to 6.

### <Method of determining base sequence>

In one aspect of the present invention, a method of determining the base sequence of single-stranded nucleic acid molecule containing an unnatural base (the method is hereinafter referred to as a "sequencing method") is provided. A sequencing method according to the present invention comprises a first amplification step and a sequencing step as essential steps.

A single-stranded nucleic acid molecule containing an unnatural base the base sequence of which molecule is to be determined by a sequencing method according to the present invention comprises a pair of primer-binding regions at the 5' end and 3' end thereof, and a central region between the pair of primer-binding regions. In this regard, even a nucleic acid aptamer not comprising a pair of primer-binding regions at the 5' end and 3' end of the central region can be sequenced, using the method of the present aspect, by linking primer-binding regions to the 5' end and 3' end by ligation or the like (herein referred to as a "linking step"). The central region includes a Ds base and a Px base. The number of the Ds bases in the central region may be 1 or more, for example, 1 to 15, 1 to 12, or 1 to 10, 9 or less, 8 or less, 7 or less, 6 or less, or 5 or less, and is preferably 1 to 4, 1 to 3, or 1 to 2. In addition, the number of the Px bases in the central region is 1 or more, for example, 1 to 15, 1 to 12, or 1 to 10, may be 9 or less, 8 or less, 7 or less, 6 or less, or 5 or less, and is preferably 1 to 4, 1 to 3, or 1 to 2. For example, the central region comprises one to three Ds bases, and comprises one to two Px bases. The single-stranded nucleic acid molecule containing an unnatural base may be, for example, any Ds/Px-containing single-stranded nucleic acid molecule library described in <Ds/Px-containing single-stranded nucleic acid molecule library> above, or may be a nucleic acid aptamer consisting of a Ds/Px-containing single-stranded nucleic acid molecule produced by any method described in <Method of producing nucleic acid aptamer> above.

### (First amplification step)

The first amplification step is a step of amplifying the single-stranded nucleic acid molecule containing an unnatural base by a nucleic acid amplification method using natural nucleotides as a substrate and using a primer pair which binds to the base sequence of the pair of primer-binding regions. As an amplification method to be used for the first amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed. Through the present step, an unnatural base in the single-stranded nucleic acid molecule containing an unnatural base is substituted with a natural base after the amplification. For example, in the case of Ds, Px, and Pa', the base is substituted with A or T.

### (Sequencing step)

The sequencing step is a step of sequencing the base sequence of an amplification product composed only of natural nucleotides which is obtained after the first amplification step. A sequencing method to be used in the present step may be performed by a sequencing method known in the art. Examples of the sequencing method include the Sanger's method (dideoxy method) and a next-generation sequencing method. The next-generation sequencing method may be a second-generation sequencing method or a third-generation sequencing method. Examples of the second-generation sequencing method include a pyrosequencing method, synthetic sequencing method, ligation sequencing method, DNBSEQ method, which is a DNA nanoball-based sequencing technology, and ion semiconductor sequencing method. Examples of the third-generation sequencing method include a sequencing method including using a single-molecule sequencer or a nanopore sequencer. These methods are known in the art. Not only various collections of protocols but also the methods disclosed in the Websites of various companies can be used as references. In sequencing in the present step, it is preferable to use a next-generation sequencing method that can determine the base sequences of a plurality of nucleic acid molecules simultaneously. The present step makes it possible that both the A and T bases are read, whereby the positions of Ds, Px, and Pa' in a single-stranded nucleic acid molecule containing an unnatural base before the above-described amplification step are identified.

A sequencing method according to the present invention can comprise, as an optional step, each step described in Embodiment 1 and Embodiment 2 below.

### <Sequencing method: Embodiment 1>

In the present Embodiment, a sequencing method according to the present invention comprises a second amplification step, a cleavage step, and a Px position determining step in addition to the above-described essential steps. In a further embodiment of the present embodiment, a sequencing method according to the present invention further comprises a Ds/Px position determining step and a Ds position identifying step.

### (Second amplification step)

The second amplification step is a step of amplifying the single-stranded nucleic acid molecule containing an unnatural base by a nucleic acid amplification method using, as a substrate, natural nucleotides, Ds-containing nucleotides, and Px-containing nucleotides, and using a primer pair which binds to the base sequences of a pair of primer-binding regions, and which are labeled with different labeling molecules (for example, labeling molecules that can be distinguished on the basis of the wavelength of fluorescence). As an amplification method to be used for the second amplification step, an arbitrary known nucleic acid amplification method can be used. Using, for example, a PCR method, several amplification cycles (for example, 1 to 40 cycles, 2 to 30 cycles, 3 to 20 cycles, 4 to 15 cycles, or 5 to 10 cycles) may be performed. Through the present step, a single-stranded nucleic acid molecule containing an unnatural base and a complementary strand thereof are labeled with different labeling molecules.

### (Cleavage step)

The cleavage step is a step of completely or partially cleaving a single-stranded nucleic acid molecule containing an unnatural base and a complementary strand thereof bound to different labeling molecules which are amplified after the second amplification step at the position(s) of the Px base. Examples of a method of cleaving a nucleic acid molecule at the position of the Px base include a method of treatment under basic conditions. The basic conditions may be weakly alkaline or strongly alkaline, for example, a pH of 8 to 14, a pH of 9 to 13, or a pH of 10 to 12. The treatment conditions may be, for example, at 10 to 95°C, 20 to 80°C, 30 to 60°C, or 30 to 40°C for several minutes to several hours, for example, 5 minutes to 6 hours, 10 minutes to 3 hours, or 20 minutes to 1 hour.

### (Px position determining step)

The Px position determining step is a step of determining the position(s) of the Px base in a single-stranded nucleic acid molecule containing an unnatural base and the complementary strand thereof by determining the size of single-stranded nucleic acid fragments derived from the single-stranded nucleic acid molecule containing an unnatural base and the complementary strand thereof after the cleavage step based on detection of the labeling molecules. In the present step, the size of a nucleic acid strand can be determined, for example, using agarose gel electrophoresis or polyacrylamide gel electrophoresis. A single-stranded nucleic acid molecule containing an unnatural base and a single-stranded nucleic acid fragment derived from a complementary strand thereof may be separately detected by detecting labeling molecules bound to the primers, for example, on the basis of the different wavelengths of fluorescence.

### (Ds/Px position determining step)

The Ds/Px position determining step is a step of determining the base positions where both of the natural bases, the A base and the T base, are sequenced in the base sequence sequenced in the sequencing step as the positions of the Ds base or the Px base.

### (Ds position identifying step)

The Ds position identifying step is a step of identifying, as the position(s) of the Ds base, the position(s) other than the position(s) determined as the position(s) of the Px base in the Px position determining step, among the positions of the Ds base or the Px base determined in the Ds/Px position determining step.

### <Sequencing method: Embodiment 2>

In the present embodiment, a sequencing method according to the present invention comprises a Ds/Px position determining step, a synthesis step, a complex formation step, a measurement step, and a selection step in addition to the above-described essential steps.

### (Ds/Px position determining step)

The Ds/Px position determining step is a step of determining the base positions where both of the natural bases, the A base and the T base, are sequenced in the base sequence sequenced in the sequencing step as the positions of the Ds base or the Px base.

### (Synthesis step)

The synthesis step is a step of synthesizing a plurality of single-stranded nucleic acid molecule which comprise the Ds base and/or the Px base at the positions of the Ds base or the Px base determined in the Ds/Px position determining step in multiple or all combinations, wherein the sequence of the single-stranded nucleic acid molecule except the position of the Ds base or the Px base consists of the base sequence sequenced in the sequencing step. In the synthesis step, the synthesis method described in the above-described library synthesis step, or the like can be used.

### (Complex formation step)

The complex formation step is a step of mixing the plurality of single-stranded nucleic acid molecules synthesized in the synthesis step with a target substance to allow for formation of a complex between the plurality of single-stranded nucleic acid molecules and the target substance. The present step can be performed in accordance with the method described in the above-described complex formation step.

### (Measurement step)

The measurement step is a step of measuring complex formation efficiency after the complex formation step. The measurement method in the present step is not particularly limited, subject to detecting binding between a single-stranded nucleic acid molecule and a target substance to measure the level of the binding. A known method may be used. For example, SPR, a quartz crystal microbalance method, nephelometric method, colorimetric method, fluorescence method, EMSA, ELISA, or sandwich method can be utilized.

### (Selection step)

The measurement step is a step of selecting the base sequence of the single-stranded nucleic acid molecule(s) which shows a higher or the highest complex formation efficiency among the plurality of single-stranded nucleic acid molecules as the base sequence of the single-stranded nucleic acid molecule containing an unnatural base after the measurement step. The base sequence selected in the present step is determined in the form of the base sequence of a single-stranded nucleic acid molecule containing an unnatural base.

### <Nucleic acid aptamer>

In one aspect of the present invention, a nucleic acid aptamer is provided.

A nucleic acid aptamer of the present invention comprises at least one of any above-described Ds base, any above-described Pa' base, and/or any above-described Px base.

In a nucleic acid aptamer of the present invention, the number of the Ds bases, the Pa' bases, and/or the Px bases is not limited, subject to being 1 or more, and may be, for example, 1 or more, 2 or more, 3 or more, or 4 or more, and/or may be 20 or less, 10 or less, 8 or less, 7 or less, 6 or less, or 5 or less. For example, a nucleic acid aptamer of the present invention comprises one to nine, one to six, or one to three, for example, one, two, three, or four of the Ds bases, the Pa' bases, and/or the Px bases.

In addition, in a nucleic acid aptamer of the present invention, the Ds base, the Pa' base, and/or the Px base is/are not limited to any position, and may be located, for example, on the 5' end and/or 3' end of a nucleic acid strand constituting the nucleic acid aptamer, and/or inside the nucleic acid strand.

In one embodiment, a nucleic acid aptamer of the present invention comprises the Ds base and the Pa' base, or the Ds base and the Px base. In the present embodiment, the number of the Ds bases and the Pa' bases, or of the Ds bases and the Px bases is not limited, subject to being 1 or more, and may be, for example, 1 or more and 20 or less, more specifically 1, 2, 3, or 4.

In one embodiment, in a nucleic acid aptamer of the present invention, the R¹ group in the Pa' base shown in the general formula (II) and/or the Px base shown in general formula (III) may have a structure shown in any one formula shown in the formula (IV) to the formula (XXV). The Pa' base is preferably a Pa'_{(methyl)} base or a Pa'_{(diol)} base, more preferably a Pa'_{(methyl)} base. In addition, the Px base is preferably a Px_{(methyl)} base or a Px_{(diol)} base, more preferably a Px'_{(methyl)} base.

In one embodiment, a nucleic acid aptamer of the present invention is composed of a 5' region, a central region, and a 3' region. As used herein, the "5' region" and the "3' region" are regions that are located on the 5' end side and the 3' end side respectively in the nucleic acid aptamer, and each comprises a pair of base sequences complementary to each other so as to form a stem structure. As used herein, the "central region" is a region that is located between the 5' region and the 3' region, and mainly has the role of binding to a target substance. A nucleic acid aptamer of the present invention can comprise a Ds base and/or a Pa' base, or a Ds base and/or a Px base, in at least the central region, in principle, but the 5' region and/or the 3' region can also comprise these unnatural bases.

The above-described pair of base sequences comprised in the 5' region and the 3' region can be constituted by arbitrary, mutually complementary base sequences which are, for example,1 bp or more, 2 bp or more, 3 bp or more, 4 bp or more, or 5 bp or more, and/or 50 bp or less, 40 bp or less, 30 bp or less, 25 bp or less, 20 bp or less, 15 bp or less, 10 bp or less, or 8 bp or less. Specific examples of the pair of base sequences include a structure formed by base-pairing of two base sequences of SEQ ID NOs: 51 and 52; SEQ ID NOs: 53 and 54; SEQ ID NOs: 55 and 56; SEQ ID NOs: 57 and 58 (the base shown as n at position 9 in SEQ ID NO: 57 is Ds); SEQ ID NOs: 59 and 60; SEQ ID NOs: 61 and 62; SEQ ID NOs: 63 and 64; SEQ ID NOs: 65 and 66; SEQ ID NOs: 67 and 68; SEQ ID NOs: 69 and 70; SEQ ID NOs: 71 and 72; SEQ ID NOs: 73 and 74 (the base shown as n at position 3 in SEQ ID NO: 74 is Ds); SEQ ID NOs: 75 and 76; SEQ ID NOs: 77 and 78; SEQ ID NOs: 79 and 80; SEQ ID NOs: 81 and 82; SEQ ID NOs: 79 and 83 (the base shown as n at position 7 in SEQ ID NO: 83 is Ds); SEQ ID NOs: 84 and 85; SEQ ID NOs: 86 and 87; SEQ ID NOs: 232 and 233; SEQ ID NOs: 234 and 235; SEQ ID NOs: 239 and 240; or SEQ ID NOs: 241 and 242.

In one embodiment, a nucleic acid aptamer of the present invention comprises a mini-hairpin sequence on the 5' end side, on the 3' end side, and/or inside the sequence, for example, the 3' end side. The mini-hairpin sequence may have a modifying group, such as biotin, bound thereto. For example, a nucleic acid aptamer of the present invention comprises the above-described 5' region, central region, and 3' region, and mini-hairpin sequence in this order from the 5' end, or comprises the mini-hairpin sequence and the above-described 5' region, central region, and 3' region in this order from the 5' end.

In one embodiment, in a nucleic acid aptamer of the present invention, a pair of base sequences comprised in the 5' region and the 3' region comprise a non-complementary base pair, and/or an insertion sequence and/or a deletion of one or more bases. The non-complementary base pair may be an arbitrary base pair other than the above-described Watson-Crick-type base pair, or may be, for example, a G-T base pair.

In addition, in one embodiment, a nucleic acid aptamer of the present invention comprises an internal loop structure, bulge structure, bulge-out structure, loop structure, hairpin structure, and/or stem-loop structure at a site not influential to the capacity of the nucleic acid aptamer to bind to a target protein, examples of which site include the 5' region and/or the 3' region. These structures may be composed of natural bases, or may comprise an unnatural base, such as a Ds base, a Pa' base, and/or a Px base.

The length of a nucleic acid aptamer of the present invention is, for example, 10 mer or more, 15 mer or more, 20 mer or more, 30 mer or more, 40 mer or more, 50 mer or more, 60 mer or more, 70 mer or more, 80 mer or more, 90 mer or more, or 100 mer or more, and/or 300 mer or less, 200 mer or less, 150 mer or less, 140 mer or less, 130 mer or less, 120 mer or less, 110 mer or less, 100 mer or less, 90 mer or less, 80 mer or less, 70 mer or less, 60 mer or less, or 50 mer or less.

A nucleic acid aptamer of the present invention optionally comprises a base analog, another unnatural base, another modified base, or the like in addition to the Ds base, the Pa' base, and/or the Px base.

A nucleic acid aptamer of the present invention may be modified by addition of another substance, for example, polyethylene glycol (PEG) (for example, an approximately 20 to approximately 60 kDa PEG polymer), an amino acid, peptide, inverted dT, lipid, pigment, fluorescent substance, enzyme, radioactive substance, or biotin. If desired, such a substance may be linked via a known linker. Examples of the linker herein include a nucleotide linker, peptide linker, and linker comprising a disulfide bond. It is known that linking PEG generally enables the half-life period of a DNA aptamer to be extended. In addition, the position of modification in a nucleic acid aptamer of the present invention is not limited, and may be, for example, the 5' end portion, the 3' end portion, or inside the aptamer sequence. For example, the mini-hairpin sequence may be modified.

A method for producing a nucleic acid aptamer of the present invention is not particularly limited. A method known in the art may be used. For example, a nucleic acid aptamer comprising the Ds base and the Pa' base may be chemically synthesized in accordance with a known solid phase synthesis method on the basis of the above-described sequence. For a method for chemically synthesizing a nucleic acid, see, for example, Current Protocols in Nucleic Acid Chemistry, Volume 1, Section 3. In addition, the nucleic acid aptamer may be produced, for example, by synthesizing some fragments on the basis of the sequence of the nucleic acid aptamer, followed by linking the fragments by intramolecular annealing, ligation with ligase, or the like. In addition, the nucleic acid aptamer can also be produced by synthesizing a nucleic acid fragment comprising the Ds base, the Pa' base, and the Px base by TdT or an enzymatic reaction that takes one base in, and linking another fragment to the fragment. Furthermore, a nucleic acid aptamer comprising the Ds base and the Px base can be produced by chemically synthesizing a nucleic acid aptamer comprising the Ds base and the Pa' base by the above-described method, and then, using the resulting nucleic acid aptamer as a template to perform amplification by a nucleic acid amplification method (for example, the below-described replacement PCR method), using a substrate comprising dPxTP and dDsTP. A nucleic acid aptamer of the present invention, after synthesis, is preferably purified using a method known in the art, before use. Examples of a purifying method include a gel purification method, affinity column purification method, and HPLC method.

In (1) to (8) below, the constitution of each binding target for a nucleic acid aptamer of the present invention will be described specifically.

### <(1) / (2) / (8) Nucleic acid aptamer that binds to IFN-y>

In one further aspect of a nucleic acid aptamer of the present invention, a nucleic acid aptamer that binds to an IFN-γ protein (the aptamer is hereinafter referred to as an "IFN-γ-targeting nucleic acid aptamer") is provided.

As used herein, "IFN-y (Interferon gamma)" is a cytokine that is secreted from an immune cell, such as a T cell or an NK cell, and is produced in an immune response to infection by a virus, bacterium, or the like. In the present invention, the origin of IFN-γ is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. IFN-y from a human is preferable. The IFN-γ may be glycosylated IFN-y or non-glycosylated IFN-y. An IFN-γ-targeting nucleic acid aptamer of the present invention preferably binds to glycosylated IFN-γ and non-glycosylated IFN-y.

In one embodiment, an IFN-γ-targeting nucleic acid aptamer of the present invention comprises the base sequence of SEQ ID NO: 2, 5, or 7 or a sequence in which a pair of base sequences complementary to each other and capable of forming a stem structure are added to the 5' end side and 3' end side of the base sequence of SEQ ID NO: 2, 5, or 7.

The base sequence of SEQ ID NO: 2 corresponds to the central region resulting from excluding a stem region from the base sequence of each of AptIFN-γ-1 and AptIFN-γ-3 in Example 3. The base sequence of SEQ ID NO: 5 corresponds to the central region resulting from excluding a stem region from the base sequence of AptIFN-γ-4 in Example 3. The base sequence of SEQ ID NO: 7 corresponds to the central region resulting from excluding a stem region from the base sequence of AptIFN-γ-5 in Example 3.

In a further embodiment of the present aspect, an IFN-γ-targeting nucleic acid aptamer is selected from the group consisting of (1-i-a) to (1-iv-d) below:
(1-i-a) a nucleic acid aptamer comprising the base sequence of SEQ ID NO: 1, wherein the bases shown as n at positions 28 and 34 in the base sequence are an unnatural base Ds, and the base shown as n at position 22 in the base sequence is an unnatural base Pa' or an unnatural base Px;
(1-i-b) a nucleic acid aptamer comprising a base sequence derived from SEQ ID NO: 1 in which one or multiple bases are deleted, substituted, or added at a position(s) except positions 22, 28, and 34 in SEQ ID NO: 1, wherein the positions in the base sequence corresponding to the bases shown as n at positions 28 and 34 in SEQ ID NO: 1 are an unnatural base Ds, and the position corresponding to the base shown as n at position 22 in SEQ ID NO: 1 is an unnatural base Pa' or an unnatural base Px;
(1-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 2, and the bases shown as n at positions 18 and 24 in the base sequence of SEQ ID NO: 2 are an unnatural base Ds, and the base shown as n at position 12 in the base sequence of SEQ ID NO: 2 is an unnatural base Pa' or an unnatural base Px;
(1-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 2 in which one or multiple bases are deleted, substituted, or added at a position(s) except positions 12, 18, and 24 in SEQ ID NO: 2, wherein the positions in the base sequence in the central region corresponding to the bases shown as n at positions 18 and 24 in SEQ ID NO: 2 are an unnatural base Ds, and the position in the base sequence in the central region corresponding to the base shown as n at position 12 in SEQ ID NO: 2 is an unnatural base Pa' or an unnatural base Px;
(1-ii-a) a nucleic acid aptamer comprising the base sequence of SEQ ID NO: 3, wherein the bases shown as n at positions 27 and 33 in the base sequence are an unnatural base Ds, and the base shown as n at position 21 in the base sequence is an unnatural base Pa' or an unnatural base Px;
(1-ii-b) a nucleic acid aptamer comprising a base sequence derived from SEQ ID NO: 3 in which one or multiple bases are deleted, substituted, or added at a position(s) except positions 21, 27, and 33 in SEQ ID NO: 3, wherein the positions in the base sequence corresponding to the bases shown as n at positions 27 and 33 in SEQ ID NO: 3 are an unnatural base Ds, and the position in the base sequence corresponding to the base shown as n at position 21 in SEQ ID NO: 3 is an unnatural base Pa' or an unnatural base Px;
(1-iii-a) a nucleic acid aptamer comprising the base sequence of SEQ ID NO: 4, wherein the bases shown as n at positions 27 and 33 in the base sequence are an unnatural base Ds;
(1-iii-b) a nucleic acid aptamer comprising a base sequence derived from SEQ ID NO: 4 in which one or multiple bases are deleted, substituted, or added at a position(s) except positions 27 and 33 in SEQ ID NO: 4, wherein the positions in the base sequence corresponding to the bases shown as n at positions 27 and 33 in SEQ ID NO: 4 are an unnatural base Ds;
(1-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 5, and the bases shown as n at positions 18 and 24 in the base sequence of SEQ ID NO: 5 are an unnatural base Ds;
(1-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 5 in which one or multiple bases are deleted, substituted, or added at a position(s) except positions 18 and 24 in SEQ ID NO: 5, wherein the positions in the base sequence in the central region corresponding to the bases shown as n at positions 18 and 24 in SEQ ID NO: 5 are an unnatural base Ds;
(1-iv-a) a nucleic acid aptamer comprising the base sequence of SEQ ID NO: 6, wherein the base shown as n at position 33 in the base sequence is an unnatural base Ds, and the base shown as n at position 21 in the base sequence is an unnatural base Pa' or an unnatural base Px;
(1-iv-b) a nucleic acid aptamer comprising a base sequence derived from SEQ ID NO: 6 in which one or multiple bases are deleted, substituted, or added at a position(s) except positions 21 and 33 in SEQ ID NO: 6, wherein the position in the base sequence corresponding to the base shown as n at position 33 in SEQ ID NO: 6 is an unnatural base Ds, and the position in the base sequence corresponding to the base shown as n at position 21 in SEQ ID NO: 6 is an unnatural base Pa' or an unnatural base Px;
(1-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 7, and the base shown as n at position 24 in the base sequence of SEQ ID NO: 7 is an unnatural base Ds, and the base shown as n at position 12 in the base sequence of SEQ ID NO: 7 is an unnatural base Pa' or an unnatural base Px; and
(1-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 7 in which one or multiple bases are deleted, substituted, or added at a position(s) except positions 12 and 24 in SEQ ID NO: 7, wherein the position in the base sequence in the central region corresponding to the base shown as n at position 24 in SEQ ID NO: 7 is an unnatural base Ds, and the position in the base sequence in the central region corresponding to the base shown as n at position 12 in SEQ ID NO: 7 is an unnatural base Pa' or an unnatural base Px.

In this regard, the above-described SEQ ID NOs: 1, 3, 4 and 6 are sequence resulting from adding, to the 5' end side and 3' end side of SEQ ID NOs: 2, 2, 5 and 7 respectively, sequences complementary to each other, and capable of forming a stem structure.

In another embodiment, an IFN-γ-targeting nucleic acid aptamer of the present invention comprises the base sequence of SEQ ID NO: 9, 11, 13, 15, 17, or 19, or a sequence in which a pair of base sequences complementary to each other and capable of forming a stem structure are added to the 5' end side and 3' end side of the base sequence of SEQ ID NO: 9, 11, 13, 15, 17, or 19.

The base sequence of SEQ ID NO: 9 corresponds to the central region resulting from excluding a stem region from the base sequence of XL2-01a in Example 3. The base sequence of SEQ ID NO: 11 corresponds to the central region resulting from excluding a stem region from the base sequence of XL2-01h in Example 3. The base sequence of SEQ ID NO: 13 corresponds to the central region resulting from excluding a stem region from the base sequence of XL2-01i in Example 3. The base sequence of SEQ ID NO: 15 corresponds to the central region resulting from excluding a stem region from the base sequence of XL2-01c in Example 3. The base sequence of SEQ ID NO: 17 corresponds to the central region resulting from excluding a stem region from the base sequence of XL2-01d in Example 3. The base sequence of SEQ ID NO: 19 corresponds to the central region resulting from excluding a stem region from the base sequence encompassing XL2-01a, XL2-01h, XL2-01i, XL2-01c, and XL2-01d in Example 3.

In a further embodiment of the present aspect, an IFN-γ-targeting nucleic acid aptamer is selected from the group consisting of (2-i-a) to (2-vi-d) below:
(2-i-a) a nucleic acid aptamer comprising the base sequence of SEQ ID NO: 8, wherein the bases shown as n at positions 24 and 37 in the base sequence are an unnatural base Ds;
(2-i-b) a nucleic acid aptamer comprising a base sequence derived from SEQ ID NO: 8 in which one or multiple bases are deleted, substituted, or added at a position(s) except positions 13, 24, 31, and 37 in SEQ ID NO: 8, wherein the positions in the base sequence corresponding to the bases shown as n at positions 24 and 37 in SEQ ID NO: 8 are an unnatural base Ds;
(2-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 9, and the bases shown as n at positions 15 and 28 in the base sequence of SEQ ID NO: 9 are an unnatural base Ds;
(2-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 9 in which one or multiple bases are deleted, substituted, or added at a position(s) except positions 4, 15, 22, and 28 in SEQ ID NO: 9, wherein the positions in the base sequence in the central region corresponding to the bases shown as n at positions 15 and 28 in SEQ ID NO: 9 are an unnatural base Ds;
(2-ii-a) a nucleic acid aptamer comprising the base sequence of SEQ ID NO: 10, wherein the bases shown as n at positions 17, 24, and 37 in the base sequence are an unnatural base Ds;
(2-ii-b) a nucleic acid aptamer comprising a base sequence derived from SEQ ID NO: 10 in which one or multiple bases are deleted, substituted, or added at a position(s) except positions 13, 17, 24, 31, and 37 in SEQ ID NO: 10, wherein the positions in the base sequence corresponding to the bases shown as n at positions 17, 24, and 37 in SEQ ID NO: 10 are an unnatural base Ds;
(2-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 11, and the bases shown as n at positions 8, 15, and 28 in the base sequence of SEQ ID NO: 11 are an unnatural base Ds;
(2-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 11 in which one or multiple bases are deleted, substituted, or added at a position(s) except positions 4, 8, 15, 22, and 28 in SEQ ID NO: 11, wherein the positions in the base sequence in the central region corresponding to the bases shown as n at positions 8, 15, and 28 in SEQ ID NO: 11 are an unnatural base Ds;
(2-iii-a) a nucleic acid aptamer comprising the base sequence of SEQ ID NO: 12, wherein the bases shown as n at positions 18, 24 and 37 in the base sequence are an unnatural base Ds;
(2-iii-b) a nucleic acid aptamer comprising a base sequence derived from SEQ ID NO: 12 in which one or multiple bases are deleted, substituted, or added at a position(s) except positions 13, 18, 24, 31, and 37 in SEQ ID NO: 12, wherein the positions in the base sequence corresponding to the bases shown as n at positions 18, 24, and 37 in SEQ ID NO: 12 are an unnatural base Ds;
(2-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 13, and the bases shown as n at positions 9, 15, and 28 in the base sequence of SEQ ID NO: 13 are an unnatural base Ds;
(2-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 13 in which one or multiple bases are deleted, substituted, or added at a position(s) except positions 4, 9, 15, 22, and 28 in SEQ ID NO: 13, wherein the positions in the base sequence in the central region corresponding to the bases shown as n at positions 9, 15, and 28 in SEQ ID NO: 13 are an unnatural base Ds;
(2-iv-a) a nucleic acid aptamer comprising the base sequence of SEQ ID NO: 14, wherein the bases shown as n at positions 24 and 37 in the base sequence are an unnatural base Ds, and the base shown as n at position 17 in the base sequence is an unnatural base Pa' or an unnatural base Px;
(2-iv-b) a nucleic acid aptamer comprising a base sequence derived from SEQ ID NO: 14 in which one or multiple bases are deleted, substituted, or added at a position(s) except positions 13, 17, 24, 31, and 37 in SEQ ID NO: 14, wherein the positions in the base sequence corresponding to the bases shown as n at positions 24 and 37 in SEQ ID NO: 14 are an unnatural base Ds, and the position in the base sequence corresponding to the base shown as n at position 17 in SEQ ID NO: 14 is an unnatural base Pa' or an unnatural base Px;
(2-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 15, and the bases shown as n at positions 15 and 28 in the base sequence of SEQ ID NO: 15 are an unnatural base Ds, and the base shown as n at position 8 in the base sequence of SEQ ID NO: 15 is an unnatural base Pa' or an unnatural base Px;
(2-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 15 in which one or multiple bases are deleted, substituted, or added at a position(s) except positions 4, 8, 15, 22, and 28 in SEQ ID NO: 15, wherein the positions in the base sequence in the central region corresponding to the bases shown as n at positions 15 and 28 in SEQ ID NO: 15 are an unnatural base Ds, and the position in the base sequence in the central region corresponding to the base shown as n at position 8 in SEQ ID NO: 15 is an unnatural base Pa' or an unnatural base Px;
(2-v-a) a nucleic acid aptamer comprising the base sequence of SEQ ID NO: 16, wherein the bases shown as n at positions 24 and 37 in the base sequence are an unnatural base Ds, and the base shown as n at position 18 in the base sequence is an unnatural base Pa' or an unnatural base Px;
(2-v-b) a nucleic acid aptamer comprising a base sequence derived from SEQ ID NO: 16 in which one or multiple bases are deleted, substituted, or added at a position(s) except positions 13, 18, 24, 31, and 37 in SEQ ID NO: 16, wherein the positions in the base sequence corresponding to the bases shown as n at positions 24 and 37 in SEQ ID NO: 16 are an unnatural base Ds, and the position in the base sequence corresponding to the base shown as n at position 18 in SEQ ID NO: 16 is an unnatural base Pa' or an unnatural base Px;
(2-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 17, and the bases shown as n at positions 15 and 28 in the base sequence of SEQ ID NO: 17 are an unnatural base Ds, and the base shown as n at position 9 in the base sequence of SEQ ID NO: 17 is an unnatural base Pa' or an unnatural base Px;
(2-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 17 in which one or multiple bases are deleted, substituted, or added at a position(s) except positions 4, 9, 15, 22, and 28 in SEQ ID NO: 17, wherein the positions in the base sequence in the central region corresponding to the bases shown as n at positions 15 and 28 in SEQ ID NO: 17 are an unnatural base Ds, and the position in the base sequence in the central region corresponding to the base shown as n at position 9 in SEQ ID NO: 17 is an unnatural base Pa' or an unnatural base Px;
(2-vi-a) a nucleic acid aptamer comprising the base sequence of SEQ ID NO: 18, wherein the base(s) shown as n at position(s) 17, 18, 24, and/or 37 in the base sequence are an unnatural base Ds, an unnatural base Pa', and/or an unnatural base Px;
(2-vi-b) a nucleic acid aptamer comprising a base sequence derived from SEQ ID NO: 18 in which one or multiple bases are deleted, substituted, or added at a position(s) except positions 13, 17, 18, 24, 31, and 37 in SEQ ID NO: 18, wherein the positions in the base sequence corresponding to the base(s) shown as n at position(s) 17, 18, 24, and/or 37 in SEQ ID NO: 18 are an unnatural base Ds, an unnatural base Pa', and/or an unnatural base Px;
(2-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 19, and the base(s) shown as n at position(s) 8, 9, 15, and/or 28 in the base sequence of SEQ ID NO: 19 are an unnatural base Ds, an unnatural base Pa', and/or an unnatural base Px; and
(2-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 19 in which one or multiple bases are deleted, substituted, or added at a position(s) except positions 4, 8, 9, 15, 22, and 28 in SEQ ID NO: 19, wherein the position(s) in the base sequence in the central region corresponding to the base(s) shown as n at positions 8, 9, 15, and/or 28 in SEQ ID NO: 19 are an unnatural base Ds, an unnatural base Pa', and/or an unnatural base Px.

In this regard, the base sequences of the above-described SEQ ID NOs: 8, 10, 12, 14, 16, and 18 are sequences resulting from adding, to the 5' end side and 3' end side of SEQ ID NOs: 9, 11, 13, 15, 17, and 19 respectively, sequences complementary to each other, and capable of forming a stem structure.

In another embodiment, an IFN-γ-targeting nucleic acid aptamer of the present invention comprises the base sequence of any one of SEQ ID NOs: 213 to 231 or a sequence in which a pair of base sequences complementary to each other and capable of forming a stem structure are added to the 5' end side and 3' end side of the base sequence of any one of SEQ ID NOs: 213 to 231.

The base sequence of SEQ ID NO: 213 corresponds to the central region resulting from excluding a stem region from the base sequence of each of B-IFN-g-701a and B-IFN-g-701b in Example 5. The base sequence of SEQ ID NO: 214 corresponds to the central region resulting from excluding a stem region from the base sequence of each of B-IFN-g-703a to B-IFN-g-703d in Example 5. The base sequences of SEQ ID NOs: 215 to 219 correspond to the central region resulting from excluding a stem region from the base sequence of each of B-IFN-g-701-1 to B-IFN-g-701-4 and B-IFN-g-701-9 in Example 5. The base sequence of SEQ ID NO: 220 corresponds to the central region resulting from excluding a stem region from the base sequence of each of B-IFN-g-701-1(DPD), B-IFN-g-701-1a(APD), B-IFN-g-701-1b(DTD), and B-IFN-g-701-1c(DPA) in Example 5. The base sequences of SEQ ID NOs: 221 to 231 correspond to the central region resulting from excluding a stem region from the base sequence of each of B-IFN-g-701-21 to B-IFN-g-701-27, B-IFN-g-701-31 to B-IFN-g-701-33, and B-IFN-g-701-42 in Example 5.

In a further embodiment of the present aspect, an IFN-γ-targeting nucleic acid aptamer is selected from the group consisting of (8-i-a) to (8-xii-b) below:
(8-i-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 213, and the bases shown as n at positions 10 and 26 in the base sequence of SEQ ID NO: 213 are Ds, and the base shown as n at position 16 in the base sequence of SEQ ID NO: 213 is Pa', Px, or the T base;
(8-i-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 213 in which one or multiple bases are deleted, substituted, or added at a position(s) except positions 10, 16, and 26 in SEQ ID NO: 213, wherein the positions in the base sequence in the central region corresponding to the bases shown as n at positions 10 and 26 in SEQ ID NO: 2 are Ds, and the position in the base sequence in the central region corresponding to the base shown as n at position 16 in SEQ ID NO: 2 is Pa', Px, or the T base;
(8-ii-a) the nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 214, wherein the base shown as n at position 2 in the base sequence of SEQ ID NO: 214 is Pa', Px, or the T base, the base shown as n at position 16 in the base sequence of SEQ ID NO: 214 is Ds, Pa', or Px, the base shown as n at position 26 in the base sequence of SEQ ID NO: 214 is Pa' or Px, the base shown as n at position 31 in the base sequence of SEQ ID NO: 214 is Ds;
(8-ii-b) the nucleic acid aptamer comprising a 5' region, a central region, and a 3' region in this order from the 5' end, wherein the 5' region and 3' region comprise a pair of base sequences which are complementary to each other, and are capable of forming a stem structure, and wherein the central region comprises a base sequence derived from SEQ ID NO: 214 in which one or multiple bases are deleted, substituted, or added at a position(s) except positions 2, 16, 26, and 31 in SEQ ID NO: 214, and wherein the position in the base sequence in the central region corresponding to the base shown as n at position 2 in SEQ ID NO: 214 is Pa', Px, or the T base, the position in the base sequence in the central region corresponding to the base shown as n at position 16 in SEQ ID NO: 214 is Ds, Pa', or Px, the position in the base sequence in the central region corresponding to the base shown as n at position 26 in SEQ ID NO: 214 is Pa' or Px, and the position in the base sequence in the central region corresponding to the base shown as n at position 31 in SEQ ID NO: 214 is Ds;
(8-iii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 215, and the bases shown as n at positions 12 and 18 in the base sequence of SEQ ID NO: 215 are Ds or the base A, and the base shown as n at position 18 in the base sequence of SEQ ID NO: 215 is Pa', Px, or the T base;
(8-iii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 215 in which one or multiple bases are deleted, substituted, or added at a position(s) except positions 12, 18, and 28 in SEQ ID NO: 215, wherein the positions in the positions in the base sequence in the central region corresponding to the bases shown as n at positions 12 and 28 in SEQ ID NO: 215 are Ds or the base A, and the position in the base sequence in the central region corresponding to the base shown as n at position 18 in SEQ ID NO: 215 is Pa', Px, or the T base;
(8-iv-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of any of SEQ ID NOs: 216 to 221, 227, and 228, and the bases shown as n at positions 12 and 18 in the base sequence of any of SEQ ID NOs: 216 to 221, 227, and 228 are Ds, and the base shown as n at position 18 in the base sequence of any of SEQ ID NOs: 216 to 221, 227, and 228 is Pa' or Px;
(8-iv-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from any of SEQ ID NOs: 216 to 221, 227, and 228 in which one or multiple bases are deleted, substituted, or added at a position(s) except positions 12, 18, and 28 in any of SEQ ID NOs: 216 to 221, 227, and 228, wherein the positions in the base sequence in the central region corresponding to the bases shown as n at positions 12 and 28 in any of SEQ ID NOs: 216 to 221, 227, and 228 are Ds, and the position in the base sequence in the central region corresponding to the base shown as n at position 18 in any of SEQ ID NOs: 216 to 221, 227, and 228 is Pa' or Px;
(8-v-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 222, and the bases shown as n at positions 12 and 28 in the base sequence of SEQ ID NO: 222 are Ds, and the bases shown as n at position 16 and 18 in the base sequence of SEQ ID NO: 222 is Pa' or Px;
(8-v-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 222 in which one or multiple bases are deleted, substituted, or added at a position(s) except positions 12, 16, 18, and 28 in SEQ ID NO: 222, wherein the positions in the base sequence in the central region corresponding to the bases shown as n at positions 12 and 28 in SEQ ID NO: 222 are Ds, and the positions in the base sequence in the central region corresponding to the bases shown as n at positions 16 and 18 in SEQ ID NO: 222 are Pa' or Px;
(8-vi-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 223, and the bases shown as n at positions 12 and 28 in the base sequence of SEQ ID NO: 223 are Ds, and the bases shown as n at position 18 and 34 in the base sequence of SEQ ID NO: 223 are Pa' or Px;
(8-vi-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 223 in which one or multiple bases are deleted, substituted, or added at a position(s) except positions 12, 18, 28, and 34 in SEQ ID NO: 223, wherein the positions in the base sequence in the central region corresponding to the bases shown as n at positions 12 and 28 in SEQ ID NO: 223 are Ds, and the positions in the base sequence in the central region corresponding to the bases shown as n at positions 18 and 34 in SEQ ID NO: 223 are Pa' or Px;
(8-vii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 224, and the bases shown as n at positions 12 and 28 in the base sequence of SEQ ID NO: 224 are Ds, and the bases shown as n at position 18 and 38 in the base sequence of SEQ ID NO: 224 are Pa' or Px;
(8-vii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 224 in which one or multiple bases are deleted, substituted, or added at a position(s) except positions 12, 18, 28, and 38 in SEQ ID NO: 224, wherein the positions in the base sequence in the central region corresponding to the bases shown as n at positions 12 and 28 in SEQ ID NO: 224 are Ds, and the positions in the base sequence in the central region corresponding to the bases shown as n at positions 18 and 38 in SEQ ID NO: 224 are Pa' or Px;
(8-viii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 225, and the bases shown as n at positions 12 and 28 in the base sequence of SEQ ID NO: 225 are Ds, and the bases shown as n at position 18, 34, and 38 in the base sequence of SEQ ID NO: 225 are Pa' or Px;
(8-viii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 225 in which one or multiple bases are deleted, substituted, or added at a position(s) except positions 12, 18, 28, 34, and 38 in SEQ ID NO: 225, wherein the positions in the base sequence in the central region corresponding to the bases shown as n at positions 12 and 28 in SEQ ID NO: 225 are Ds, and the positions in the base sequence in the central region corresponding to the bases shown as n at positions 18, 34, and 38 in SEQ ID NO: 225 are Pa' or Px;
(8-ix-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 226, and the bases shown as n at positions 12 and 28 in the base sequence of SEQ ID NO: 226 are Ds, and the bases shown as n at position 16, 18, 34 and 38 in the base sequence of SEQ ID NO: 226 are Pa' or Px;
(8-ix-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 226 in which one or multiple bases are deleted, substituted, or added at a position(s) except positions 12, 16, 18, 28, 34, and 38 in SEQ ID NO: 226, wherein the positions in the base sequence in the central region corresponding to the bases shown as n at positions 12 and 28 in SEQ ID NO: 226 are Ds, and the positions in the base sequence in the central region corresponding to the bases shown as n at positions 16, 18, 34, and 38 in SEQ ID NO: 226 are Pa' or Px;
(8-x-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 229, and the bases shown as n at positions 11 and 27 in the base sequence of SEQ ID NO: 229 are Ds, and the base shown as n at position 17 in the base sequence of SEQ ID NO: 229 is Pa' or Px;
(8-x-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 229 in which one or multiple bases are deleted, substituted, or added at a position(s) except positions 11, 17, and 27 in SEQ ID NO: 229, wherein the positions in the base sequence in the central region corresponding to the bases shown as n at positions 11 and 27 in SEQ ID NO: 229 are Ds, and the position in the base sequence in the central region corresponding to the base shown as n at position 17 in SEQ ID NO: 229 is Pa' or Px;
(8-xi-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 230, and the bases shown as n at positions 11 and 27 in the base sequence of SEQ ID NO: 230 are Ds, and the base shown as n at position 15 and 17 in the base sequence of SEQ ID NO: 230 are Pa' or Px;
(8-xi-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 230 in which one or multiple bases are deleted, substituted, or added at a position(s) except positions 11, 17, and 27 in SEQ ID NO: 230, wherein the positions in the base sequence in the central region corresponding to the bases shown as n at positions 11 and 27 in SEQ ID NO: 230 are Ds, and the positions in the base sequence in the central region corresponding to the bases shown as n at positions 15 and 17 in SEQ ID NO: 230 are Pa' or Px;
(8-xii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 231, and the bases shown as n at positions 8 and 24 in the base sequence of SEQ ID NO: 231 are Ds, and the base shown as n at position 14 in the base sequence of SEQ ID NO: 231 is Pa' or Px; and
(8-xii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 231 in which one or multiple bases are deleted, substituted, or added at a position(s) except positions 8, 14, and 24 in SEQ ID NO: 231, wherein the positions in the base sequence in the central region corresponding to the bases shown as n at positions 8 and 24 in SEQ ID NO: 231 are Ds, and the position in the base sequence in the central region corresponding to the base shown as n at position 14 in SEQ ID NO: 231 is Pa' or Px.

### <(3) Nucleic acid aptamer that binds to CRP>

In one further aspect of a nucleic acid aptamer of the present invention, a nucleic acid aptamer that binds to C-reactive protein (CRP) (the aptamer is hereinafter referred to as a "CRP-targeting nucleic acid aptamer") is provided.

As used herein, the "C-reactive protein (CRP)" is a cyclic pentamer protein known as an acute-phase reactant protein because the protein is detected in blood when an inflammatory reaction is caused in a living body. In the present invention, the origin of CRP is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. CRP from a human is preferable.

In one embodiment, a CRP-targeting nucleic acid aptamer of the present invention comprises the base sequence of SEQ ID NO: 21 or 24 or a sequence in which a pair of base sequences complementary to each other and capable of forming a stem structure are added to the 5' end side and 3' end side of the base sequence of SEQ ID NO: 21 or 24.

The base sequence of SEQ ID NO: 21 corresponds to the central region resulting from excluding a stem region from the base sequence of each of AptCRP-01 and AptCRP-03 in Example 4. The base sequence of SEQ ID NO: 24 corresponds to the central region resulting from excluding a stem region from the base sequence of each of AptCRP-06, AptCRP-07, and AptCRP-08 in Example 4.

In a further embodiment of the present aspect, a CRP-targeting nucleic acid aptamer is selected from the group consisting of (3-i-a) to (3-v-b) below:
(3-i-a) a nucleic acid aptamer comprising the base sequence of SEQ ID NO: 20, wherein the bases shown as n at positions 9 and 20 in the base sequence are an unnatural base Ds, and the base shown as n at position 32 in the base sequence is an unnatural base Pa' or an unnatural base Px;
(3-i-b) a nucleic acid aptamer comprising a base sequence derived from SEQ ID NO: 20 in which one or multiple bases are deleted, substituted, or added at a position(s) except positions 9, 20, and 32 in SEQ ID NO: 20, wherein the positions in the base sequence corresponding to the bases shown as n at positions 9 and 20 in SEQ ID NO: 20 are an unnatural base Ds, and the position in the base sequence corresponding to the base shown as n at position 32 in SEQ ID NO: 20 is an unnatural base Pa' or an unnatural base Px;
(3-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 21, and the base shown as n at position 2 in the base sequence of SEQ ID NO: 21 is an unnatural base Ds, and the base shown as n at position 14 in the base sequence of SEQ ID NO: 21 is a natural base Pa' or an unnatural base Px;
(3-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 21 in which one or multiple bases are deleted, substituted, or added at a position(s) except positions 2 and 14 in SEQ ID NO: 21, wherein the positions in the base sequence in the central region corresponding to the base shown as n at position 2 in SEQ ID NO: 21 is an unnatural base Ds, and the position in the base sequence in the central region corresponding to the base shown as n at position 14 in SEQ ID NO: 21 is an unnatural base Pa' or an unnatural base Px;
(3-ii-a) a nucleic acid aptamer comprising the base sequence of SEQ ID NO: 22, wherein the base shown as n at position 20 in the base sequence is an unnatural base Ds, and the base shown as n at position 32 in the base sequence is an unnatural base Pa' or an unnatural base Px;
(3-ii-b) a nucleic acid aptamer comprising a base sequence derived from SEQ ID NO: 22 in which one or multiple bases are deleted, substituted, or added at a position(s) except positions 20 and 32 in SEQ ID NO: 22, wherein the position in the base sequence corresponding to the base shown as n at position 20 in SEQ ID NO: 22 is an unnatural base Ds, and the position in the base sequence corresponding to the base shown as n at position 32 in SEQ ID NO: 22 is an unnatural base Pa' or an unnatural base Px;
(3-iii-a) a nucleic acid aptamer comprising the base sequence of SEQ ID NO: 23, wherein the bases shown as n at positions 9 and 20 in the base sequence are an unnatural base Ds, and the base shown as n at position 31 in the base sequence is an unnatural base Pa' or an unnatural base Px;
(3-iii-b) a nucleic acid aptamer comprising a base sequence derived from SEQ ID NO: 23 in which one or multiple bases are deleted, substituted, or added at a position(s) except positions 9, 20, and 31 in SEQ ID NO: 23, wherein the positions in the base sequence corresponding to the bases shown as n at positions 9 and 20 in SEQ ID NO: 23 are an unnatural base Ds, and the position in the base sequence corresponding to the base shown as n at position 31 in SEQ ID NO: 23 is an unnatural base Pa' or an unnatural base Px;
(3-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 24, and the base shown as n at position 2 in the base sequence of SEQ ID NO: 24 is an unnatural base Ds, and the base shown as n at position 13 in the base sequence of SEQ ID NO: 24 is an unnatural base Pa' or an unnatural base Px;
(3-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 24 in which one or multiple bases are deleted, substituted, or added at a position(s) except positions 2 and 13 in SEQ ID NO: 24, wherein the position in the base sequence in the central region corresponding to the base shown as n at position 2 in SEQ ID NO: 24 is an unnatural base Ds, and the position in the base sequence in the central region corresponding to the base shown as n at position 13 in SEQ ID NO: 24 is an unnatural base Pa' or an unnatural base Px;
(3-iv-a) a nucleic acid aptamer comprising the base sequence of SEQ ID NO: 25, wherein the base shown as n at position 14 in the base sequence is an unnatural base Ds, and the base shown as n at position 26 in the base sequence is an unnatural base Pa' or an unnatural base Px;
(3-iv-b) a nucleic acid aptamer comprising a base sequence derived from SEQ ID NO: 25 in which one or multiple bases are deleted, substituted, or added at a position(s) except positions 14 and 26 in SEQ ID NO: 25, wherein the position in the base sequence corresponding to the base shown as n at position 14 in SEQ ID NO: 25 is an unnatural base Ds, and the position in the base sequence corresponding to the base shown as n at position 26 in SEQ ID NO: 25 is an unnatural base Pa' or an unnatural base Px;
(3-v-a) a nucleic acid aptamer comprising the base sequence of SEQ ID NO: 26, wherein the base shown as n at positions 14 in the base sequence is an unnatural base Ds, and the base shown as n at position 25 in the base sequence is an unnatural base Pa' or an unnatural base Px; and
(3-v-b) a nucleic acid aptamer comprising a base sequence derived from SEQ ID NO: 26 in which one or multiple bases are deleted, substituted, or added at a position(s) except positions 14 and 25 in SEQ ID NO: 26, wherein the position in the base sequence corresponding to the base shown as n at position 14 in SEQ ID NO: 26 is an unnatural base Ds, and the position in the base sequence corresponding to the base shown as n at position 25 in SEQ ID NO: 26 is an unnatural base Pa' or an unnatural base Px.

In this regard, the base sequences of the above-described SEQ ID NOs: 20, 22, 23, 25, and 26 are sequences resulting from adding, to the 5' end side and 3' end side of SEQ ID NOs: 21, 21, 24, 24, and 24 respectively, sequences complementary to each other, and capable of forming a stem structure.

### <(4) Nucleic acid aptamer that binds to Thrombin>

In one further aspect of a nucleic acid aptamer of the present invention, a nucleic acid aptamer that binds to Thrombin (the aptamer is hereinafter referred to as a "Thrombin-targeting nucleic acid aptamer") is provided.

As used herein, "Thrombin" is a serine protease that plays a leading role in hemostasis through converting fibrinogen into fibrin. In the present invention, the origin of Thrombin is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. Thrombin from a human is preferable.

In one embodiment, a Thrombin-targeting nucleic acid aptamer of the present invention comprises the base sequence of SEQ ID NO: 27, 29, 31, 33, 35, or 37 or a sequence in which a pair of base sequences complementary to each other and capable of forming a stem structure are added to the 5' end side and 3' end side of the base sequence of SEQ ID NO: 28, 30, 32, 34, 36, or 38.

The base sequence of SEQ ID NO: 28 corresponds to the central region resulting from excluding a stem region from the base sequence of Thr204 (SEQ ID NO: 27) in Example 4. The base sequence of SEQ ID NO: 30 corresponds to the central region resulting from excluding a stem region from the base sequence of Thr205 (SEQ ID NO: 29) in Example 4. The base sequence of SEQ ID NO: 32 corresponds to the central region resulting from excluding a stem region from the base sequence of Thr101 (SEQ ID NO: 31) in Example 4. The base sequence of SEQ ID NO: 34 corresponds to the central region resulting from excluding a stem region from the base sequence of Thr104 (SEQ ID NO: 33) in Example 4. The base sequence of SEQ ID NO: 36 corresponds to the central region resulting from excluding a stem region from the base sequence of Thr106 (SEQ ID NO: 35) in Example 4. The base sequence of SEQ ID NO: 38 corresponds to the central region resulting from excluding a stem region from the base sequence of Thr201 (SEQ ID NO: 37) in Example 4.

In a further embodiment of the present aspect, a Thrombin-targeting nucleic acid aptamer is selected from the group consisting of (4-i-a) to (4-vi-d) below:
(4-i-a) a nucleic acid aptamer comprising the base sequence of SEQ ID NO: 27, wherein the bases shown as n at positions 16 and 22 in the base sequence are an unnatural base Ds;
(4-i-b) a nucleic acid aptamer comprising a base sequence derived from SEQ ID NO: 27 in which one or multiple bases are deleted, substituted, or added at a position(s) except positions 16 and 22 in SEQ ID NO: 27, wherein the positions in the base sequence corresponding to the bases shown as n at positions 16 and 22 in SEQ ID NO: 27 are an unnatural base Ds;
(4-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 28, and the bases shown as n at positions 6 and 12 in the base sequence of SEQ ID NO: 28 are an unnatural base Ds;
(4-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 28 in which one or multiple bases are deleted, substituted, or added at a position(s) except positions 6 and 12 in SEQ ID NO: 28, wherein the positions in the base sequence in the central region corresponding to the bases shown as n at positions 6 and 12 in SEQ ID NO: 28 are an unnatural base Ds;
(4-ii-a) a nucleic acid aptamer comprising the base sequence of SEQ ID NO: 29, wherein the bases shown as n at positions 10 and 16 in the base sequence are an unnatural base Ds, and the base shown as n at position 28 in the base sequence is an unnatural base Pa' or an unnatural base Px;
(4-ii-b) a nucleic acid aptamer comprising a base sequence derived from SEQ ID NO: 29 in which one or multiple bases are deleted, substituted, or added at a position(s) except positions 10, 16, and 28 in SEQ ID NO: 29, wherein the positions in the base sequence corresponding to the bases shown as n at positions 10 and 16 in SEQ ID NO: 29 are an unnatural base Ds, and the position in the base sequence corresponding to the base shown as n at position 28 in SEQ ID NO: 29 is an unnatural base Pa' or an unnatural base Px;
(4-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 30, and the bases shown as n at positions 1 and 7 in the base sequence of SEQ ID NO: 30 are an unnatural base Ds, and the base shown as n at position 19 in the base sequence of SEQ ID NO: 30 is an unnatural base Pa' or an unnatural base Px;
(4-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 30 in which one or multiple bases are deleted, substituted, or added at a position(s) except positions 1, 7, and 19 in SEQ ID NO: 30, wherein the positions in the base sequence in the central region corresponding to the bases shown as n at positions 1 and 7 in SEQ ID NO: 30 are an unnatural base Ds, and the position in the base sequence in the central region corresponding to the base shown as n at position 19 in SEQ ID NO: 30 is an unnatural base Pa' or an unnatural base Px;
(4-iii-a) a nucleic acid aptamer comprising the base sequence of SEQ ID NO: 31, wherein the bases shown as n at positions 26 and 41 in the base sequence are an unnatural base Ds;
(4-iii-b) a nucleic acid aptamer comprising a base sequence derived from SEQ ID NO: 31 in which one or multiple bases are deleted, substituted, or added at a position(s) except positions 26 and 41 in SEQ ID NO: 31, wherein the positions in the base sequence corresponding to the bases shown as n at positions 26 and 41 in SEQ ID NO: 31 are an unnatural base Ds;
(4-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 32, and the bases shown as n at positions 18 and 33 in the base sequence of SEQ ID NO: 32 are an unnatural base Ds;
(4-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 32 in which one or multiple bases are deleted, substituted, or added at a position(s) except positions 18 and 33 in SEQ ID NO: 32, wherein the positions in the base sequence in the central region corresponding to the bases shown as n at positions 18 and 33 in SEQ ID NO: 32 are an unnatural base Ds;
(4-iv-a) a nucleic acid aptamer comprising the base sequence of SEQ ID NO: 33, wherein the bases shown as n at positions 16 and 24 in the base sequence are an unnatural base Ds, and the base shown as n at position 35 in the base sequence is an unnatural base Pa' or an unnatural base Px;
(4-iv-b) a nucleic acid aptamer comprising a base sequence derived from SEQ ID NO: 33 in which one or multiple bases are deleted, substituted, or added at a position(s) except positions 16, 24, and 35 in SEQ ID NO: 33, wherein the positions in the base sequence corresponding to the bases shown as n at positions 16 and 24 in SEQ ID NO: 33 are an unnatural base Ds, and the position in the base sequence corresponding to the base shown as n at position 35 in SEQ ID NO: 33 is an unnatural base Pa' or an unnatural base Px;
(4-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 34, and the bases shown as n at positions 10 and 18 in the base sequence of SEQ ID NO: 34 are an unnatural base Ds, and the base shown as n at position 29 in the base sequence of SEQ ID NO: 34 is an unnatural base Pa' or an unnatural base Px;
(4-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 34 in which one or multiple bases are deleted, substituted, or added at a position(s) except positions 10, 18, and 29 in SEQ ID NO: 34, wherein the positions in the base sequence in the central region corresponding to the bases shown as n at positions 10 and 18 in SEQ ID NO: 34 are an unnatural base Ds, and the position in the base sequence in the central region corresponding to the base shown as n at position 29 in SEQ ID NO: 34 is an unnatural base Pa' or an unnatural base Px;
(4-v-a) a nucleic acid aptamer comprising the base sequence of SEQ ID NO: 35, wherein the bases shown as n at positions 17 and 24 in the base sequence are an unnatural base Ds, and the bases shown as n at position 36 and 39 in the base sequence are an unnatural base Pa' or an unnatural base Px;
(4-v-b) a nucleic acid aptamer comprising a base sequence derived from SEQ ID NO: 35 in which one or multiple bases are deleted, substituted, or added at a position(s) except positions 17, 24, 36, and 39 in SEQ ID NO: 35, wherein the positions in the base sequence corresponding to the bases shown as n at positions 17 and 24 in SEQ ID NO: 35 are an unnatural base Ds, and the positions in the base sequence corresponding to the bases shown as n at position 36 and 39 in SEQ ID NO: 35 are an unnatural base Pa' or an unnatural base Px;
(4-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 36, and the bases shown as n at positions 9 and 16 in the base sequence of SEQ ID NO: 36 are an unnatural base Ds, and the bases shown as n at position 28 and 31 in the base sequence of SEQ ID NO: 36 are an unnatural base Pa' or an unnatural base Px;
(4-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 36 in which one or multiple bases are deleted, substituted, or added at a position(s) except positions 9, 16, 28, and 31 in SEQ ID NO: 36, wherein the positions in the base sequence in the central region corresponding to the bases shown as n at positions 9 and 16 in SEQ ID NO: 36 are an unnatural base Ds, and the positions in the base sequence in the central region corresponding to the bases shown as n at positions 28 and 31 in SEQ ID NO: 36 are an unnatural base Pa' or an unnatural base Px;
(4-vi-a) a nucleic acid aptamer comprising the base sequence of SEQ ID NO: 37, wherein the bases shown as n at positions 29 and 38 in the base sequence are an unnatural base Ds;
(4-vi-b) a nucleic acid aptamer comprising a base sequence derived from SEQ ID NO: 37 in which one or multiple bases are deleted, substituted, or added at a position(s) except positions 29 and 38 in SEQ ID NO: 37, wherein the positions in the base sequence corresponding to the bases shown as n at positions 29 and 38 in SEQ ID NO: 37 are an unnatural base Ds;
(4-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 38, and the base shown as n at position 19 in the base sequence of SEQ ID NO: 38 is an unnatural base Ds; and
(4-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 38 in which one or multiple bases are deleted, substituted, or added at a position(s) except position 19 in SEQ ID NO: 38, wherein the position in the base sequence in the central region corresponding to the bases shown as n at position 19 in SEQ ID NO: 38 is an unnatural base Ds.

In this regard, the base sequences of the above-described SEQ ID NOs: 27, 29, 31, 33, 35, and 37 are sequences resulting from adding, to the 5' end side and 3' end side of SEQ ID NOs: 28, 30, 32, 34, 36, and 38 respectively, sequences complementary to each other, and capable of forming a stem structure.

### <(5) Nucleic acid aptamer that binds to IL-6>

In one further aspect of a nucleic acid aptamer of the present invention, a nucleic acid aptamer that binds to Interleukin-6 (IL-6) (the aptamer is hereinafter referred to as a "IL-6-targeting nucleic acid aptamer") is provided.

As used herein, "Interleukin-6 (IL-6)" is one of the cytokines that are produced by a cell, such as a T-cell or a macrophage, and control humoral immunity. In the present invention, the origin of IL-6 is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. IL-6 from a human is preferable.

In one embodiment, a IL-6-targeting nucleic acid aptamer of the present invention comprises the base sequence of SEQ ID NO: 39 or 41 or a sequence in which a pair of base sequences complementary to each other and capable of forming a stem structure are added to the 5' end side and 3' end side of the base sequence of SEQ ID NO: 40 or 42.

The base sequence of SEQ ID NO: 40 corresponds to the central region resulting from excluding a stem region from the base sequence of Bio-IL6-201b in Example 4. The base sequence of SEQ ID NO: 42 corresponds to the central region resulting from excluding a stem region from the base sequence of Bio-IL6-101 in Example 4.

In a further embodiment of the present aspect, a IL-6-targeting nucleic acid aptamer is selected from the group consisting of (5-i-a) to (5-ii-d) below:
(5-i-a) a nucleic acid aptamer comprising the base sequence of SEQ ID NO: 39, wherein the bases shown as n at positions 25 and 34 in the base sequence are an unnatural base Ds, and the bases shown as n at positions 13 and 20 in the base sequence are an unnatural base Pa' or an unnatural base Px;
(5-i-b) a nucleic acid aptamer comprising a base sequence derived from SEQ ID NO: 39 in which one or multiple bases are deleted, substituted, or added at a position(s) except positions 13, 20, 25, and 34 in SEQ ID NO: 39, wherein the positions in the base sequence corresponding to the bases shown as n at positions 25 and 34 in SEQ ID NO: 39 are an unnatural base Ds, and the positions in the base sequence corresponding to the bases shown as n at positions 13 and 20 in SEQ ID NO: 39 are an unnatural base Pa' or an unnatural base Px;
(5-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 40, and the bases shown as n at positions 14 and 23 in the base sequence of SEQ ID NO: 40 are an unnatural base Ds, and the bases shown as n at positions 2 and 9 in the base sequence of SEQ ID NO: 40 are an unnatural base Pa' or an unnatural base Px;
(5-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 40 in which one or multiple bases are deleted, substituted, or added at a position(s) except positions 2, 9, 14, and 23 in SEQ ID NO: 40, wherein the positions in the base sequence in the central region corresponding to the bases shown as n at positions 14 and 23 in SEQ ID NO: 40 are an unnatural base Ds, and the positions in the base sequence in the central region corresponding to the bases shown as n at positions 2 and 9 in SEQ ID NO: 40 is an unnatural base Pa' or an unnatural base Px;
(5-ii-a) a nucleic acid aptamer comprising the base sequence of SEQ ID NO: 41, wherein the bases shown as n at positions 17 and 33 in the base sequence are an unnatural base Ds, and the base shown as n at position 38 in the base sequence is an unnatural base Pa' or an unnatural base Px;
(5-ii-b) a nucleic acid aptamer comprising a base sequence derived from SEQ ID NO: 41 in which one or multiple bases are deleted, substituted, or added at a position(s) except positions 17, 33, and 38 in SEQ ID NO: 41, wherein the positions in the base sequence corresponding to the bases shown as n at positions 17 and 33 in SEQ ID NO: 41 are an unnatural base Ds, and the position in the base sequence corresponding to the base shown as n at position 41 in SEQ ID NO: 41 is an unnatural base Pa' or an unnatural base Px;
(5-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 42, and the bases shown as n at positions 1 and 17 in the base sequence of SEQ ID NO: 42 are an unnatural base Ds, and the base shown as n at position 22 in the base sequence of SEQ ID NO: 42 is an unnatural base Pa' or an unnatural base Px; and
(5-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 42 in which one or multiple bases are deleted, substituted, or added at a position(s) except positions 1, 17, and 22 in SEQ ID NO: 42, wherein the positions in the base sequence in the central region corresponding to the bases shown as n at positions 1 and 17 in SEQ ID NO: 42 are an unnatural base Ds, and the position in the base sequence in the central region corresponding to the base shown as n at position 22 in SEQ ID NO: 42 is an unnatural base Pa' or an unnatural base Px.

In this regard, the base sequences of the above-described SEQ ID NOs: 39 and 41 are sequences resulting from adding, to the 5' end side and 3' end side of SEQ ID NOs: 40 and 42 respectively, sequences complementary to each other, and capable of forming a stem structure.

### <(6) Nucleic acid aptamer that binds to IL-8>

In one further aspect of a nucleic acid aptamer of the present invention, a nucleic acid aptamer that binds to Interleukin-8 (IL-8) (the aptamer is hereinafter referred to as a "IL-8-targeting nucleic acid aptamer") is provided.

As used herein, "Interleukin-8 (IL-8)" is a chemokine produced by a macrophage, epithelial cell, vascular endothelial cell, or the like. In the present invention, the origin of IL-8 is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. IL-8 from a human is preferable.

In one embodiment, a IL-8-targeting nucleic acid aptamer of the present invention comprises the base sequence of SEQ ID NO: 43, 45, or 49 or a sequence in which a pair of base sequences complementary to each other and capable of forming a stem structure are added to the 5' end side and 3' end side of the base sequence of SEQ ID NO: 44, 46, or 50.

The base sequence of SEQ ID NO: 44 corresponds to the central region resulting from excluding a stem region from the base sequence of IL8-102PD or IL8-102DD (SEQ ID NO: 43) in Example 4. The base sequence of SEQ ID NO: 46 corresponds to the central region resulting from excluding a stem region from the base sequence of IL8-223PD or IL8-223DD (SEQ ID NO: 45) in Example 4. The base sequence of SEQ ID NO: 50 corresponds to the central region resulting from excluding a stem region from the base sequence of IL8-201DPD (SEQ ID NO: 49) in Example 4.

In a further embodiment of the present aspect, a IL-8-targeting nucleic acid aptamer is selected from the group consisting of the following (6-i-a) to (6-vii-d):
(6-i-a) a nucleic acid aptamer comprising the base sequence of SEQ ID NO: 43, wherein the base shown as n at position 26 in the base sequence is an unnatural base Ds, and the base shown as n at position 17 in the base sequence is an unnatural base Pa' or an unnatural base Px;
(6-i-b) a nucleic acid aptamer comprising a base sequence derived from SEQ ID NO: 43 in which one or multiple bases are deleted, substituted, or added at a position(s) except positions 17 and 26 in SEQ ID NO: 43, wherein the position in the base sequence corresponding to the base shown as n at position 26 in SEQ ID NO: 43 is an unnatural base Ds, and the position in the base sequence corresponding to the base shown as n at position 17 in SEQ ID NO: 43 is an unnatural base Pa' or an unnatural base Px;
(6-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 44, and the base shown as n at position 12 in the base sequence of SEQ ID NO: 44 is an unnatural base Ds, and the base shown as n at position 3 in the base sequence of SEQ ID NO: 44 is an unnatural base Pa' or an unnatural base Px;
(6-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 44 in which one or multiple bases are deleted, substituted, or added at a position(s) except positions 3 and 12 in SEQ ID NO: 44, wherein the position in the base sequence in the central region corresponding to the base shown as n at position 12 in SEQ ID NO: 44 is an unnatural base Ds, and the position in the base sequence in the central region corresponding to the base shown as n at position 3 in SEQ ID NO: 44 is an unnatural base Pa' or an unnatural base Px;
(6-ii-a) a nucleic acid aptamer comprising the base sequence of SEQ ID NO: 45, wherein the base shown as n at position 26 in the base sequence is an unnatural base Ds, and the base shown as n at position 17 in the base sequence is an unnatural base Pa' or an unnatural base Px;
(6-ii-b) a nucleic acid aptamer comprising a base sequence derived from SEQ ID NO: 45 in which one or multiple bases are deleted, substituted, or added at a position(s) except positions 17 and 26 in SEQ ID NO: 45, wherein the position in the base sequence corresponding to the base shown as n at position 26 in SEQ ID NO: 45 is an unnatural base Ds, and the position in the base sequence corresponding to the base shown as n at position 17 in SEQ ID NO: 45 is an unnatural base Pa' or an unnatural base Px;
(6-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 46, and the base shown as n at position 12 in the base sequence of SEQ ID NO: 46 is an unnatural base Ds, and the base shown as n at position 3 in the base sequence of SEQ ID NO: 46 is an unnatural base Pa' or an unnatural base Px;
(6-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 46 in which one or multiple bases are deleted, substituted, or added at a position(s) except positions 3 and 12 in SEQ ID NO: 46, wherein the position in the base sequence in the central region corresponding to the base shown as n at position 12 in SEQ ID NO: 46 is an unnatural base Ds, and the position in the base sequence in the central region corresponding to the bases shown as n at position 3 in SEQ ID NO: 46 is an unnatural base Pa' or an unnatural base Px;
(6-iii-a) a nucleic acid aptamer comprising the base sequence of SEQ ID NO: 47, wherein the bases shown as n at positions 26 and 37 in the base sequence are an unnatural base Ds, and the base shown as n at position 17 in the base sequence is an unnatural base Pa' or an unnatural base Px;
(6-iii-b) a nucleic acid aptamer comprising a base sequence derived from SEQ ID NO: 47 in which one or multiple bases are deleted, substituted, or added at a position(s) except positions 17, 26, and 37 in SEQ ID NO: 47, wherein the positions in the base sequence corresponding to the bases shown as n at positions 26 and 37 in SEQ ID NO: 47 are an unnatural base Ds, and the position in the base sequence corresponding to the base shown as n at position 17 in SEQ ID NO: 47 is an unnatural base Pa' or an unnatural base Px;
(6-iii-a) a nucleic acid aptamer comprising the base sequence of SEQ ID NO: 48, wherein the base shown as n at position 25 in the base sequence is an unnatural base Ds, and the base shown as n at position 16 in the base sequence is an unnatural base Pa' or an unnatural base Px;
(6-iii-b) a nucleic acid aptamer comprising a base sequence derived from SEQ ID NO: 48 in which one or multiple bases are deleted, substituted, or added at a position(s) except positions 16 and 25 in SEQ ID NO: 48, wherein the position in the base sequence corresponding to the base shown as n at position 25 in SEQ ID NO: 48 is an unnatural base Ds, and the position in the base sequence corresponding to the base shown as n at position 16 in SEQ ID NO: 48 is an unnatural base Pa' or an unnatural base Px;
(6-iv-a) a nucleic acid aptamer comprising the base sequence of SEQ ID NO: 43, wherein the bases shown as n at positions 17 and 26 in the base sequence are an unnatural base Ds;
(6-iv-b) a nucleic acid aptamer comprising a base sequence derived from SEQ ID NO: 43 in which one or multiple bases are deleted, substituted, or added at a position(s) except positions 17 and 26 in SEQ ID NO: 43, wherein the positions in the base sequence corresponding to the bases shown as n at positions 17 and 26 in SEQ ID NO: 43 are an unnatural base Ds;
(6-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 44, and the bases shown as n at positions 3 and 12 in the base sequence of SEQ ID NO: 44 are an unnatural base Ds;
(6-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 44 in which one or multiple bases are deleted, substituted, or added at a position(s) except positions 3 and 12 in SEQ ID NO: 44, wherein the positions in the base sequence in the central region corresponding to the bases shown as n at positions 3 and 12 in SEQ ID NO: 44 are an unnatural base Ds;
(6-v-a) a nucleic acid aptamer comprising the base sequence of SEQ ID NO: 45, wherein the bases shown as n at positions 17 and 26 in the base sequence are an unnatural base Ds;
(6-v-b) a nucleic acid aptamer comprising a base sequence derived from SEQ ID NO: 45 in which one or multiple bases are deleted, substituted, or added at a position(s) except positions 17 and 26 in SEQ ID NO: 45, wherein the positions in the base sequence corresponding to the bases shown as n at positions 17 and 26 in SEQ ID NO: 45 are an unnatural base Ds;
(6-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 46, and the bases shown as n at positions 3 and 12 in the base sequence of SEQ ID NO: 46 are an unnatural base Ds;
(6-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 46 in which one or multiple bases are deleted, substituted, or added at a position(s) except positions 3 and 12 in SEQ ID NO: 46, wherein the positions in the base sequence in the central region corresponding to the bases shown as n at positions 3 and 12 in SEQ ID NO: 46 are an unnatural base Ds;
(6-vi-a) a nucleic acid aptamer comprising the base sequence of SEQ ID NO: 49, wherein the bases shown as n at positions 17 and 30 in the base sequence are an unnatural base Ds, and the base shown as n at position 23 in the base sequence is an unnatural base Pa' or an unnatural base Px;
(6-vi-b) a nucleic acid aptamer comprising a base sequence derived from SEQ ID NO: 49 in which one or multiple bases are deleted, substituted, or added at a position(s) except positions 17, 23, and 30 in SEQ ID NO: 49, wherein the positions in the base sequence corresponding to the bases shown as n at positions 17 and 30 in SEQ ID NO: 49 are an unnatural base Ds, and the position in the base sequence corresponding to the base shown as n at position 23 in SEQ ID NO: 49 is an unnatural base Pa' or an unnatural base Px;
(6-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 50, and the bases shown as n at positions 3 and 16 in the base sequence of SEQ ID NO: 50 are an unnatural base Ds, and the base shown as n at position 9 in the base sequence of SEQ ID NO: 50 is an unnatural base Pa' or an unnatural base Px; and
(6-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 50 in which one or multiple bases are deleted, substituted, or added at a position(s) except positions 3, 9, and 16 in SEQ ID NO: 50, wherein the positions in the base sequence in the central region corresponding to the bases shown as n at positions 3 and 16 in SEQ ID NO: 50 are an unnatural base Ds, and the position in the base sequence in the central region corresponding to the base shown as n at position 9 in SEQ ID NO: 50 is an unnatural base Pa' or an unnatural base Px.

In this regard, the base sequences of the above-described SEQ ID NOs: 43, 45, 47, 48, and 49 are sequences resulting from adding, to the 5' end side and 3' end side of the base sequences of SEQ ID NOs: 44, 46, 44, 46, and 50 respectively, sequences complementary to each other, and capable of forming a stem structure.

### <(7) Nucleic acid aptamer that binds to HMGB1>

In one further aspect of a nucleic acid aptamer of the present invention, a nucleic acid aptamer that binds to HMGB1 (the aptamer is hereinafter referred to as a "HMGB1-targeting nucleic acid aptamer") is provided.

"HMGB1" (High Mobility Group Box 1) herein functions as a chromatin protein, and in addition, is secreted from an immune cell, such as a macrophage or a monocyte, to function also as a cytokine mediator of inflammation. In the present invention, the origin of HMGB1 is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. HMGB1 from a human is preferable.

In one embodiment, an HMGB1-targeting nucleic acid aptamer of the present invention comprises the base sequence of SEQ ID NO: 135 or a sequence in which a pair of base sequences complementary to each other and capable of forming a stem structure are added to the 5' end side and 3' end side of the base sequence of SEQ ID NO: 134.

The base sequence of SEQ ID NO: 134 corresponds to the central region resulting from excluding a stem region and a mini-hairpin sequence from the base sequence of Bio-HMGB1-301-DD (SEQ ID NO: 171) in Example 4.

In a further embodiment of the present aspect, an HMGB1-targeting nucleic acid aptamer is selected from the group consisting of (7-a) to (7-d) below:
(7-a) a nucleic acid aptamer comprising the base sequence of SEQ ID NO: 135, wherein the bases shown as n at positions 22 and 33 in the base sequence are an unnatural base Ds;
(7-b) a nucleic acid aptamer comprising a base sequence derived from SEQ ID NO: 135 in which one or multiple bases are deleted, substituted, or added at a position(s) except positions 22 and 33 in SEQ ID NO: 135, wherein the positions in the base sequence corresponding to the bases shown as n at positions 22 and 33 in SEQ ID NO: 135 are an unnatural base Ds;
(7-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 134, and the bases shown as n at positions 12 and 23 in the base sequence of SEQ ID NO: 134 are an unnatural base Ds; and
(7-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 134 in which one or multiple bases are deleted, substituted, or added at a position(s) except positions 12 and 23 in SEQ ID NO: 134, wherein the positions in the base sequence in the central region corresponding to the bases shown as n at positions 12 and 23 in SEQ ID NO: 134 are an unnatural base Ds.

In this regard, the base sequences of the above-described SEQ ID NO: 135 are sequence resulting from adding, to the 5' end side and 3' end side of SEQ ID NO: 134, sequences complementary to each other, and capable of forming a stem structure.

### <Pharmaceutical composition>

In one aspect of the present invention, a pharmaceutical composition comprising any of the above-described nucleic acid aptamers is provided. A pharmaceutical composition according to the invention comprises any one or more of the above-described nucleic acid aptamers, and can be used to treat or prevent an arbitrary disease, such as for antiinflammation, rheumatism treatment, or cancer treatment. A pharmaceutical composition of the present invention may comprise a combination of any two or more of the above-described nucleic acid aptamers. In addition, the pharmaceutical composition may comprise one or more other drugs to the extent of not losing the binding capacity of a nucleic acid aptamer of the present invention.

A disease to be prevented and/or treated with a pharmaceutical composition comprising the above-described (1) / (2) / (8) a nucleic acid aptamer that binds to IFN-y is a disease that needs the inhibition of the function of IFN-γ (the disease is hereinafter also referred to as a "IFN-γ-associated disease"). Examples of the IFN-γ-associated disease include an autoimmune disease and an inflammatory disorder. The autoimmune disease or the inflammatory disorder may be, for example, Crohn's disease, systemic lupus erythematosus, psoriasis, rheumatoid arthritis, vasculitis, uveitis, atopic dermatitis, type I diabetes mellitus, multiple sclerosis, schizophrenia, cornea transplant rejection, alopecia areata, plaque psoriasis, vitiligo, pemphigus vulgaris, epidermolysis bullosa, acne vulgaris, herpes simplex virus type 1, or Hunner's interstitial cystitis. In addition, a pharmaceutical composition comprising the above-described (1) / (2) / (8) a nucleic acid aptamer that binds to IFN-y can be used for apheresis.

A disease to be prevented and/or treated with a pharmaceutical composition comprising the above-described (3) a nucleic acid aptamer that binds to CRP is a disease that needs the inhibition of the function of CRP (the disease is hereinafter also referred to as a "CRP-associated disease").

A disease to be prevented and/or treated with a pharmaceutical composition comprising the above-described (4) a nucleic acid aptamer that binds to Thrombin is a disease that needs the inhibition of the function of Thrombin (the disease is hereinafter also referred to as a "Thrombin-associated disease"). Examples of the Thrombin-associated disease include thrombotic disorder. Examples of the thrombotic disorder include venous thrombosis (for example, deep venous thrombosis), pulmonary embolism, atrial fibrillation, myocardial infarction, arterial thrombosis, stroke (for example, thrombosis-induced stroke), atherosclerosis, and diffuse intravascular coagulation. A pharmaceutical composition according to the present embodiment can also be used to suppress or inhibit blood clotting.

A disease to be prevented and/or treated with a pharmaceutical composition comprising the above-described (5) a nucleic acid aptamer that binds to IL-6 is a disease that needs the inhibition of the function of IL-6 (the disease is hereinafter also referred to as an "IL-6-associated disease"). Examples of the IL-6-associated disease include a disease associated with the overproduction of IL-6. Examples include a neurological disease, autoimmune disease, cancer, and inflammatory disease. Specific examples include a central nervous system disease, rheumatism (for example, rheumatoid arthritis), leukemia, solid cancer, juvenile idiopathic arthritis, adult-onset Still's disease, Takayasu's arteritis, giant cell arteritis, neuromyelitis optica spectrum disorder, and Castleman's disease.

A disease to be prevented and/or treated with a pharmaceutical composition comprising the above-described (6) a nucleic acid aptamer that binds to IL-8 is a disease that needs the inhibition of the function of IL-8 (the disease is hereinafter also referred to as an "IL-8-associated disease"). Examples of the IL-8-associated disease include, but are not limited to, a neurological disease, autoimmune disease, cancer, and inflammatory disease. Specific examples include chronic rheumatoid arthritis, gout, psoriasis, adult respiratory distress syndrome (ARDS), asthma, and endometriosis.

A disease to be prevented and/or treated with a pharmaceutical composition comprising the above-described (7) a nucleic acid aptamer that binds to HMGB1 is a disease that needs the inhibition of the function of HMGB1 (the disease is hereinafter also referred to as an "HMGB1-associated disease"). Examples of the HMGB1-associated disease include an inflammatory disease, autoimmune disease, cancer, cardiovascular disease, neurological disease, and infection. Specific examples include rheumatoid arthritis, inflammatory bowel disease, sepsis, inflammatory bowel disease, lupus, Sjogren's syndrome, myocardial infarction, arteriosclerosis, stroke, cerebral infarction, cerebral edema, cerebrovascular disease, cerebral vasospasm, multiple sclerosis, traumatic brain injury, atherosclerosis, neuropathic pain, arthritis, acute pulmonary trauma, cerebral ischemia, renal ischemia, hepatic ischemia, central nervous system disease and peripheral nervous system disease, neurodegenerative disease (Alzheimer's disease, Parkinson's disease, and the like), neuropathic pain, and periodontal disease.

A pharmaceutical composition of the present invention may comprise a pharmaceutically acceptable carrier. A "pharmaceutically acceptable carrier" refers to a substance that facilitates formulation of a pharmaceutical composition to be usually used in the technical field of formulation, and the application of the pharmaceutical composition to an organism, and is added to the extent of not inhibiting or suppressing the action of the pharmaceutical composition. Examples of carriers include an excipient, binder, disintegrant, filler, emulsifier, lubricant, an agent for lubricating, and stabilizer.

Examples of the "excipient" include; sugars, such as monosaccharides, disaccharides, cyclodextrin, and polysaccharides (specifically comprising, but not limited to, glucose, sucrose, lactose, raffinose, mannitol, sorbitol, inositol, dextrin, maltodextrin, starch, and cellulose), metal salts (for example, sodium phosphate, calcium phosphate, calcium sulfate, and magnesium sulfate); citric acid; tartaric acid; glycine; low-, middle-, and high-molecular-weight polyethylene glycols (PEG); Pluronic; and combinations thereof.

Examples of the "binder" include corn, wheat, rice, starch paste produced using potato starch, gelatin, tragacanth, methylcellulose, hydroxypropylmethylcellulose, carboxymethylcellulose sodium, and/or polyvinylpyrrolidone.

Examples of the "disintegrant" include the starch, carboxymethyl starch, cross-linked polyvinylpyrrolidone, agar, alginic acid, alginic acid sodium, and salts thereof.

Examples of the "filler" include the sugar and/or calcium phosphate (for example, tricalcium phosphate or calcium hydrogenphosphate).

Examples of the "emulsifier" include a sorbitan fatty acid ester, glycerin fatty acid ester, sucrose fatty acid ester, and propylene glycol fatty acid ester.

Examples of the "lubricant" and the "agent for lubricating" include a silicic acid salt, talc, stearic acid salt, and polyethylene glycol.

Examples of the "stabilizer" include; agents for preventing oxidation, such as ascorbic acid and sulfite; and sugars, such as trehalose and dextrose.

Such a carrier may be suitably used, if desired. If desired, a pharmaceutical composition of the present invention may comprise, besides the above-described additives, flavoring agents, dissolution aids (solubilizers), suspending agents, diluents, surfactants, absorption promoters (e.g., quaternary ammonium salts, sodium lauryl sulfate, etc.), bulking agents, wetting agents, moisturizing agents (e.g., glycerin, starch, etc.), adsorbents (e.g., starch, lactose, kaolin, bentonite, colloidal silicic acid, etc.), disintegration inhibitors (e.g., sucrose, stearin, cocoa butter, hydrogenated oil, etc.), coating agents, coloring agents, preservatives, fragrances, flavorings, sweeteners, buffers, isotonicity agents, soothing agents, dissolution aids.

Examples of the "surfactant" include: an alkali metal salt, alkaline earth metal salt, and ammonium salt each of lignosulfonic acid, naphthalenesulfonic acid, phenolsulfonic acid, or dibutylnaphthalenesulfonic acid; alkylarylsulfonate; alkylsulfate; alkylsulfonate; fatty alcohol sulfate; fatty acid; and sulfated fatty alcohol glycol ether; and furthermore, a condensate of sulfonated naphthalene or a naphthalene derivative and formaldehyde; a condensate of naphthalene or naphthalenesulfonic acid and phenol or formaldehyde; polyoxyethyleneoctylphenyl ether; ethoxylated isooctylphenol; octylphenol; nonylphenol; alkylphenyl polyglycol ether; tributylphenyl polyglycol ether; tristearylphenyl polyglycol ether; alkylaryl polyether alcohol; a condensate of alcohol and fatty alcohol or ethylene oxide; ethoxylated castor oil; polyoxyethylene alkyl ether; ethoxylated polyoxypropylene; lauryl alcohol polyglycol ether acetal; sorbitol ester; lignosulfite waste liquor; and methylcellulose.

A pharmaceutical composition of the present embodiment may encompass one or more of the above-described carriers per pharmaceutical composition.

The dosage form of a pharmaceutical composition of the present invention is not particularly limited, subject to being a form that does not allow inactivation of an active ingredient, and allows achieving the pharmacological effect in an organism after administration. The dosage form usually depends on an administration method and/or a prescribing condition.

Examples of a dosage form suitable for oral administration include solid preparations (comprising tablets, pills, lingusorbs, capsules, drops, and troches), granules, dusting powder, powder, and liquid preparations. Furthermore, if desired, the solid preparation can take a coated dosage form known in the art, for example, a sugar-coated tablet, gelatin-coated tablet, enteric-coated tablet, film-coated tablet, bilayered tablet, or multilayered tablet.

The parenteral administration is subclassified into systemic administration and topical administration, and the topical administration is further subclassified into interstitial administration, transepidermal administration, transmucosal administration, and transrectal administration. The pharmaceutical composition can take a dosage form suitable for each administration method. Examples of a dosage form suitable for systemic or interstitial administration include an injection that is a liquid preparation. Examples of a dosage form suitable for transepidermal administration or transmucosal administration include liquid preparations (comprising liniments, eyedrops, nasal drops, and inhalants), suspending agents (comprising emulsions and creams), dusting powders (comprising nasal drops and inhalants), pastes, gels, ointments, and plasters. Examples of a dosage form suitable for transrectal administration include suppositories.

In this regard, the specific shape and size of each of the above-described dosage forms are not particularly limited. Any of the dosage forms may be in the scope of a dosage form known in the art.

To produce a pharmaceutical composition of the present invention, a formulation method known in the art may be applied, in principle. For example, the method described in Remington's Pharmaceutical Sciences (Merck Publishing Co., Easton, Pa.) can be used as a reference.

For example, in the case of an injection, a nucleic acid aptamer of the present invention is dissolved in a pharmaceutically acceptable solvent, a pharmaceutically acceptable carrier is added, if desired, and a method commonly used in the art can be used to produce an injection.

Examples of the "pharmaceutically acceptable solvent" include water, ethanol, propylene glycol, ethoxylated isostearyl alcohol, polyoxylated isostearyl alcohol, and polyoxyethylene sorbitan fatty acid esters. If desired, these are preferably adjusted to be isotonic with blood.

A pharmaceutical composition of the present invention can be administered in a pharmaceutically effective amount to an organism for the purpose of treating or preventing an object disease. An organism as an object of administration is a vertebrate, preferably a mammal, more preferably a human.

A pharmaceutical composition of the present invention may be administered through any of systemic administration and topical administration. Any of these can be suitably selected in accordance with the kind of disease, the site of onset, the degree of progression, or the like. For a disease at the site of onset of which is local, it is preferable to use topical administration in which direct administration is performed, through injection or the like, on the site of onset and an area around the site. This is because topical administration enables a nucleic acid aptamer of the present invention to be administered in a sufficient amount to a site (tissue or organ) to be treated, and in addition, is less prone to affect another tissue. On the other hand, in a case where a site to be treated is unidentifiable, or in a case where the onset of a disease is systemic, systemic administration through intravenous injection or the like is preferable, without limitation. This is because systemic administration allows a nucleic acid aptamer of the present invention to spread systemically via a blood flow, thus enabling the nucleic acid aptamer to be administered even to a lesion site undetectable by diagnosis.

A pharmaceutical composition of the present invention can be administered using a suitable method which does not deactivate an active ingredient. The administration may be, for example, parenteral (for example, with injection, aerosol, application, eyedrops, or nasal drops) or oral. Injection is preferable.

In the case of administration by injection, the site of injection is not particularly limited. The site may be any site, subject to enabling a DNA aptamer as an active ingredient to bind to a target substance. Examples include: administration; intramuscular administration; subcutaneous administration (comprising implantable, continuous, subcutaneous administration); intracutaneous administration; intravenous, intraarterial, intrahepatic, intramuscular, intraarticular, intramedullary, intrathecal, intraventricular, pulmonary, percutaneous, subcutaneous, intracutaneous, intraocular, intraperitoneal, and intravesical administration; transvaginal administration; rectal administration; inhalational or nasal drop administration; and tracheal/bronchial administration.

### <Method for treating and/or preventing>

In one aspect, a method for treating and/or preventing a disease, comprising administering a nucleic acid aptamer of the present invention or the above-described pharmaceutical composition to a subject (test subject), is provided.

Herein, an animal species that can be a "subject" or a "test subject" is, for example: a mammal, for example, a primate, such as a human or a chimpanzee; a laboratory animal, such as a rat or a mouse; a livestock animal, such as a pig, bovine, horse, sheep, or goat; or a pet animal, such as a dog or a cat. The test subject is preferably a human.

In a method of treatment and/or prevention according to the present aspect, a disease to be treated and/or prevented, the kind of a nucleic acid aptamer to be administered to a subject, and an administration method are in accordance with the above description of a pharmaceutical composition, and thus, detailed description is omitted here.

### <Agent for detecting, composition for detecting, and kit for detecting>

In one aspect of the present invention, an agent for detecting, a composition for detecting, and a kit for detecting are also provided. An agent for detecting according to the present invention is a drug for detecting a target protein in vivo or in vitro by utilizing the binding capacity of a nucleic acid aptamer of the present invention to the target protein. The target protein is IFN-γ, CRP, Thrombin, IL-6, IL-8, or HMGB1. For example, preliminarily labeling a nucleic acid aptamer with a fluorescent reagent or the like, followed by administering the resulting aptamer, makes it possible to determine the intensity of expression of any of the above-described target proteins in an organism, and in addition, to examine the localization of the target protein. This makes it possible to assist in diagnosing the above-described diseases. A nucleic acid aptamer of the present invention is also useful in imaging, tissue stain, and the like.

In one embodiment, the present invention relates to a composition for detecting a target protein, in which the composition comprises a nucleic acid aptamer of the present invention. The constitution of the composition is the same as the constitution of the above-described pharmaceutical composition, and thus, the description is omitted here.

In one aspect, the present invention relates to a kit for detecting a target protein, in which the kit comprises a nucleic acid aptamer of the present invention. A kit of the present invention may comprise a buffer, labeling reagent, and/or manual, in addition to a nucleic acid aptamer of the present invention.

### <Detecting method>

In one aspect, the present invention relates to a method for detecting any of the above-described target proteins. The present method comprises: a step of bringing a sample obtained from a test subject, into contact with a nucleic acid aptamer of the present invention; and a step of detecting any of the above-described target proteins on the basis of binding between the sample and the nucleic acid aptamer. The present method makes it possible to assist in diagnosing diseases associated with the above-described target proteins.

Examples of a sample to be used in the present method include tissues and biological samples. Examples of the tissue include a lesion site, brain, heart, liver, pancreas, lung, bone marrow, lymph node, and spleen. For example, a biopsy sample of such a tissue can be used. Examples of the biological sample include: bodily fluids (for example, blood, blood plasma, lymph liquid, tissue liquid, or urine); and cells, for example, peripheral blood cells, hair matrix cells, oral-cavity cells, nasal-cavity cells, intestinal cells, intravaginal cells, mucosal cells, and sputa (that may comprise alveolar cells, tracheal cells, or the like).

A detection step in a detecting method of the present invention is not particularly limited, subject to utilizing binding between a sample and a nucleic acid aptamer. A known method may be used. For example, an SPR method, quartz crystal microbalance method, nephelometric method, colorimetric method, or fluorescence method can be utilized.

SPR (surface plasmon resonance) refers to a phenomenon in which, on a thin metal film irradiated with a laser beam, the intensity of reflected light is markedly attenuated at a specific incidence angle (resonance angle). The SPR method is a measuring method to be performed utilizing this phenomenon. An adsorbate on a sensor portion, i.e., the surface of a thin metal film, can be measured with high sensitivity. In the present invention, for example, a nucleic acid aptamer of the present invention is preliminarily fixed on the surface of a thin metal film, a sample is allowed to pass on the surface of the thin metal film, a difference in an adsorbate on the surface of the metal between before and after the passing of the sample is generated by binding between the nucleic acid molecule and a target substance, and the difference is detected, whereby the target substance in the sample can be detected. As the SPR method, a substitution method, an indirect competition method, and the like are known. Any of these may be used.

The QCM (Quartz Crystal Microbalance) method is a method to be performed utilizing a phenomenon in which a substance is adsorbed in the surface of an electrode attached to a quartz oscillator, with the result that the resonance frequency of the quartz oscillator is decreased in accordance with the mass of the substance. A QCM sensor based on using this method can quantitatively seize a trace amount of adsorbate in accordance with the quantity of change in the quartz resonance frequency. In the present invention, a DNA aptamer is preliminarily fixed on the surface of an electrode in the same manner as in the SPR method, and a sample was brought into contact with the surface of the electrode, whereby a target substance in the sample can be quantitatively detected in accordance with the quantity of change caused in the quartz resonance frequency by binding between the DNA aptamer and the target substance. This technology is well known in the art. For example, Christopher J., et al. (2005) Self-Assembled Monolayers of a Form of Nanotechnology, Chemical Review, 105: 1103-1169 may be used as a reference.

A nephelometric method is a method in which a solution is irradiated with light, the attenuation of the light scattered by a substance floating in the solution is optically measured, or the light transmitted by the solution is optically measured, using a colorimeter or the like to measure the amount of the substance in the solution. In the present invention, measuring the absorbance before and after adding a nucleic acid aptamer of the present invention to a sample makes it possible to quantitatively detect the target substance in the sample.

In addition, an antibody to a target substance is used together to enable the target substance to be detected. For example, a method in which the sandwich method of ELISA is applied may be used. In this method, a nucleic acid aptamer of the present invention is first fixed on a solid-phase carrier, and next, a sample is added so that a target substance present in the sample can be bound to the nucleic acid aptamer. Subsequently, the sample was rinsed, and then, an anti-target-substance antibody is added to bind to the target substance. The resulting sample is washed. Then, using a secondary antibody suitably labeled, the anti-target-substance antibody is detected, whereby the target substance in the sample can be detected. Examples of the solid-phase carrier that can be used include an insoluble carrier in the shape of beads, a microplate, test tube, stick, test piece, or the like consisting of a material, such as polystyrene, polycarbonate, polyvinyl toluene, polypropylene, polyethylene, polyvinyl chloride, nylon, polymethacrylate, latex, gelatin, agarose, cellulose, Sepharose, glass, metal, ceramics, or magnetic material.

In addition, an antibody to a target substance is used together to enable the target substance to be detected. For example, a method in which the sandwich method of ELISA is applied may be used.

In a case where a target protein is detected using the detection step of the present aspect, a test subject from which a sample is derived is revealed to have a disease associated with the target protein.

In one embodiment, any of the above-described agent for detecting, composition for detecting, kit for detecting, or detecting method can detect an inflammation or an inflammatory disease, and in addition, can measure an inflammatory level in an inflammation or an inflammatory disease.

In a further embodiment, the inflammatory disease may be an infection, collagen disease, cardiovascular disease, or tumor. In addition, the inflammation may be associated with tissue damage, necrosis, or surgery. In this case, the agent for detecting, composition for detecting, and kit for detecting can comprise the above-described (3) a nucleic acid aptamer that binds to CRP. In addition, the above-described detecting method can include using the above-described (3) a nucleic acid aptamer that binds to CRP.

In one further aspect of the present invention, any of the above-described nucleic acid aptamers of the present invention is also provided for use in the treatment and/or prevention of a disease in a test subject such as a human. Furthermore, the present invention provides any of the above-described nucleic acid aptamers of the present invention in the production of a drug for the treatment and/or prevention of a disease.

In one further aspect, the present invention relates to a method for assisting in diagnosing any or no contraction of a disease in a test subject. The present method comprises: a step of administering a nucleic acid aptamer of the present invention to a test subject; and a step of detecting the nucleic acid aptamer. For example, in a case where a nucleic acid aptamer at a high concentration has been detected at a specific site in an organism, it can be judged that a disease has been generated at the site. In the detection step, a known method may be used. For example, the above-described fluorescence method may be used.

### Examples

### <Example 1: Investigation of compatibility between Px base and Pa' base>

### (Purpose)

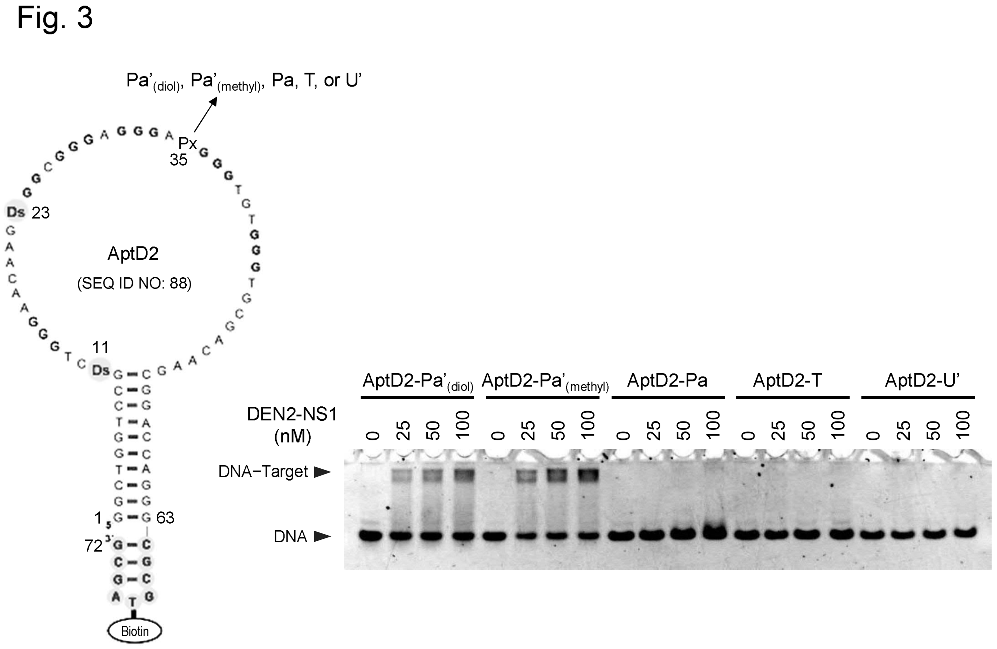

In the AptD2 aptamer (SEQ ID NO: 88) that binds to Dengue Virus NS1 Protein Serotype 2 (hereinafter referred to as "DEN2-NS1 protein"), and that was developed in the past by the present inventors, using ExSELEX on the basis of a five-letter DNA library, an unnatural base shown in the following general formula (III) in which the R¹ group is the below-described general formula (XXIV) (4-(4-pentyne-1,2-diol)-1-propynyl-2-nitropyrrole; hereinafter referred to as "Px_{(diol)}"), in addition to an unnatural base shown in the following general formula (I) (7-(2-thienyl)-3*H*-imidazo[4,5-b]pyridine-3-yl; hereinafter referred to as "Ds"), was found to be incorporated in the form of a mutation in a PCR amplification process in ExSELEX (the left diagram in Figure 3,; Matsunaga, K. et al., Nucleic Acids Res., 49, 11407-11424 (2021).).

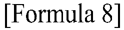

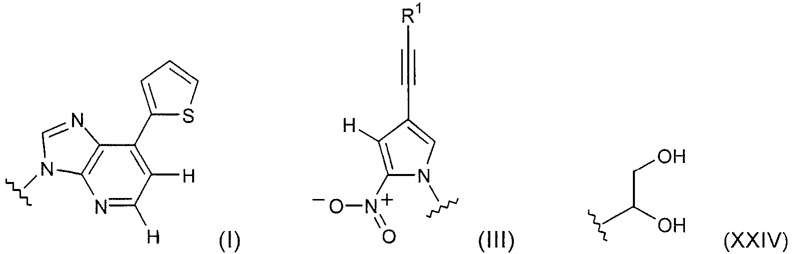

This Example investigates the effect of substituting the Px_{(diol)} base of the above-described AptD2 aptamer with any of the different unnatural bases and natural bases on the binding capacity of the aptamer to the DEN2-NS1 protein.

### (Method and results)

### (1) Production of modified AptD2 aptamer

The Px_{(diol)} base of the AptD2 aptamer was substituted with (i) an unnatural base shown in the following general formula (II) in which the R¹ group is the above-described general formula (XXIV) (the unnatural base is 4-(4-pentyne-1,2-diol)-1-propynylpyrrole-2-carbaldehyde; hereinafter referred to as "Pa'_{(diol)}"), (ii) an unnatural base shown in the following general formula (II) in which the R¹ group is a methyl group (the unnatural base is 4-propynylpyrrole-2-carbaldehyde; hereinafter referred to as "Pa'_{(methyl)}"), (iii) an unnatural base shown in Figure 1D (pyrrole-2-carbaldehyde; hereinafter referred to as "Pa"), (iv) the thymidine base, or (v) a 5-(1-propynyl)uridine base (hereinafter referred to as "U"'), and furthermore, a biotin-bound mini-hairpin sequence 5'-CGCG(Bio-T)AGCG-3' was allowed to bind to the 3' end, whereby each of the AptD2-Pa'_{(diol)} aptamer, AptD2-Pa'_{(methyl)} aptamer, AptD2-Pa aptamer, AptD2-T aptamer, and AptD2-U' aptamer was produced in the form of a modified AptD2 aptamer (the left diagram in Figure 3). In this regard, the bases and the nucleotides (dDsTP, dPa'_{(diol)} TP, dPa'_{(methyl)} TP, dPx_{(diol)} TP, dPaTP, dPx_{(methyl)} TP, and the like) comprising the respective bases were synthesized using the method described in the literature listed as follows: Hirao, I. et al., Nat. Methods, 3: 729-735 (2006); Kimoto, M. et al., Biopolymers, 112: e23407 (2021); Mitsui, T. et al., Bioorg. Med. Chem. Lett. 13: 4515-4518 (2003); and Mitsui, T. et al., J. Am. Chem. Soc., 125: 5298-5307 (2003).

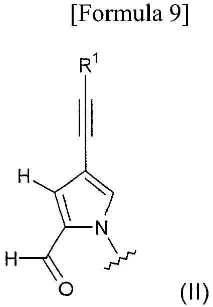

The specific structures of the above-described (i) to (v) are shown below.

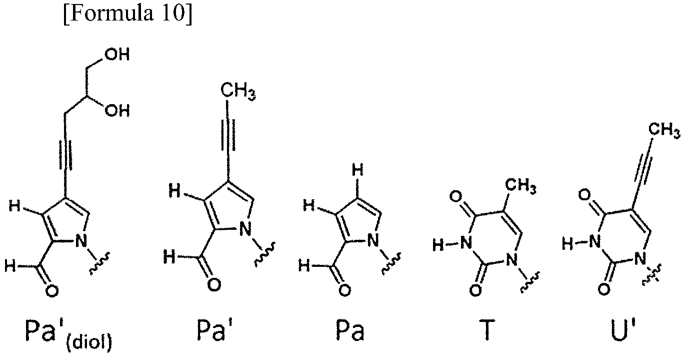

The modified AptD2 aptamers were each chemically synthesized with a DNA/RNA synthesizer using phosphoramidite chemistry, and using a commercially available natural base amidite, a biotin-dT amidite, and an unnatural base amidite synthesized by the method described in the above-described literature. The nucleic acid aptamers were deprotected with a concentrated ammonia solution. Then, the resulting full-length DNA fragments were purified using denaturing polyacrylamide gel electrophoresis.

### (2) Evaluation of binding capacity on the basis of EMSA

The binding capacity of each modified AptD2 aptamer to the DEN2-NS1 protein as a target protein was analyzed by EMSA (Electrophoresis gel-mobility shift assays). Specifically, each modified AptD2 aptamer was diluted with a binding buffer (20 mM Tris-HCl, pH 7.5, 150 mM NaCl, 1 mM MgCl₂, and 2.7 mM KCl), heated at 90°C for 5 minutes, and then cooled for 10 minutes. This DNA solution was mixed with DEN2-NS1 protein in a binding buffer having 0.05% Nonidet P-40 added thereto. The resulting mixture was incubated at 25°C for 30 minutes, and each aptamer (50 nM) was thus allowed to bind to the DEN2-NS1 protein (0 nM, 25 nM, 50 nM, or 100 nM). Then, the sample was mixed with glycerol having a final concentration of 5%, and the resulting mixture was electrophoresed in native 4% acrylamide gel. The gel was stained with SYBR Gold, and subjected to detection using an LAS-4000 imager.

Figure 3 shows the results of evaluation of the binding of each modified AptD2 aptamer to the DEN2-NS1 protein. The AptD2-Pa'_{(diol)} aptamer and the AptD2-Pa'_{(methyl)} aptamer exhibited a band corresponding to a complex with the DEN2-NS1 protein. On the basis of comparison of the band densities, the band of the AptD2-Pa'_{(methyl)} aptamer exhibited stronger binding than the band of the AptD2-Pa'_{(diol)} aptamer. On the other hand, in the case of the AptD2-Pa aptamer, the AptD2-T aptamer, and the AptD2-U' aptamer, binding to the DEN2-NS1 protein was not detected.

### (3) Evaluation of binding affinity on the basis of SPR

The binding affinity profile of each modified AptD2 aptamer to the DEN2-NS1 protein as a target protein was analyzed by SPR (surface plasmon resonance). Specifically, Biacore T200 (Cytiva) was used to make an SPR analysis. The binding of each nucleic acid aptamer at 0.5 nM with respect to the target protein at 1.25 nM, 2.5 nM, 5 nM, 10 nM, or 20 nM was evaluated at 25°C to determine the *K*_{D} value. According to the analysis based on SPR, the *K*_{D} value of the AptD2-Pa'_{(diol)} aptamer was 115 pM, and the *K*_{D} value of the AptD2-Pa'_{(methyl)} aptamer was 69.5 pM, thus verifying that the binding capacity of the AptD2-Pa'_{(methyl)} aptamer was higher. The above-described results have revealed that the propynyl group bound to pyrrole is important for binding to the target.

The above-described results have suggested a new ExSELEX method based on mutually combining different advantages of two different unnatural bases having compatibility. A nucleotide comprising the Px_{(methyl)} base (2-nitro-4-propynylpyrrole; an unnatural base shown in the above-described general formula (III) in which the R¹ group is a methyl group) is unstable under basic conditions used in a step of nucleic acid synthesis, but in a step of PCR amplification, the nucleotide has high fidelity on the basis of the formation of an unnatural base pair with the Ds base. On the other hand, a nucleotide comprising the Pa'_{(methyl)} base is stable even under basic conditions. Because of this, as the sixth letter used in ExSELEX in the following Examples, the Pa'_{(methyl)} base was used in synthesis of a nucleic acid aptamer, and Px_{(methyl)} was used in PCR amplification. In this regard, in the following Examples, the Pa'_{(methyl)} base is abbreviated as a "Pa' base", and the Px_{(methyl)} base is abbreviated as a "Px base".

### <Example 2: Production of six-letter DNA library>

### (Purpose)

To perform an ExSELEX method in which two kinds of unnatural bases were used in addition to four natural bases (the method is hereinafter referred to as "six-letter ExSELEX" to be distinguished from conventional five-letter ExSELEX), a DNA library comprising six kinds of bases was produced.

### (Method and results)

An XL1 library and an XL2 library were produced in the form of two different libraries having two Ds bases located at different positions in the respective 29 mer random regions consisting of five kinds of bases, i.e., the Pa' base in addition to the A base, G base, C base, and T base. In this regard, in the 27 bases resulting from excluding two Ds bases from the 29 mer random region, the ratio of introduction of each of the A base, G base, C base, and T base was set at 24%, and the ratio of introduction of the Pa' base was set at 4%.

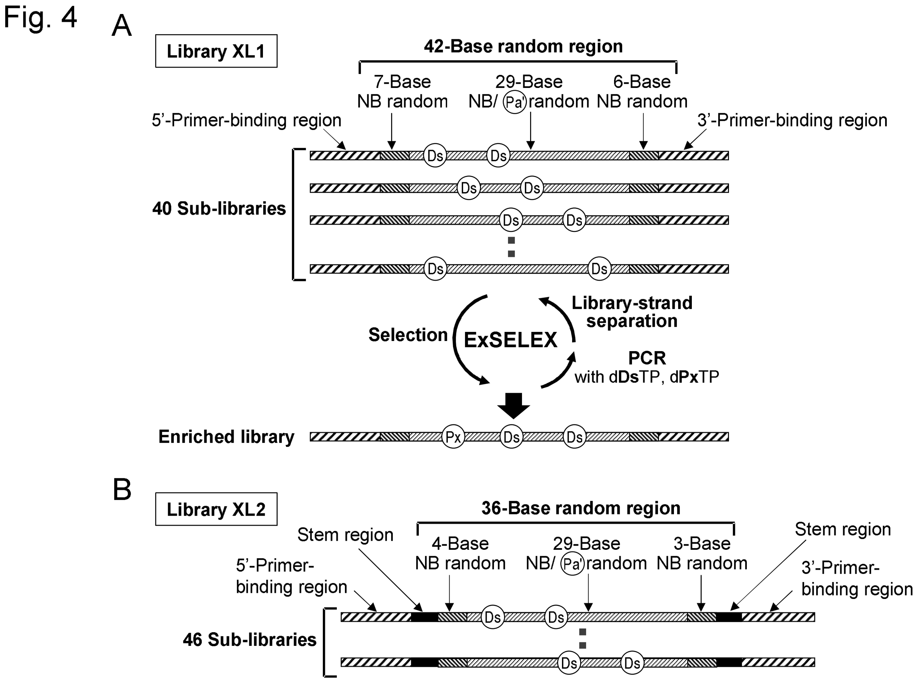

The XL1 library comprises a region consisting of seven bases located on the 5' side of the random region and a region consisting of six bases located on the 3' side of the random region, and comprises a primer-binding sequence for PCR amplification on each of the 5' side and the 3' side further from the regions. The sequence of the XL1 library is shown as 5'-TGATTAGCTGAGACCTGATGCT-N₄₂-GACAAGCGGAGTAGTTAGACCGT-3' (SEQ ID NO: 173), and consists of 40 kinds of sublibraries (Figure 4A).

The XL2 library comprises a region consisting of four bases located on the 5' side of the random region and a region consisting of three bases located on the 3' side of the random region, and comprises a 5 mer mutually complementary stem sequence and a primer-binding sequence for PCR amplification on each of the 5' side and the 3' side further from the regions. The sequence of the XL2 library is shown as 5'-GCTCATTCGCCTCTCCTATTCTCACG-N₃₆-CGTGACAAGCGGAGTAGTTAGACCGT-3' (SEQ ID NO: 174), and consists of 46 kinds of sublibraries (Figure 4B).

Each sublibrary was chemically synthesized using a standard phosphoramidite method with amidites of four natural bases, Ds and Pa', and purified with denaturing polyacrylamide gel electrophoresis (PAGE). In ExSELEX in the following Examples, the sublibraries mixed in equivalent amounts were used.

### <Example 3: Human IFN-γ-targeting nucleic acid aptamer>

### (Purpose)

Six-letter ExSELEX was performed for the purpose of creating a nucleic acid aptamer that binds to human IFN-γ.

### (Method and results)

Below, the XL1 library and the XL2 library produced in Example 2 were used in six-letter ExSELEX.

### (1) Six-letter ExSELEX for XL1 library

### (a) Isolation of novel nucleic acid aptamer

Six-letter ExSELEX was performed, using, as a target protein, a commercially available glycosylated human IFN-γ (IFG-H4211, ACROBiosystems Co. Ltd.) expressed in a HEK293 cell, and using the XL1 library. As a first selection, a 3.6 nmol library (comprising 2.2 × 10¹⁵ different sequences consisting of six bases) was used. The library at 50 nM or 100 nM was rapidly cooled from 90°C to 4°C, and then mixed with the target protein.

A library in each stage of six-letter ExSELEX was incubated with a target protein in a binding buffer, and the target protein was biotinylated with an NHS-activated biotin reagent. A biotinylated protein having a DNA fragment bound thereto was captured with streptavidin-bound magnetic beads, and a DNA fragment in a complex on the beads was eluted with 150 mM NaOH. The DNA fragment isolated was amplified by PCR using dDsTP (deoxynucleoside triphosphate comprising a Ds base) and dPxTP (deoxynucleoside triphosphate comprising a Px base) in addition to dNTP. The Pa' base in the first library was substituted with Px during the PCR amplification, and used in the next selection. After the fifth to seventh selection, the DNA-protein complex on the beads was washed with 2 to 3 M urea. After the seventh selection, it was verified using EMSA that the library concentrated bound to a target protein. As the final eighth selection, the DNA fragment bound was isolated from a complex in a band that shifted in the gel.

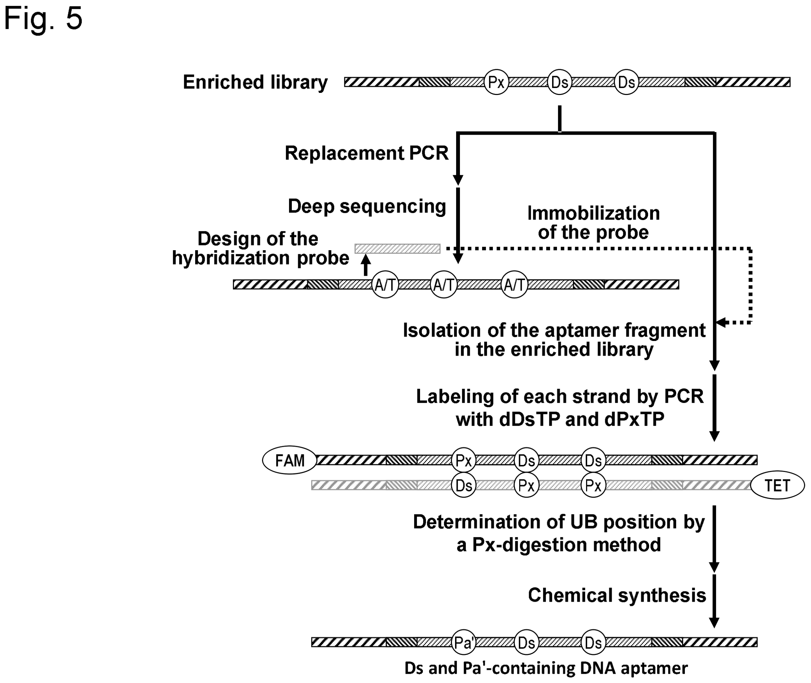

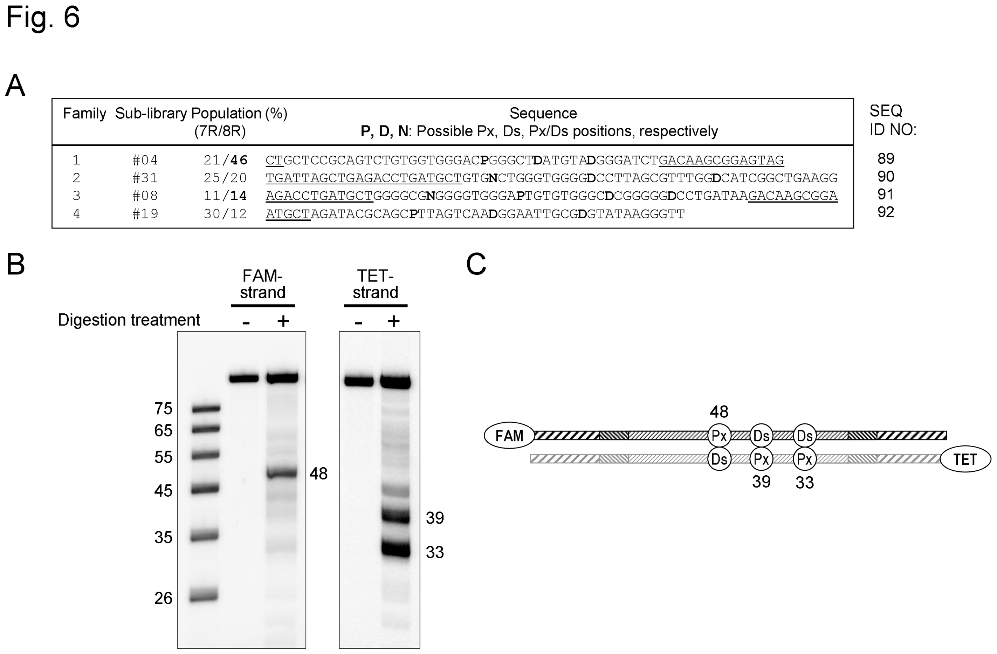

The concentrated library after the seventh selection (R7) and the concentrated library after the eighth selection (R8), obtained from the XL1 library, were amplified using a replacement PCR method in which an unnatural base in the library was replaced with a natural base. In deep sequencing performed using an Ion-PGM system, 87,013 (R7) and 78,581 (R8) extracted sequence reads were obtained. The sequence reads were classified into a family having similar sequences. Higher-level families larger in the number of reads are shown in Figure 6A. In each sequence, complementary sequences were recognized at the 5' end side and the 3' end side. It was possible that the unnatural base was substituted with the A base and the T base by the above-described replacement PCR, and that the positions of the Pa'/Px bases and the Ds base were determined on the basis of the position of the Ds base in the first sublibrary. Among the sequences shown in Figure 6A, the sequence of Family 1 was further analyzed using the method shown in Figure 5.

A nucleic acid aptamer that belongs to Family 1 was isolated using a probe. Then, PCR amplification was performed using an FAM-labeled forward primer and a TET-labeled reverse primer, and using, as a template, the concentrated library (R8) after the eighth selection. Furthermore, the amplification product obtained was partly digested at the position of the Px base under basic conditions, and electrophoresed in modified gel. Then, a nucleic acid fragment bound to the FAM or the TET was fluorescently detected (Figure 6B). The results shown in Figure 6B have revealed that position 48 of the FAM-bound strand and position 33 and position 39 of the TET-bound strand were the Px bases, and the complementary bases of these positions are the Ds bases (Figure 6C).

### (b) Production of nucleic acid aptamer

The sequence of Family 1, determined as described above, was trimmed. The end stem region was optimized. The Px base was substituted with the Pa' base to produce AptIFN-γ-1 and AptIFN-γ-3 (Table 1). Furthermore, a biotinylated mini-hairpin DNA was allowed to bind to produce Bio-AptIFN-γ-3. AptIFN-γ-2, AptIFN-γ-4, AptIFN-γ-5, and AptIFN-γ-6 (Table 1) were also produced in the form of nucleic acid aptamers obtained by substituting all or part of the unnatural bases with natural bases.

**[Table 1]**

| Table 1: IFN-γ-targeting nucleic acid aptamers derived from XL1 library | | |
|---|---|---|
| Name of nucleic acid aptamer | Base sequence of nucleic acid aptamer (5'-3') | SEQ ID NO: |
| AptIFNγ-1 | CTCCGC AGTC TGTGGTGGGACPa'GGGCTDsATGTADsGGGATCT GACAA GCGGAG | 1 |
| AptIFNγ-2 | CTCCGC AGTC TGTGGTGGGACT GGGCTA ATGTAA GGGATCT GACAA GCGGAG | 136 |
| AptIFNγ-3 | CTCCGC GTC TGTGGTGGGACPa'GGGCTDsATGTADsGGGATCT GAC GCGGAG | 3 |
| AptIFNγ-4 | CTCCGC GTC TGTGGTGGGACT GGGCTDsATGTADsGGGATCT GAC GCGGAG | 4 |
| AptIFNγ-5 | CTCCGC GTC TGTGGTGGGACPa'GGGCTA ATGTADsGGGATCT GAC GCGGAG | 6 |
| AptIFNγ-6 | CTCCGC GTC TGTGGTGGGACPa'GGGCTDsATGTAA GGGATCT GAC GCGGAG | 137 |
| Bio-AptIFNγ-3 | CTCCGC GTC TGTGGTGGGACPa'GGGCTDsATGTADsGGGATCT GAC GCGGAG CGCGLAGCG | 138 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Pa'" represents a Pa' base. | | |

### (c) Evaluation of affinity to human glycosylated IFN-γ

The affinity of the IFN-γ-targeting nucleic acid aptamer shown in Table 1 above to human glycosylated IFN-γ was analyzed using EMSA. The nucleic acid aptamer was incubated with the human glycosylated IFN-γ at 25°C for 30 minutes in a binding buffer supplemented with 0.05% Nonidet P-40 or Tween 20. The sample solution was mixed with glycerol (at a final concentration of 5%), and the resulting complex was analyzed with native 8% PAGE at 26 to 28°C. The other conditions were the same as in (2) in Example 1.

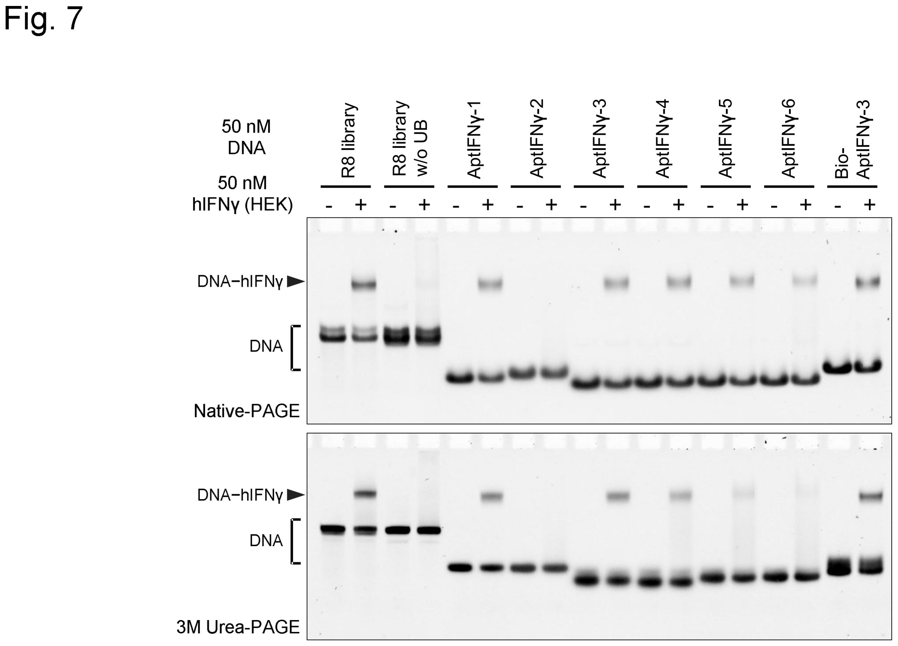

The results are shown in Figure 7. The nucleic acid aptamers (AptIFN-γ-1, AptIFN-γ-3, and Bio-AptIFN-γ-3) comprising two Ds and one Pa' bound strongly to the glycosylated IFN-γ, but the nucleic acid aptamers not comprising these unnatural bases did not exhibit a band shift.

### (d) Comparison with nucleic acid aptamer developed in the past

Next, the above-described novel nucleic acid aptamers were compared with AptDs-IFN-γ that is an IFN-γ-targeting nucleic acid aptamer developed using five-letter ExSELEX in the past, and with a biotinylated mini-hairpin DNA conjugate (Bio-AptDs-IFN-γ) of the AptDs-IFN-γ. AptDs-IFN-γ and Bio-AptDs-IFN-γ are aptamers isolated using the five-letter ExSELEX, and using, as a target protein, a non-glycosylated human IFN-γ expressed in *Escherichia coli.* The sequences of the aptamers are shown in Table 2 below.

**[Table 2]**

| Table 2: IFN-γ-targeting nucleic acid aptamers as controls for comparison | | |
|---|---|---|
| Name of nucleic acid aptamer | Base sequence of nucleic acid aptamer (5'-3') | SEQ ID NO: |
| AptDs-IFNγ | CCCGCC CGGGTCCGCGAAGCGGTAGGTDsTGGCTAGGCDsGCT GGCGGG | 139 |
| Bio-AptDs-IFNγ | CCCGCC CGGGTCCGCGAAGCGGTAGGTDsTGGCTAGGCDsGCT GGCGGG CGCGLAGCG | 140 |

| | | |
|---|---|---|
| * The underlines denote sequences complementary to each other, and capable of forming a stem structure; the black frame denotes a hairpin sequence; "L" in the sequence represents Biotin-dT; "and Ds" represents a Ds base. | | |

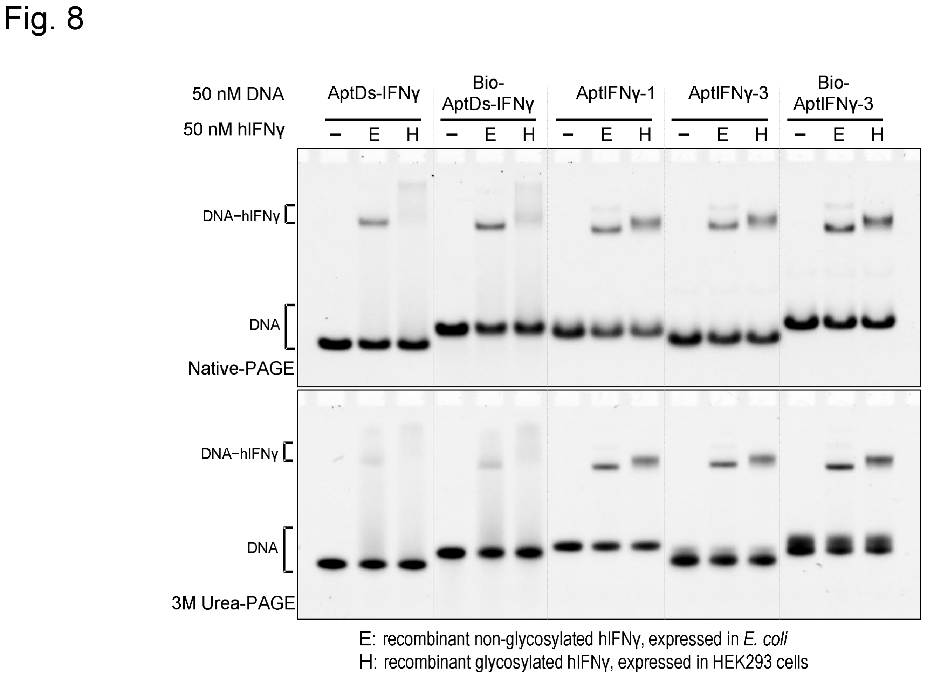

Regarding AptIFN-γ-1, AptIFN-γ-3, and Bio-AptIFN-γ-3 that were derived from the XL1 library, and AptDs-IFN-γ and Bio-AptDs-IFN-γ as comparative controls, EMSA was performed in the same manner as the above-described method. The results are shown in Figure 8. In this regard, as the non-glycosylated IFN-γ, IFN-γ (300-02, Peprotech) expressed in *Escherichia coli* was used. It has been revealed that AptDs-IFN-γ and Bio-AptDs-IFN-γ exhibited a significant decrease in binding to glycosylated IFN-γ, but that on the other hand, AptIFN-γ-1, AptIFN-γ-3, and Bio-AptIFN-γ-3 bound efficiently to both glycosylated IFN-γ and non-glycosylated IFN-γ.

### (2) Six-letter ExSELEX for XL2 library

### (a) Isolation of novel nucleic acid aptamer

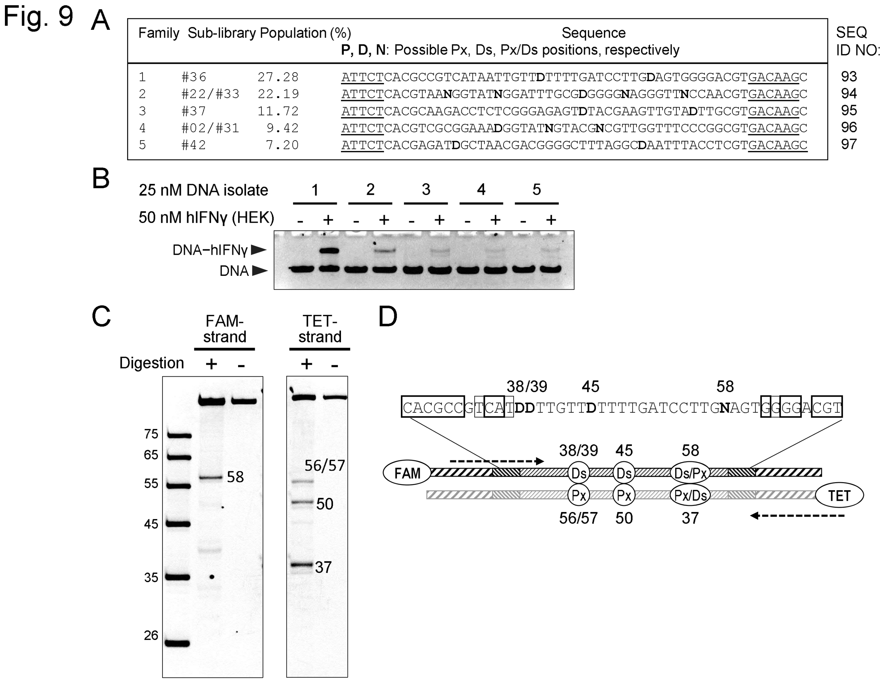

Six-letter ExSELEX was performed on the XL2 library, using a glycosylated IFN-γ as a target protein. The concentrated library after the seventh selection was sequenced using the Ion-PGM sequencer system. The sequences of the higher-level families larger in the number of reads are shown in Figure 9A. Among these, only Family 1 exhibited high affinity to the target protein in the analysis by EMSA (Figure 9B), and thus, Family 1 was analyzed by the same method as in (1) above to determine the positions of the Ds base and the Px base (Figure 9C). The results of the analysis have revealed that Family 1 comprises the Ds bases at positions 38/39 and position 45, and comprises the Ds base or the Px base at position 58 (Figure 9D).

### (b) Production of nucleic acid aptamer

On the basis of the results of (a) above, 10 kinds of nucleic acid aptamers shown in Table 3 below were produced.

**[Table 3]**

| Table 3: IFN-γ-targeting nucleic acid aptamers derived from XL2 library | | |
|---|---|---|
| Name of nucleic acid aptamer | Base sequence of nucleic acid aptamer (5'-3') | SEQ ID NO: |
| XL2-01a | GTCCTCACG CCGT CATA A TTGTTDs TTTTGAT CCTTGDs AGTGGGGA CGTGAGGAC | 8 |
| XL2-01b | GTCCTCACG CCGPa'CATA A TTGTTDs TTTTGAT CCTTGDs AGTGGGGA CGTGAGGAC | 141 |
| XL2-01c | GTCCTCACG CCGT CATPa'A TTGTTDs TTTTGAT CCTTGDs AGTGGGGA CGTGAGGAC | 14 |
| XL2-01d | GTCCTCACG CCGT CATA Pa'TTGTTDs TTTTGAT CCTTGDs AGTGGGGA CGTGAGGAC | 16 |
| XL2-01e | GTCCTCACG CCGT CATA A TTGTTDs TTTTGAPa'CCTTGDs AGTGGGGA CGTGAGGAC | 142 |
| XL2-01h | GTCCTCACG CCGT CATDs A TTGTTDs TTTTGAT CCTTGDs AGTGGGGA CGTGAGGAC | 10 |
| XL2-01i | GTCCTCACG CCGT CATA Ds TTGTTDs TTTTGAT CCTTGDs AGTGGGGA CGTGAGGAC | 12 |
| XL2-01j | GTCCTCACG CCGT CATA A TTGTTDs TTTTGAT CCTTGPa'AGTGGGGA CGTGAGGAC | 143 |
| XL2-01k | GTCCTCACG CCGT CATDs A TTGTTDs TTTTGAT CCTTGPa'AGTGGGGA CGTGAGGAC | 144 |
| XL2-01l | GTCCTCACG CCGT CATA DsTTGTTDs TTTTGAT CCTTGPa'AGTGGGGA CGTGAGGAC | 145 |

| | | |
|---|---|---|
| * The underlines denote sequences complementary to each other, and capable of forming a stem structure; "Ds" in the sequence represents a Ds base; and "Pa'" represents a Pa' base. | | |

The affinity of the IFN-γ-targeting nucleic acid aptamer shown in Table 3 above to glycosylated IFN-γ and non-glycosylated IFN-γ was analyzed using EMSA. The nucleic acid aptamer was incubated with IFN-γ at 25°C for 30 minutes in a binding buffer supplemented with 0.05% Nonidet P-40 or Tween 20. The sample solution was mixed with glycerol (at a final concentration of 5%), and the resulting complex was analyzed with native 8% PAGE at 26 to 28°C. The other conditions were the same as in (2) in Example 1.

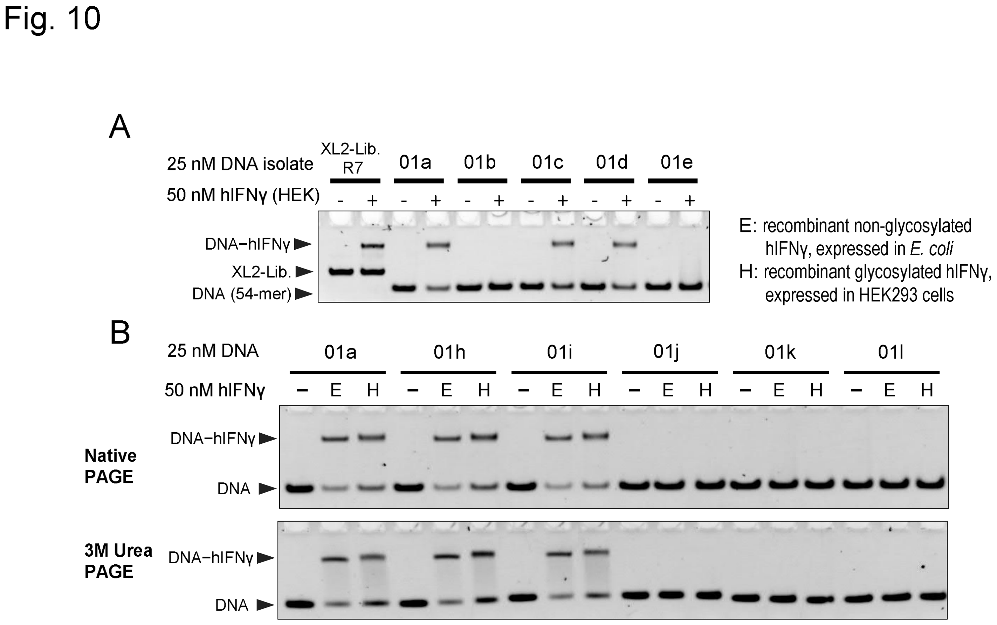

The results are shown in Figure 10. XL2-01a, XL2-01h, and XL2-011 exhibited the highest affinity, and exhibited high affinity to glycosylated IFN-γ and non-glycosylated IFN-γ also in the 3M urea gel.

Next, a biotin-bound mini-hairpin was linked to XL2-01a and XL2-01i to produce IFN-γ-targeting nucleic acid aptamers shown in Table 4 below.

**[Table 4]**

| Table 4: IFN-γ-targeting nucleic acid aptamers with biotin-bound mini-hairpin linked thereto | | |
|---|---|---|
| Name of nucleic acid aptamer | Base sequence of nucleic acid aptamer (5'-3') | SEQ ID NO: |
| Bio-XL2-01a | GTCCTCACG CCGTCATAA TTGTTDs TTTTGATCCTTGDsAGTGGGGA CGTGAGGAC CGCGLAGCG | 146 |
| Bio-XL2-01i | GTCCTCACG CCGTCATADsTTGTTDs TTTTGATCCTTGDsAGTGGGGA CGTGAGGAC CGCGLAGCG | 147 |
| Bio-AptDs-IFNγ | CCCGCC CGGGTCCGCGAAGCGGTAGGTDsTGGGCTAGGCDsGCT GGCGGG CGCGLAGCG | 148 |

| | | |
|---|---|---|
| * The underlines denote sequences complementary to each other, and capable of forming a stem structure; the black frame denotes a hairpin sequence; and "L" in the sequence represents Biotin-dT. | | |

The affinity of the IFN-γ-targeting nucleic acid aptamer shown in Table 4 above to glycosylated IFN-γ was analyzed using ELONA (enzyme-linked oligonucleotides assay). Specifically, human IFN-γ (0 to 25 nM) was fixed in a 96-well maxisorp plate, and blocked with 1% BSA. A biotinylated aptamer (100 nM/100 µL well) as a primary detector was added, and bound for 1 hour. Then, SA-HRP as a secondary detector was bound for 30 minutes to allow coloring detection. Reaction between HRP and TMB was allowed for 30 minutes. The reaction was terminated with 1 N HCl. Absorbance at 450 nm was measured using NanoDrop.

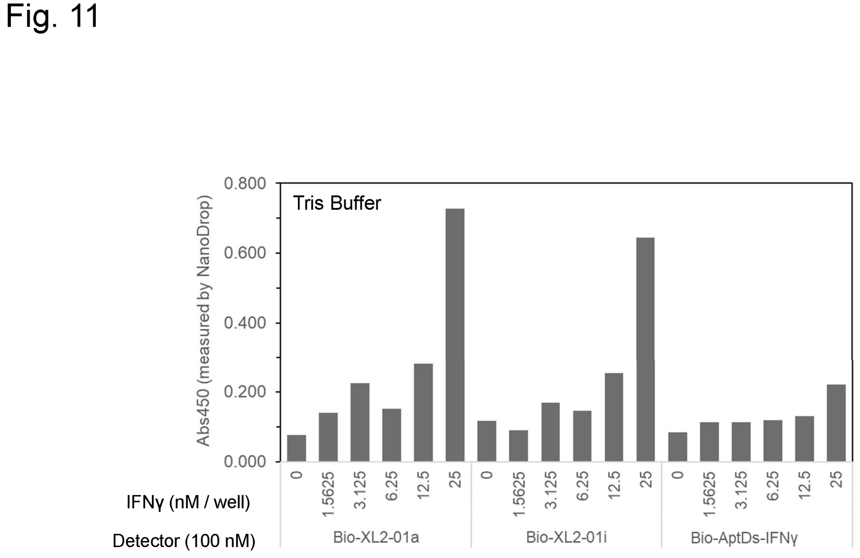

The results are shown in Figure 11. With Bio-XL2-01a and Bio-XL2-01i, a markedly higher signal intensity was observed, compared with Bio-AptDs-IFN-γ obtained using non-glycosylated IFN-γ as a target through the five-letter ExSELEX.

### <Example 4: Nucleic acid aptamer that binds to CRP, Thrombin, IL-6, IL-8, or HMGB1>

### (Purpose)

Six-letter ExSELEX was performed for the purpose of creating a nucleic acid aptamer that binds to human-derived CRP (C-reactive protein), Thrombin, IL-6, IL-8, or HMGB1.

### (Method and results)

### (1) CRP-targeting nucleic acid aptamer

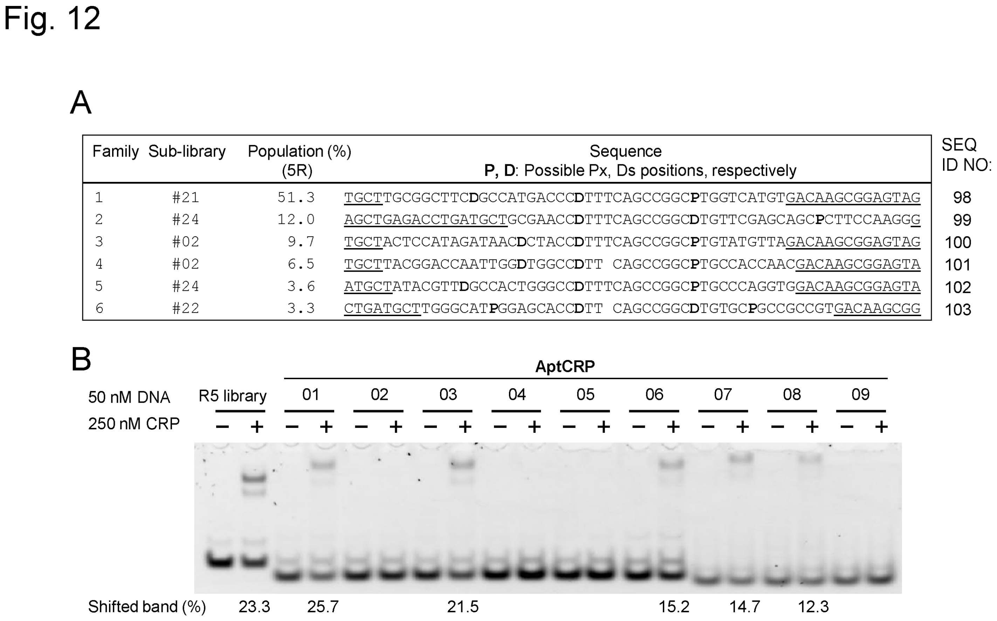

In accordance with the same method as in Example 3, six-letter ExSELEX was performed using, as a target protein, CRP (C4063, Sigma-Aldrich) isolated from human blood plasma, and using the XL1 library. However, no urea solution was used in washing. After the fifth selection, a target binding was studied using EMSA. The concentrated library was amplified by replacement PCR, and then sequenced using the Ion PGM system. Furthermore, the positions of Ds and Ps were sequenced. Many of the 61,906 sequences had the consensus sequence CDsTT(T)CAGCCGGCPxT between a pair of complementary sequences, and 51.3% of the sequences converged into the sequence of Family 1 shown in Figure 12A. On the basis of this sequence of Family 1, the nucleic acid aptamers shown in Table 5 below were chemically synthesized.

**[Table 5]**

| Table 5: CRP-targeting nucleic acid aptamers | | |
|---|---|---|
| Name of nucleic acid aptamer | Base sequence of nucleic acid aptamer (5'-3') | SEQ ID NO: |
| AptCRP-01 | GCTGCTT CDsGC CATGACC CDsTTTCAGCCGGCPa'T GGTCATG TGAC AAGCAGC | 20 |
| AptCRP-02 | GCTGCTT CDsGC CATGACC CDsTTTCAGCCGGCDs T GGTCATG TGAC AAGCAGC | 149 |
| AptCRP-03 | GCTGCTT CA GC CATGACC CDsTTTCAGCCGGCPa'T GGTCATG TGAC AAGCAGC | 22 |
| AptCRP-04 | GCTGCTT CDsGC CATGACC CA TTTCAGCCGGCPa'T GGTCATG TGAC AAGCAGC | 150 |
| AptCRP-05 | GCTGCTT CDsGC CATGACC CDsTTTCAGCCGGCT T GGTCATG TGAC AAGCAGC | 151 |
| AptCRP-06 | GCTGCTT CDsGC CATGACC CDs TTCAGCCGGCPa'T GGTCATG TGAC AAGCAGC | 23 |
| AptCRP-07 | CGCTGCATGACC CDsTTTCAGCCGGCPa'T GGTCATGCAGCG | 25 |
| AptCRP-08 | CGCTGCATGACC CDs TTCAGCCGGCPa'T GGTCATGCAGCG | 26 |
| AptCRP-09 | CGCTGCATGACC CDs TTCAGCCGGCDs T GGTCATGCAGCG | 152 |

| | | |
|---|---|---|
| * The underlines denote sequences complementary to each other, and capable of forming a stem structure; "Ds" in the sequence represents a Ds base: and "Pa'" represents a Pa' base. | | |

The results obtained by analyzing the affinity of the CRP-targeting nucleic acid aptamers shown in Table 5 above to CRP, using EMSA, are shown in Figure 12B. AptCRP-01, AptCRP-03, AptCRP-06, AptCRP-07, and AptCRP-08 exhibited a band shift, and the intensity of the band shift was larger in this order. It has been revealed that particularly AptCRP-01 and AptCRP-03 bind strongly to CRP.

### (2) Thrombin-targeting nucleic acid aptamer

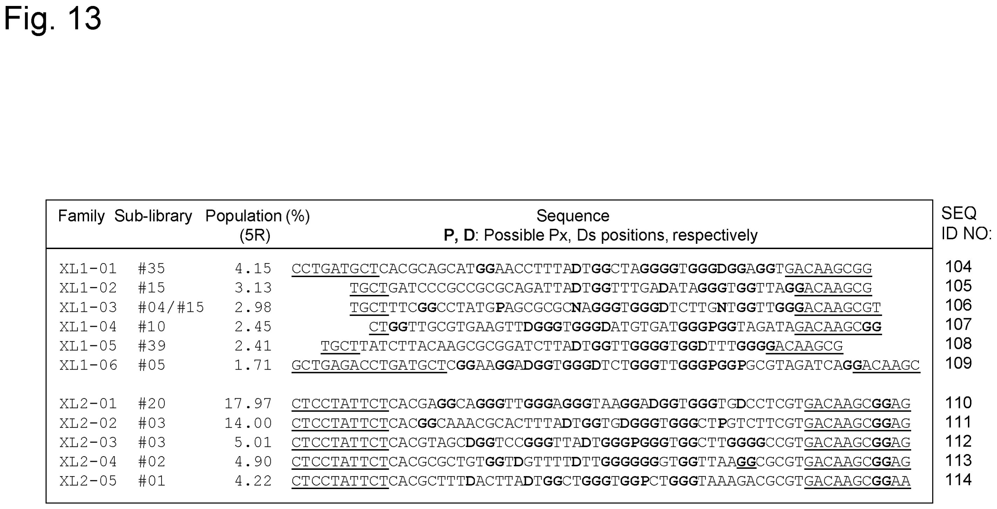

In accordance with the same method as in Example 3, six-letter ExSELEX was performed using, as a target protein, human (alpha-)Thrombin (Factor IIa) (HT1002a, Enzyme Research Lab) isolated from human blood plasma, and using the XL1 library and the XL2 library. The enriched library after the fifth selection was sequenced using the Ion PGM system. The results obtained by analyzing the higher-level families larger in the number of reads are shown in Figure 13. Any of the sequences comprised four or more G tracts, suggesting the formation of a guanine-quadruplex. In addition, a TTADsTGG sequence and an AAGGADsGGTGGG sequence were found out in the form of a consensus sequence in the sequence obtained from the XL1 and XL2 libraries. On the basis of the sequences obtained, the nucleic acid aptamers shown in Table 6 below were chemically synthesized.

**[Table 6]**

| Table 6: Thrombin-targeting nucleic acid aptamers | | |
|---|---|---|
| Name of nucleic acid aptamer | Base sequence of nucleic acid aptamer (5'-3') | SEQ ID NO: |
| Thr101 | GCTGTCAC GCAGCATGGAACCTTTADsTGGCTAGGGGTGGGDsGGAG GTGACAGC | 31 |
| Thr102 | GCTGTCC CGCCGCGCAGATTADsTGGTTTGADsATAGGGTGGTTA GGACAGC | 153 |
| Thr104 | GCGTTG CGTGAAGTTDsGGGTGGGDsATGTGATGGGPa'GGTAGATAGA CAACGC | 33 |
| Thr106 | GCTGATCT CGGAAGGADsGGTGGGDsTCTGGGTTGGGPa'GGPa'GCGT AGATCAGC | 35 |
| Thr201 | GCACGAGG CA GGGTTGGGAGGGTAAGGADsGGTGGGTGDs CCTCGTGC | 37 |
| Thr202 | GTCCGTTCACG GCAAACGCACTTTADsTGGTGDsGGGTGGCTPa'GTCTT CGTGAACGGAC | 154 |
| Thr203 | GTCCTTTCACG TAGCDsGGTCCGGGTTADsTGGGPa'GGGTGGCTTGGGGC CGTGAAAGGAC | 155 |
| Thr204 | GTCACGCGC TGTGGTDsGTTTTDsTTGGGGGGTGGTTAAG GCGCGTGAC | 27 |
| Thr205 | GCACGCTTT DsACTTADsTGGCTGGGTGGPa'CTGGGT AAAGCGTGC | 29 |

| | | |
|---|---|---|
| * The underlines denote sequences complementary to each other, and capable of forming a stem structure; "Ds" in the sequence represents a Ds base: and "Pa'" represents a Pa' base. | | |

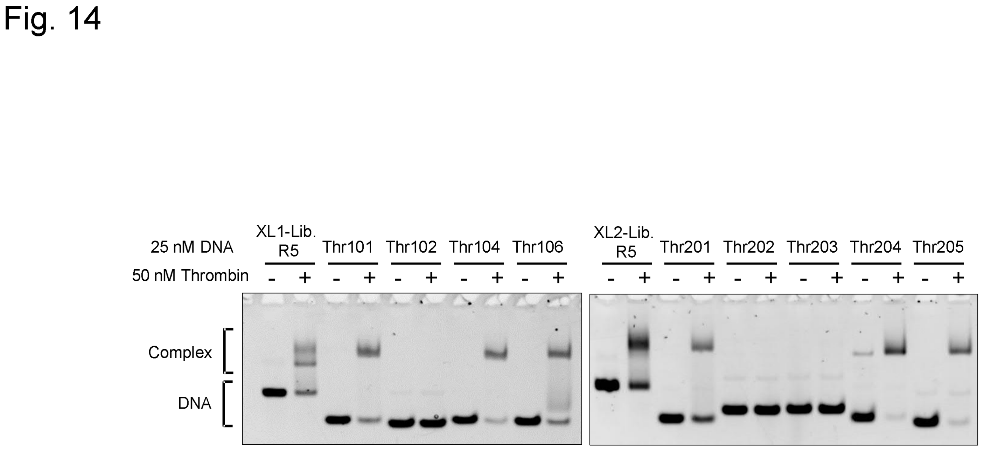

The results obtained by analyzing the affinity of the Thrombin-targeting nucleic acid aptamers shown in Table 6 above to Thrombin, using EMSA, are shown in Figure 14. Thr104, Thr204, and Thr205 exhibited high affinity to Thrombin.

Next, to the 3' end side of Thr104, Thr204, and Thr205, a biotinylated mini-hairpin sequence was linked to produce the aptamers shown in Table 7 below. BioMH-RE31 in the following Table is derived from the DNA aptamer (RE31) described in the literature (Russo, K.I. et al., Nucleic Acids Res., 2016, 44: 983-991).

**[Table 7]**

| Table 7: Thrombin-targeting nucleic acid aptamers with biotinylated mini-hairpin sequence linked thereto | | |
|---|---|---|
| Name of nucleic acid aptamer | Base sequence of nucleic acid aptamer (5'-3') | SEQ ID NO: |
| Bio-Thr104b | GCGT GCGTGAAGTTDsGGGTGGGDsATGTGATGGGPa'GGTAGA TACGCAACGC CGCGLAGCG | 156 |
| Bio-Thr204 | GTCACGCGC TGTGGTDsGTTTTDsTTGGGGGGTGGTTAAG GCGCGTGAC CGCGLAGCG | 157 |
| Bio-Thr205 | GCACGCTTT DsACTTADsTGGCTGGGTGGPa'CTGGGT AAAGCGTGC CGCGLAGCG | 158 |
| BioMH-RE31 | GTGACG TAGGTTGGTGTGGTTGGGG CGTCAC CGCGLAGCG | 159 |

| | | |
|---|---|---|
| * The underlines denote sequences complementary to each other, and capable of forming a stem structure; the black frame denotes a hairpin sequence; and "L" in the sequence represents Biotin-dT. | | |

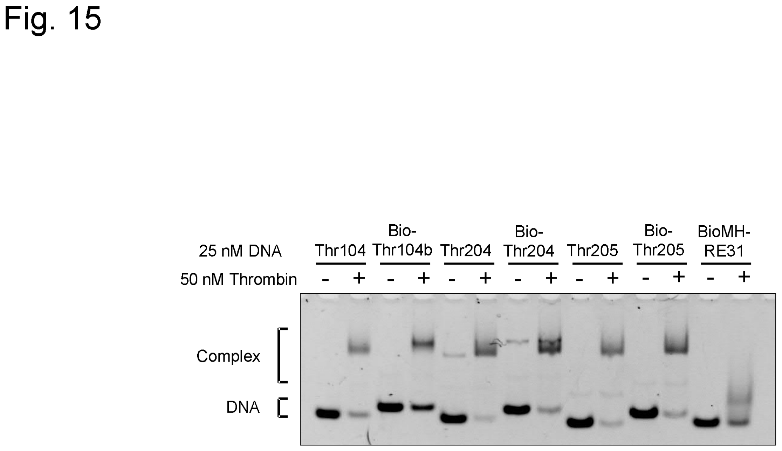

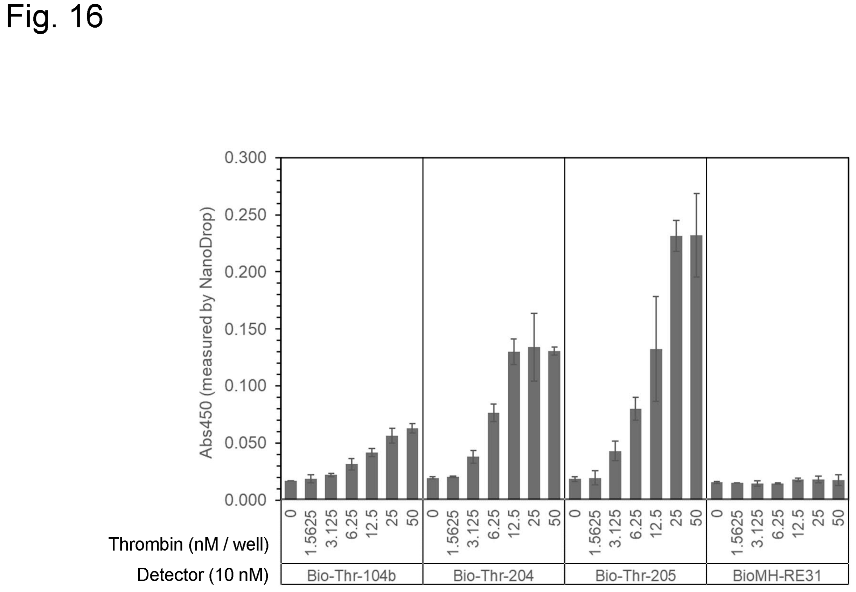

The affinity of the Thrombin-targeting nucleic acid aptamer shown in Table 7 above to Thrombin was analyzed using EMSA, ELONA, and the SPR method. The results are shown in Figure 15 and Figure 16. In EMSA and ELONA investigations, Bio-Thr104b, Bio-Thr204, and Bio-Thr205 exhibited markedly higher affinity, compared with BioMH-RE31. The *K*_{D} value measured using the SPR method was 9.0 pM for Bio-Thr204 and 7.3 pM for Bio-Thr205.

### (3) IL-6-targeting nucleic acid aptamer

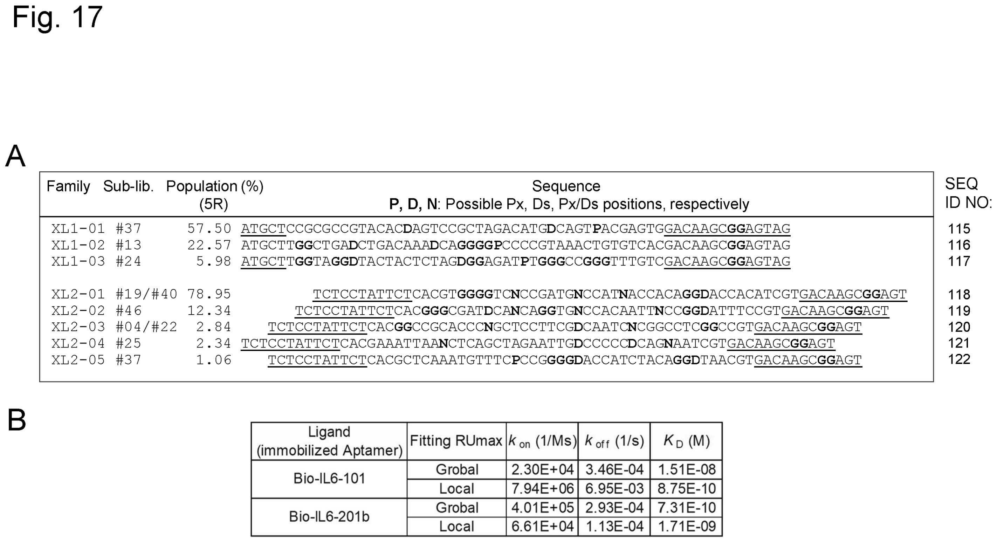

In accordance with the same method as in Example 3, six-letter ExSELEX was performed using, as a target protein, IL-6 (7270-IL, R&D Systems) expressed in a HEK293 cell, and using the XL1 library and the XL2 library. However, no urea solution was used in washing. The concentrated library after the fifth selection was sequenced using the Ion PGM system. The results obtained by analyzing the higher-level families larger in the number of reads are shown in Figure 17A. On the basis of the sequences obtained (specifically XL1-01 and XL2-01 in Figure 17A), the nucleic acid aptamers shown in Table 8 below were chemically synthesized.

**[Table 8]**

| Table 8: IL-6-targeting nucleic acid aptamers with biotinylated mini-hairpin sequence linked thereto | | |
|---|---|---|
| Name of nucleic acid aptamer | Base sequence of nucleic acid aptamer (5'-3') | SEQ ID NO: |
| Bio-IL6-101 | GCTCCGC GCC GT A CAC DsA GT CCG CT AGACATGDsC AG TPa' AC GA GTG G AC AA GCGGAGC CGCGLAGCG | 160 |
| Bio-IL6-201b | GCACGT GG GGT CPa'CCGATGPa'CCATDsACCACAGGDs ACCACGTGC CGCGLAGCG | 161 |
| Bio-IL6-201c | GCGGTC Pa'C CGATG Pa'CCATDsACCACAGGDsACCA CATCG T GACCGC CGCGLAGCG | 162 |
| Bio-IL6-201d | GTCCGATG Pa'CCATDsACCACAGGDsACCA CATCGGAC CGCGLAGCG | 163 |

| | | |
|---|---|---|
| * The underlines denote sequences complementary to each other, and capable of forming a stem structure; the black frame denotes a hairpin sequence; and "L" in the sequence represents Biotin-dT. | | |

The affinity of the IL-6-targeting nucleic acid aptamers shown in Table 8 above to IL-6 was analyzed using SPR. The results are shown in Figure 17B. Bio-IL6-101 exhibited relatively weak binding on nM levels, but on the other hand, Bio-IL6-201b exhibited relatively high affinity on sub-nM levels.

### (4) IL-8-targeting nucleic acid aptamer

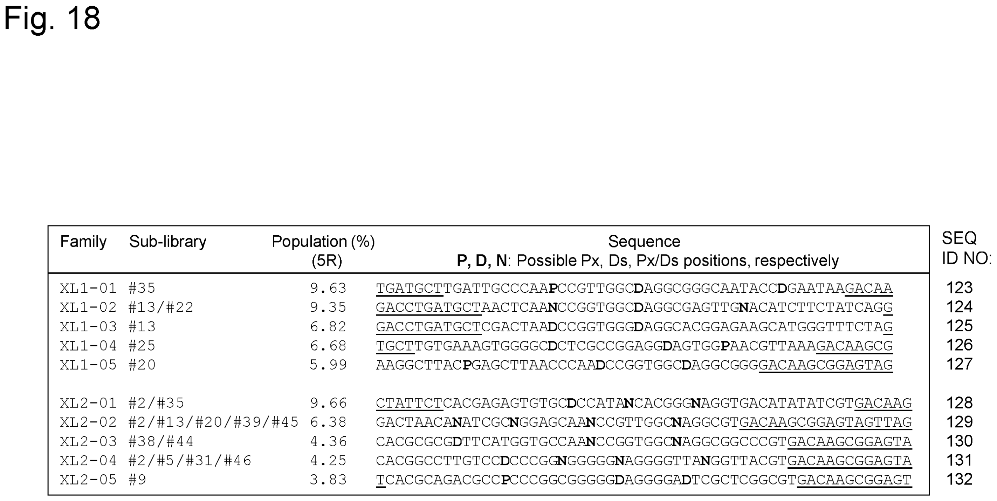

In accordance with the same method as in Example 3, six-letter ExSELEX was performed using, as a target protein, human IL-8 (10098-HNCH1, Sino Biological Inc.) expressed in a HEK293 cell, and using the XL1 library and the XL2 library. However, no urea solution was used in washing. The concentrated library after the fifth selection was sequenced using the Ion PGM system. The results obtained by analyzing the higher-level families larger in the number of reads are shown in Figure 18. An AANCCG(T/G)TGG(C/G)DsAGGC (N = Ds or Pa') sequence was found out in the form of a consensus sequence in the sequence obtained from the XL1 and XL2 libraries. On the basis of the sequences obtained, the nucleic acid aptamers shown in Table 9 below were chemically synthesized.

**[Table 9]**

| Table 9: IL-8-targeting nucleic acid aptamers | | |
|---|---|---|
| Name of nucleic acid aptamer | Base sequence of nucleic acid aptamer (5'-3') | SEQ ID NO: |
| IL8-102DD | GGAGATG CT AACTC AADs CCGGTGGCDsAGGC GAGTT GAA CATCTCC | 43 |
| IL8-102PD | GGAGATG CT AACTC AAPa'CCGGTGGCDsAGGC GAGTT GAA CATCTCC | 43 |
| IL8-223DD | GACTCCGCTTG A AC AADs CCGGTGGGDsAGGC GT GA CAAGCGGAGTC | 45 |
| IL8-223PD | GACTCCGCTTG A AC AAPa'CCGGTGGGDsAGGC GT GA CAAGCGGAGTC | 45 |
| IL8-201DDP | GTTCACGA GAG TGT GCDsCCATADs CACGGGPa'AGGTG ACA TATA TCGTGAAC | 164 |
| IL8-201DPD | GTTCACGA GAG TGT GCDsCCATAPa'CACGGGDs AGGTG ACA TATA TCGTGAAC | 49 |

| | | |
|---|---|---|
| * The underlines denote sequences complementary to each other, and capable of forming a stem structure. | | |

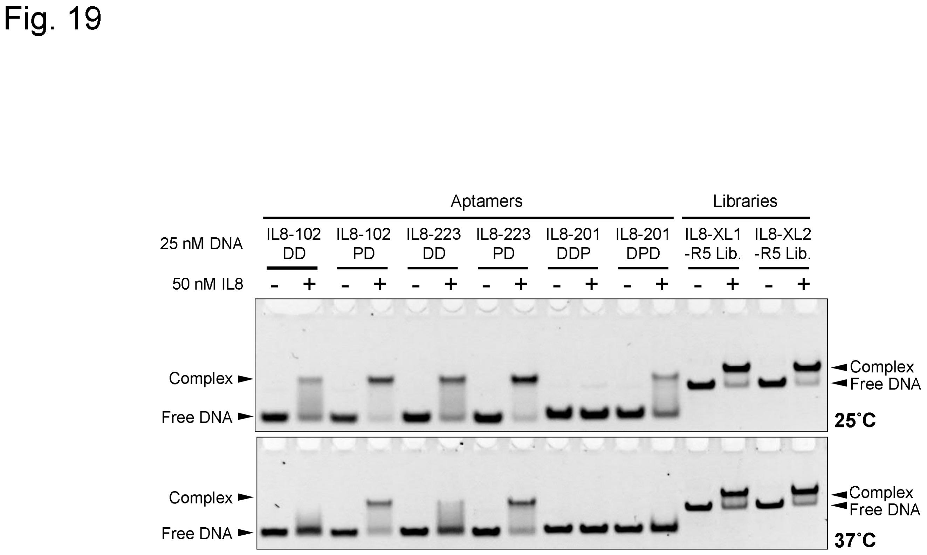

The results obtained by analyzing the affinity of the IL-8-targeting nucleic acid aptamers shown in Table 9 above to IL-8, using EMSA, are shown in Figure 19. IL8-102PD and IL8-223PD exhibited the highest affinity to IL-8.

Next, to the 3' end side, a biotinylated mini-hairpin sequence was linked to produce the aptamers shown in Table 10 below.

**[Table 10]**

| Table 10: IL-8-targeting nucleic acid aptamers with biotinylated mini-hairpin sequence linked thereto | | |
|---|---|---|
| Name of nucleic acid aptamer | Base sequence of nucleic acid aptamer (5'-3') | SEQ ID NO: |
| IL8-102PD | GGAGATG CT AACTC AAPa'CCGGTGGCDsAGGC GAGTT GA A CATCTCC | 43 |
| Bio-IL8-102PD | GGAGATG CT AACTC AAPa'CCGGTGGCDsAGGC GAGTT GA A CATCTCC CGCGLAGCG | 166 |
| Bio-IL8-102PDb | GGAGATG CT AACTC AAPa'CCGGTGGCDsAGGC GAGTT GDsA CATCTCC CGCGLAGCG | 167 |
| IL8-223PD | GACTCCGCTTG A AC AAPa'CCGGTGGGDsAGGC GT GA CAAGCGGAGTC | 168 |
| Bio-IL8-223PD | GACTCCGCTTG A AC AAPa'CCGGTGGGDsAGGC GT GA CAAGCGGAGT CCGCGLAGCG | 169 |
| Bio-IL8-223PDb | GACTCCGCTTGAC AAPa'CCGGTGGGDsAGGC GTCAAGCGGAGT CCGCGLAGCG | 170 |

| | | |
|---|---|---|
| * The underlines denote sequences complementary to each other, and capable of forming a stem structure; the black frame denotes a hairpin sequence; and "L" in the sequence represents Biotin-dT. | | |

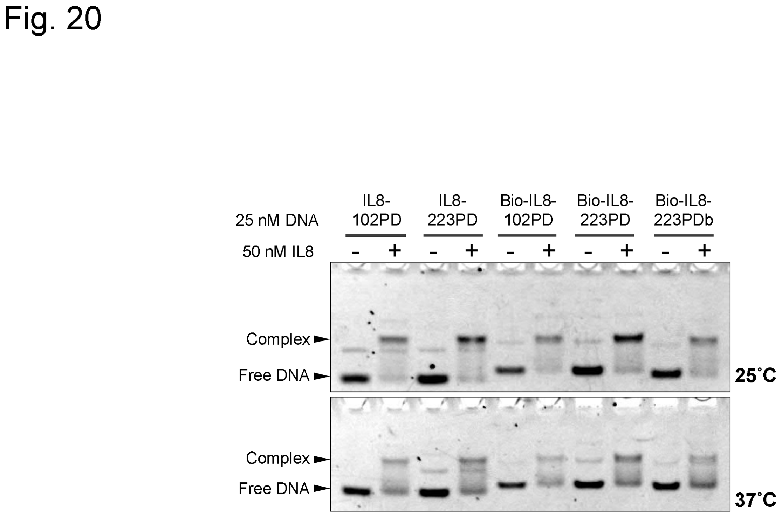

The affinity of the IL-8-targeting nucleic acid aptamers shown in Table 10 above to IL-8 was analyzed using EMSA and SPR. The results are shown in Figure 19 and Figure 20. With the aptamer having a bulge structure added to the end region thereof, the affinity rose. Also with the aptamer having Ds added to the bulge loop thereof, the affinity rose. SPR resulted as follows: for Bio-IL8-102PD, *K*_{D} = 163 pM; for Bio-IL8-102PDb, *K*_{D} = 144 pM; for Bio-IL8-223PD, *K*_{D} = 202 pM; and for Bio-IL8-223PDb, *K*_{D} = 640 pM. The affinity of Bio-IL8-102PDb was the highest.

### (5) HMGB1-targeting nucleic acid aptamer

In accordance with the same method as in (1) to (4) above, six-letter ExSELEX was performed using HMGB 1 protein as a target protein. From the results obtained by analyzing the sequences of the higher-level families larger in the number of reads, a Bio-HMGB1-301-DD aptamer (SEQ ID NO: 143) shown in the following Table 11 was designed and chemically synthesized.

**[Table 11]**

| Table 11: HMGB1-targeting nucleic acid aptamers | | |
|---|---|---|
| Name of nucleic acid aptamer | Base sequence of nucleic acid aptamer (5'-3') | SEQ ID NO: |
| Bio-HMGB1-301-DD | CTCCTTCAGC GCCACCGTATGDsACGCTCACAGDsAGTTGCCGA GCTGAAGGAG CGCGLAGCG | 171 |
| Bio-HMGB1-301-AA | CTCCTTCAGC GCCACCGTATGA ACGCTCACAGA AGTTGCCGA GCTGAAGGAG CGCGLAGCG | 172 |

| | | |
|---|---|---|
| * The underlines denote sequences complementary to each other, and capable of forming a stem structure; the black frame denotes a hairpin sequence; "L" in the sequence represents Biotin-dT; and "Ds" represents a Ds base. | | |

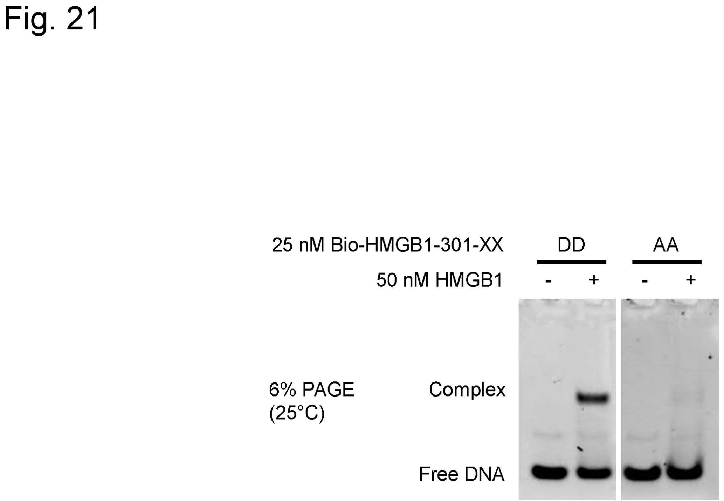

The results obtained by analyzing the affinity of the nucleic acid aptamers shown in Table 11 above to human HMGB1 protein, using EMSA, are shown in Figure 21. The Bio-HMGB1-301-DD aptamer exhibited binding to HMGB1, but Bio-HMGB1-301-AA having natural adenine bases substituted for the Ds bases was markedly decreased in the binding activity. Next the *K*_{D} value was determined using SPR. The *K*_{D} value of Bio-HMGB1-301-DD was 23.4 pM.

### <Example 5: Further human IFN-γ-targeting nucleic acid aptamer>

### (Purpose)

A further nucleic acid aptamer that binds to both of glycosylated IFN-γ and non-glycosylated IFN-γ was created.

### (Method and results)

### (1) Six-letter ExSELEX

To obtain a further nucleic acid aptamer that binds to both of glycosylated IFN-γ and non-glycosylated IFN-γ, both of glycosylated IFN-γ and non-glycosylated IFN-γ were used as target proteins. Specifically, for an object that is the XL1 library described in "(1) Six-letter ExSELEX intended for XL1 library" in Example 3, glycosylated IFN-γ (commercially available glycosylated human IFN-γ (IFG-H4211, ACROBiosystems) expressed in a HEK293 cell) was used as a target protein in the first, second, fourth, and sixth rounds of selection. In the third and fifth rounds of selection, non-glycosylated IFN-γ (IFN-γ (300-02, Peprotech) expressed in *Escherichia coli*) was used. As the results obtained by analyzing of the sequences of the libraries obtained after the sixth selection, IFN-g-701 and IFN-g-703 shown in Table 12 below were identified as novel sequences. IFN-g-701 was found to contain two GTCGCTGGDsC base sequences in the variable region.

**[Table 12]**

| Table 12: IFNg-701 and IFNg-703 | | |
|---|---|---|
| Name of sequence | Base sequence (5'-3') | SEQ ID NO: |
| IFNg-701 | CTGATGCT CCTAT GTCGCTGGDsC TGCAPxA GTCGCTGGDsC AAGGGATTAGG GACAAGCG | 175 |
| IFNg-703 | CTGATGCT CGTGCGPxGTACGCGGCGTGGDsTGGTGCTGGNTGGGNCAGGCG GACAAGCG | 176 |

| | | |
|---|---|---|
| * The underlined capital letters represent part of the primer region; and "N" represents a Ds base or a Px base. | | |

### (2) Isolation of clone and synthesis of nucleic acid aptamer

Using a probe comprising the complementary sequences of the base sequences of the above-described IFN-g-701 and IFN-g-703, clones of interest were isolated from the libraries after the sixth selection (each library is hereinafter referred to as a "R6-library"), and subjected to PCR amplification (the amplification products are hereinafter referred to as a "701 isolate" and a "703 isolate" respectively). In addition, the base sequence of this amplification product was determined, and a nucleic acid aptamer was synthesized on the basis of the sequence obtained. The names of the amplification products and nucleic acid aptamers and the base sequences are shown in Table 13 below.

**[Table 13]**

| Table 13: Sequences of amplification products and nucleic acid aptamers | | |
|---|---|---|
| Name of sequence | Base sequence (5'-3') | SEQ ID NO: |
| 701 isolate | (Primer) CCTAT GTCGCTGGDsC TGCAPxA GTCGCTGGDsC AAGGGATTAGGACAAGCG (Primer) | 177 |
| B-IFNg-701a | GGCTCCTAT GTCGCTGGDsC TGCAPa'A GTCGCTGGDsC AAGGGATTAGGAGCC CGCGLAGCG | 178 |
| B-IFNg-701b | GGCTCCTAT GTCGCTGGDsC TGCATA GTCGCTGGDsC AAGGGATTAGGAGCC CGCGLAGCG | 179 |
| 703 isolate | (Primer) CGTGCGPxGTACGCGGCGTGGNTGGTGCTGGPa'TGGGDCAGGC G (Primer) | 180 |
| B-IFNg-703a | TGCTCCGCGPa'GTACGCGGCGTGGDsTGGTGCTGGPa'TGGGD sCAGGCGGACAAGCGGAGCA CGCGLAGCG | 181 |
| B-IFNg-703b | TGCTCCGCGPa'GTACGCGGCGTGGPa'TGGTGCTGGPa'TGGG DsCAGGCGGACAAGCGGAGCA CGCGLAGCG | 182 |
| B-IFNg-703c | TGCTCCGCGTGTACGCGGCGTGGDsTGGTGCTGGPa'TGGGDsC AGGCGGACAAGCGGAGCA CGCGLAGCG | 183 |
| B-IFNg-703d | TGCTCCGCGTGTACGCGGCGTGGPa'TGGTGCTGGPa'TGGGDs CAGGCGGACAAGCGGAGCA CGCGLAGCG | 184 |

| | | |
|---|---|---|
| * The black frame denotes a hairpin sequence; "L" in the sequence represents Biotin-dT; "N" represents a Ds base or a Px base; and "(Primer)" represents a binding sequence of a primer used for PCR amplification. | | |

### (3) Evaluation of affinity to glycosylated IFN-γ and non-glycosylated IFN-γ

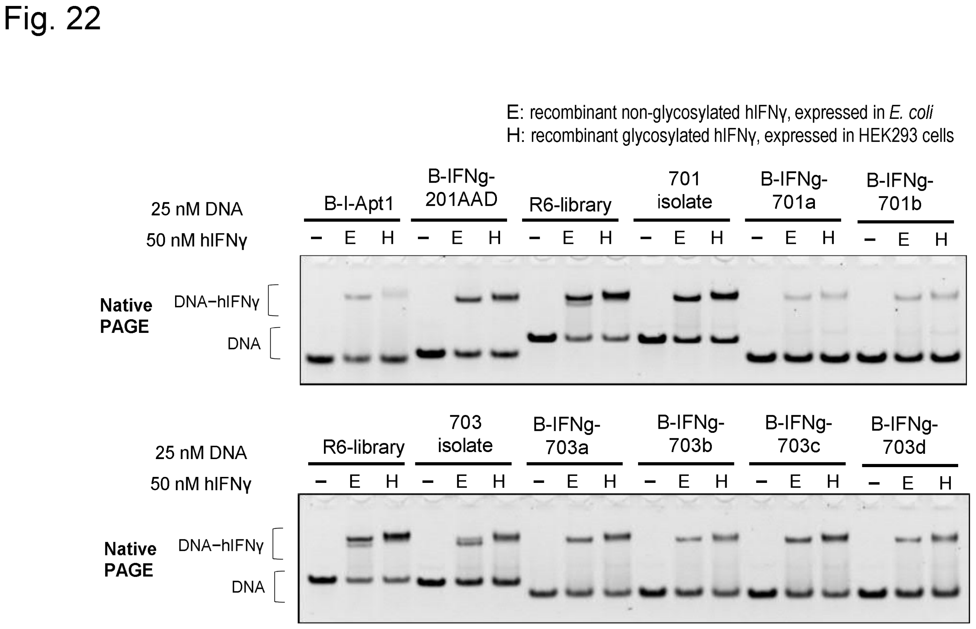

For the nucleic acids and R6-libraries described in Table 13 above and the above-described B-I-Apt1 and B-IFN-g-201AAD, the affinity to glycosylated IFN-γ and non-glycosylated IFN-γ was analyzed using EMSA based on the same method as in (c) in Example 3. The results are shown in Figure 22. It has been revealed that, to both glycosylated IFN-γ and non-glycosylated IFN-γ, the nucleic acids described in Table 13 exhibited a binding capacity equal to or stronger than the binding capacity that B-I-Apt1 and B-IFN-g-201AAD exhibited.

### (4) Further generation of nucleic acid aptamer by six-letter ExSELEX

According to the results shown in Figure 22, the R6-library and 701 isolate exhibited strong binding, but the fragments (B-IFN-g-701a/b) resultant from shortening the sequence of the 701 isolate were decreased in the binding capacity. On the basis of this result, the base sequence of the 701 isolate was optimized below.

Specifically, the below-described XL1 (a) library comprising the above-described conserved sequence GTCGCTGGDsC as a common motif was produced. The XL1 (a) library comprises, between the primer-binding sequences (5'-GCTCATTCGCCTCTCCTATTCT-3' and 5'-GACAAGCGGAGTAGTTAGACCGTC-3') at the 5' end and 3' end, the following sequence: NNNNNNNNNGTCGCTGGDCTGNAPAGTCGCTGGDCNNNNNNNNNNNNNNN (SEQ ID NO: 185; N represents a base selected randomly from the A, T, G, and C bases; P represents the Pa' base; and D represents the Ds base). Using this XL1 (a) library as a subject, six-letter ExSELEX comprising five rounds of selection was performed. As a target protein, glycosylated IFN-γ was used for the first to fourth rounds of selection, and non-glycosylated IFN-γ was used for the fifth selection. The base sequences of the libraries after the fifth selection were analyzed. On the basis of the results obtained by analyzing 100 kinds of higher-level sequences larger in the number of reads, nucleic acid aptamers shown in Table 14 below were synthesized.

**[Table 14]**

| Table 14: Sequences of nucleic acid aptamers | | |
|---|---|---|
| Name of sequence | Base sequence (5'-3') | SEQ ID NO: |
| B-IFNg701-1 | CTTGTCAA GGT GTCGCTCGDsC TGCAPa'A GTCGCTGGDsC AGAACTTAGCCA TTGACAAG CGCGLAGCG | 186 |
| B-IFNg701-2 | CTTGTCA GGT GTCGCTGGDsC TGTAPa'A GTCGCTGGDsC AGTACCTTTGGCCA TGACAAG CGCGLAGCG | 187 |
| B-IFNg701-3 | GGCTTGTC GT GTCGCTGGDsC TGAAPa'A GTCGCTGGDsC AAAAAGCTTTTTCGA GACAAGCC CGCGLAGCG | 188 |
| B-IFNg701-4 | CTGCTTGT GGT GTCGCTGGDsC TGCAPa'A GTCGCTGGDsC ATGAAACCTTTGGCCG ACAAGCAG CGCGLAGCG | 189 |
| B-IFNg701-9 | CTTGTCAA GGA GTCGCTGGDsC TGTAPa'A GTCGCTGGDsC TGTAGTTTACCCA TTGACAAG CGCGLAGCG | 190 |
| IFNg701-1 (DPD) | CTTGTCAA GGT GTCGCTGGDsC TGGAPa'A GTCGCTGGDsC AGAACTTTAGCCA TTGACAAG | 191 |
| IFNg701-1a (APD) | CTTGTCAA GGT GTCGCTGGAC TGGAPa'A GTCGCTGGDsC AGAACTTTAGCCA TTGACAAG | 192 |
| IFNg701-1b (DTD) | CTTGTCAA GGT CTCCCTGGDsC TGGATA CTCCCTCCDsC AGAACTTTAGCCA TTGACAAG | 193 |
| IFNg701-1c (DPA) | CTTGTCAA GGT GTCGCTGGDsC TGGAPa'A GTCGCTGGAC AGAACTTTAGCCA TTGACAAG | 194 |
| IFNg701-1d (ATA) | CTTGTCAA GGT GTCGCTGGAC TGGATA GTCGCTGGAC AGAACTTTAGCCA TTGACAAG | 195 |

| | | |
|---|---|---|
| * The underlines denote sequences complementary to each other, and capable of forming a stem structure; the black frame denotes a hairpin sequence; and "L" in the sequence represents Biotin-dT. | | |

### (5) Evaluation of affinity to glycosylated IFN-γ and non-glycosylated IFN-γ

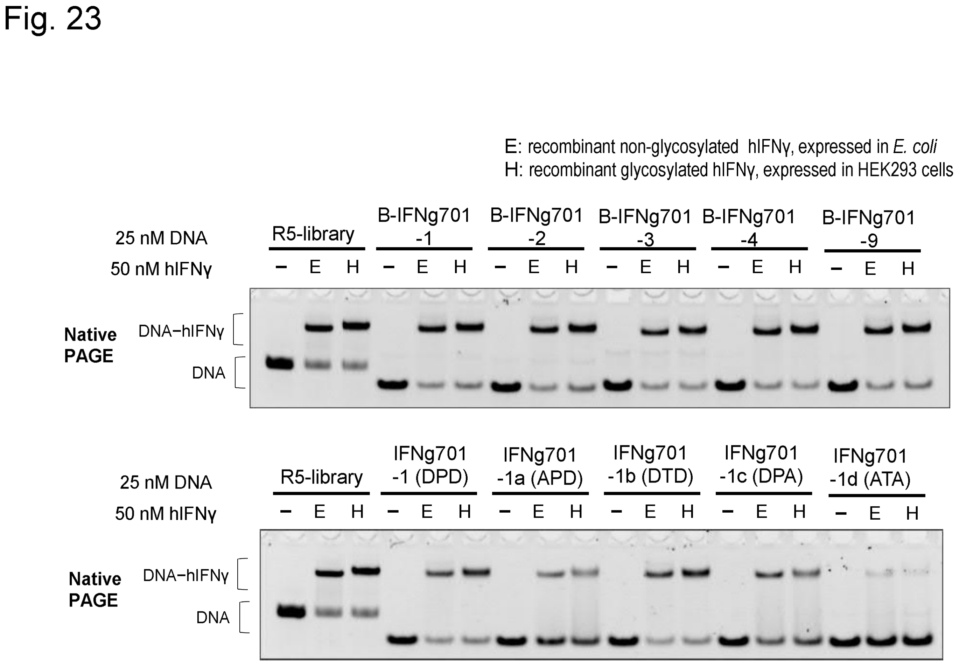

Using the same EMSA as in (3) above, the affinity of the nucleic acid aptamer described in Table 14 above to glycosylated IFN-γ and non-glycosylated IFN-γ was analyzed. The results are shown in Figure 23. The results shown in Figure 23 have revealed that all the nucleic acid aptamers except IF701-1a (ATA) bind strongly to both non-glycosylated IFN-γ and glycosylated IFN-γ. On the other hand, the result that IF701-1a(ATA) resulting from substituting all the unnatural bases with natural bases was decreased in the binding capacity has revealed that the binding capacity depends on the unnatural base.

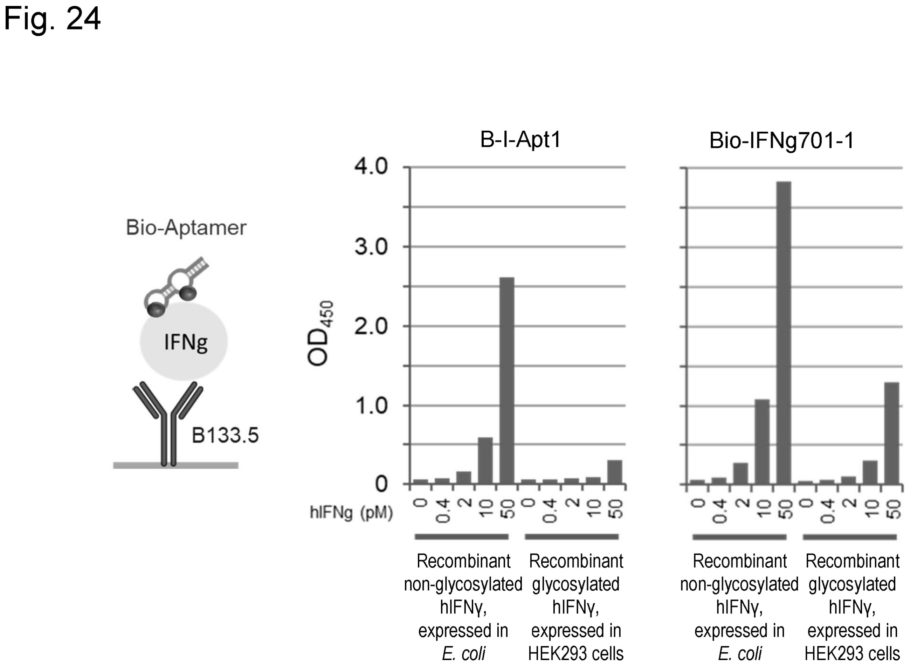

Next, the detection sensitivity of each of the B-I-Apt1 and B-IF701-1 nucleic acid aptamers to IFN-γ was studied using ELISA. For ELISA, a commercially available monoclonal antibody B133.5 (Thermo Fisher Scientific Inc.) was used. The B133.5 antibody was used as an antibody for capturing IFN-γ, and the nucleic acid aptamer at 1 nM was used for detection. The results of detection by streptavidin-bound HRP are shown in Figure 24. It has been revealed that B-IF701-1 had higher detection sensitivity to non-glycosylated IFN-γ and glycosylated IFN-γ than B-I-Apt1.

### (6) Generation of further nucleic acid aptamer

A library obtained by randomizing part of the base sequence of the above-described IFN-g-701-1 was produced, and six-letter ExSELEX was performed to generate a further nucleic acid aptamer. Specifically, an XL1 (b) library comprising, between the primer-binding sequences (5'-TGATTAGCTGAGACCTGATGCT-3' and 5'-GGCGGGACAAGCGGAGTAGTTAGACCGTC-3') at the 5' end and 3' end, the following sequence: GTCCCGCCTGTCGCTGGDCTG-N₃₋AGTCGCTGGDCA-Nₙ (N represents a base selected randomly from the A, T, G, C, and Pa' bases at a ratio of 23:23:23:23:8; D represents the Ds base; and n represents 8, 9, or 10; SEQ ID NOs: 196 to 198) was produced. Using this XL1 (b) library as a subject, six-letter ExSELEX comprising five rounds of selection was performed. As a target protein, glycosylated IFN-γ was used for the first to third rounds of selection, and non-glycosylated IFN-γ was used for the fourth to fifth rounds of selection. In addition, in the third selection, the IF701-1 nucleic acid aptamer was added in an amount 5 times as large as the concentration of the library during the formation of a complex. In the fourth to fifth rounds of selection, the B-I-Apt1 nucleic acid aptamer was added in an amount 5 times as large as the concentration of the library, so that each aptamer was made to compete in the binding to IFN-γ. The base sequences of the libraries after the fifth selection were analyzed. On the basis of the results obtained by analyzing 100 kinds of higher-level sequences larger in the number of reads, nucleic acid aptamers shown in Table 15 below were synthesized.

**[Table 15]**

| Table 15: Sequences of nucleic acid aptamers | | |
|---|---|---|
| Name of sequence | Base sequence (5'-3') | SEQ ID NO: |
| B-IFNg701-21 | GGGCCCG CCTG TCGCTGGDsCTGAAPa'AGTCGCTGGDs CAGG TTTCCTTCGG CGGGCCC CGCGLAGCG | 199 |
| B-IFNg701-22 | GGGCCCG CCTG TCGCTGGDsCTGPa'APa'AGTCGCTGGDs CAGGTTTCCTTCGG CGGGCCC CGCGLAGCG | 200 |
| B-IFNg701-23 | GGGCCCG CCTG TCGCTGGDsCTGAAPa'AGTCGCTGGDs CAGG TPa'TCCTTCGG CGGGCCC CGCGLAGCG | 201 |
| B-IFNg701-24 | GGGCCCG CCTG TCGCTGGDsCTGAAPa'AGTCGCTGGDs CAGGTTTCCPa'TCGG CGGGCCC CGCGLAGCG | 202 |
| B-IFNg701-25 | GGGCCCG CCTG TCGCTGGDsCTGAAPa'AGTCGCTGGDs CAGGTPa'TCCPa'TCGG CGGGCCC CGCGLAGCG | 203 |
| B-IFNg701-26 | GGGCCCG CCTG TCGCTGGDsCTGPa'APa'AGTCGCTGGDs CAGGTPa'TCCPa'TCGG CGGGCCC CGCGLAGCG | 204 |
| B-IFNg701-27 | GGGCCCG CCTG TCGCTGGDsCTGAAPa'AGTCGCTGGDs CAGGTTTCCTTC CGGGCCC CGCGLAGCG | 205 |
| B-IFNg701-31 | GCCCGTCCCG CCT GTCGCTGGDsC TGAAPa'A GTCGCTGGDsC AG CGGGACGGC CGCGLAGCG | 206 |
| B-IFNg701-32 | GGCGGTCCCG CTGT CGCTGGDsCTGAAPa'AGTCGCTGGDs CA CGGGACGGCC CGCGLAGCG | 207 |
| B-IFNg701-33 | GGCGGTCCCG CTGT CGCTGGDsCTGPa'APa'AGTCGCTGGDs CA CGGGACGGCC CGCGLAGCG | 208 |
| B-IFNg701-42 | GGCCCGCCTG TCGCTGGDsCTGAAPa'AGTCGCTGGDs CAGGCGGGCC CGCGLAGCG | 209 |

| | | |
|---|---|---|
| * The black frame denotes a hairpin sequence; and "L" in the sequence represents Biotin-dT. | | |

### (7) Evaluation of affinity to glycosylated IFN-γ and non-glycosylated IFN-γ

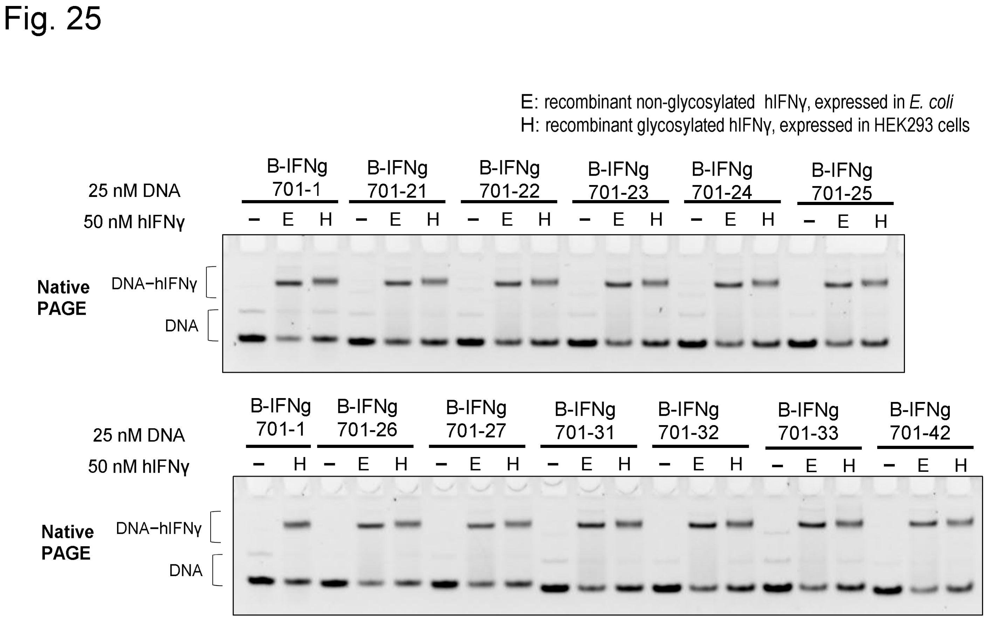

Using the same EMSA as in (3) above, the affinity of the nucleic acid aptamer described in Table 15 above to glycosylated IFN-γ and non-glycosylated IFN-γ was analyzed. The results are shown in Figure 25. The results shown in Figure 25 have revealed that all the nucleic acid aptamers bind strongly to both non-glycosylated IFN-γ and glycosylated IFN-γ.

Next, the binding of B-IF701-1 to a target protein was analyzed at 37°C by SPR, and as a result, the *K*_{d} value of the binding to non-glycosylated IFN-γ was 53.2 pM, and the *K*_{d} value of the binding to glycosylated IFN-γ was 87.5 pM.

### <Example 6: Investigation of stem region>

### (Purpose)

The base sequence of a stem region and the length of the sequence in a nucleic acid aptamer that binds to IFN-γ or Thrombin were studied.

### (Method and results)

### (1) Stem region of IFN-γ-targeting nucleic acid aptamer

A nucleic acid aptamer was chemically synthesized by substituting, with a different sequence, a stem region in the nucleic acid aptamer B-IFN-g701-1 that binds to IFN-γ. The nucleic acid aptamers produced in this Example are shown in Table 16 below.

**[Table 16]**

| Table 16: Nucleic acid aptamers comprising stem regions consisting of various sequences | | |
|---|---|---|
| Name of sequence | Base sequence (5'-3') | SEQ ID NO: |
| B-IFNg 701-1 | CTTGTCAAGG TGTCCCTGCDsCTGCAPa'ACTCGCTGCDsCACAACTTTAC CCATTGACAAG CGCGLAGCG | 186 |
| B-IFNg 701-1e | CCCGGCAAGG TGTCGCTGGDsCTGGAPa'AGTCGCTGGDsCAGAACTTTAG CCATTGCCGGG CGCGLAGCG | 236 |
| B-IFNg 701-1f | CCCGGCGCGG TGTCGCTGGDsCTGGAPa'AGTCGCTGGDsCAGAACTTTAG CCAGCGCCGGG CGCGLAGCG | 237 |
| B-IFNg 701-1g | CTTGTCAAGG TGTCGCTGGDsCTGGAT AGTCGCTGGDsCAGAACTTTAG CCATTGACAAG CGCGLAGCG | 238 |

| | | |
|---|---|---|
| * The underlines denote sequences complementary to each other, and capable of forming a stem structure; the black frame denotes a hairpin sequence; and "L" in the sequence represents Biotin-dT. | | |

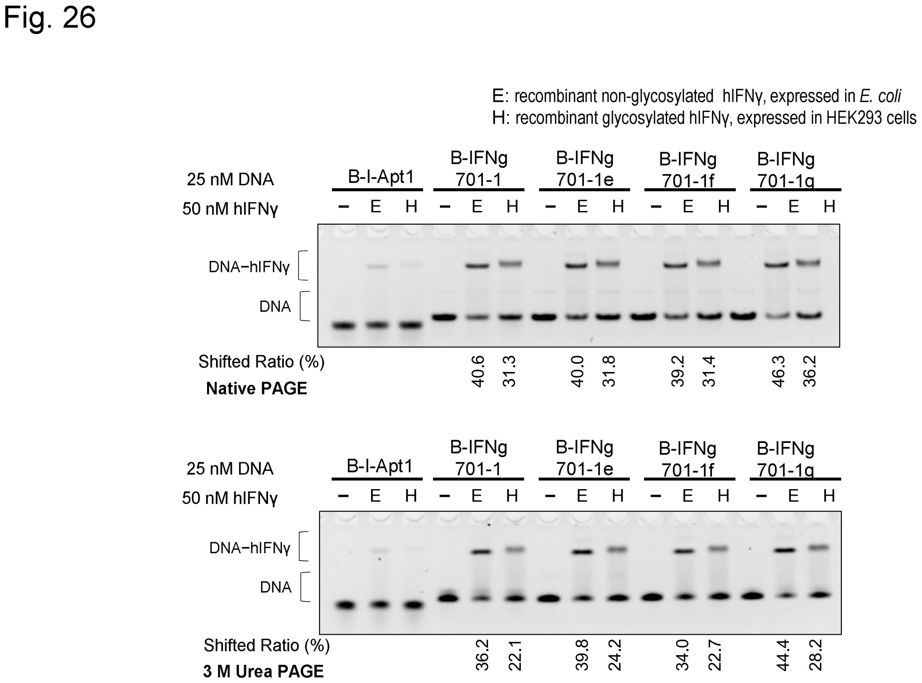

Using EMSA based on the same method as in (c) in Example 3, the affinity of the nucleic acid aptamer described in Table 16 above to IFN-γ was analyzed. The results are shown in Figure 26. It has been revealed that the nucleic acid aptamer obtained by substituting the stem region with a different sequence was not impaired in terms of the binding to glycosylated IFN-γ and non-glycosylated IFN-γ.

### (2) Stem region of Thrombin-targeting nucleic acid aptamer

A modified nucleic acid aptamer was chemically synthesized by shortening, to 7 bp or 5 bp, the 9 bp stem region in the nucleic acid aptamer Thr205-ADP that binds to Thrombin. The nucleic acid aptamers produced in this Example are shown in Table 17 below.

**[Table 17]**

| Table 17: Nucleic acid aptamers comprising stem regions having various lengths | | |
|---|---|---|
| Name of sequence | Base sequence (5'-3') | SEQ ID NO: |
| Thr205-ADP-43 | GCACGCTTT AACTTADsTGGCTGGGTGGPa'CTGGGT AAAGCGTGC | 210 |
| Thr205-ADP-39 | GCGCTTT AACTTA**Ds**TGGCTGGGTGG**Pa**'CTGGGT AAAGCGC | 211 |
| Thr205-ADP-35 | GCGTT AACTTA**Ds**TGGCTGGGTGG**Pa**'CTGGGT AACGC | 212 |

| | | |
|---|---|---|
| * The underlines denote sequences complementary to each other, and capable of forming a stem structure. | | |

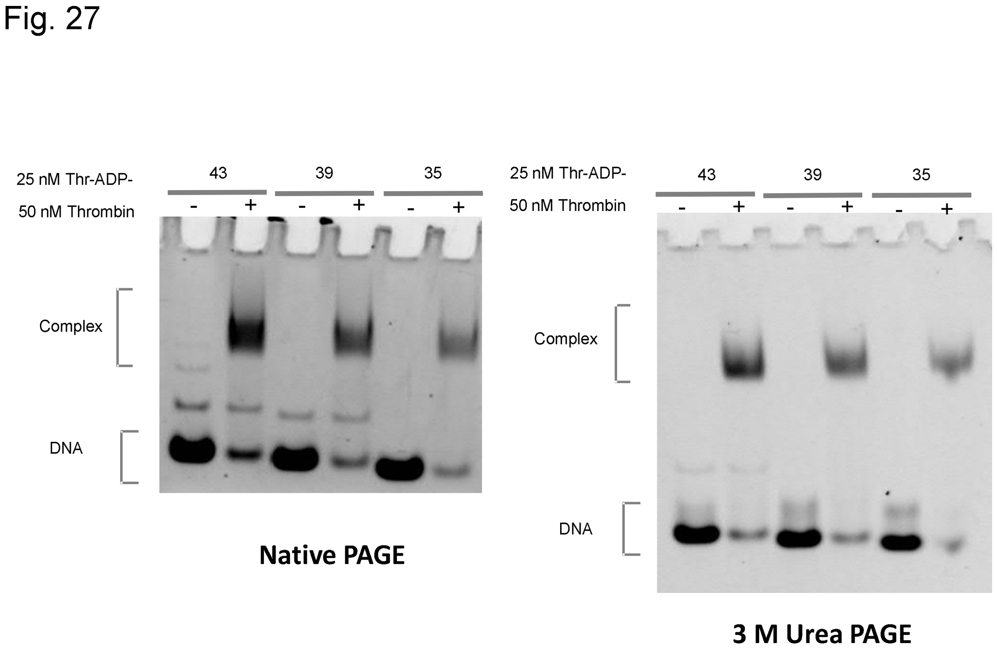

Using EMSA based on the same method as in (c) in Example 3, the affinity of the nucleic acid aptamer described in Table 17 above to Thrombin was analyzed. The results are shown in Figure 27. It has been revealed that also the nucleic acid aptamer obtained by shortening the nine-base-pair stem region to seven base pairs or five base pairs was not impaired in terms of the binding to Thrombin.

All publications, patents, and patent applications cited herein are incorporated herein by reference in their entirety.

## Claims

1. A single-stranded nucleic acid molecule library comprising a plurality of single-stranded nucleic acid molecules, wherein:
the plurality of single-stranded nucleic acid molecules comprise a Ds/Pa'-containing single-stranded nucleic acid molecule,
the Ds/Pa'-containing single-stranded nucleic acid molecule comprises
a pair of primer-binding regions at the 5' end and 3' end thereof, and
a variable region between the pair of primer-binding regions,
the pair of primer-binding regions consist of base sequences which are common among the plurality of single-stranded nucleic acid molecules, and
the variable region comprises
an unnatural base Ds (7-(2-thienyl)-3H-imidazo[4,5-b]pyridine-3-yl) shown in the following general formula (I) and
an unnatural base Pa' shown in the following general formula (II): 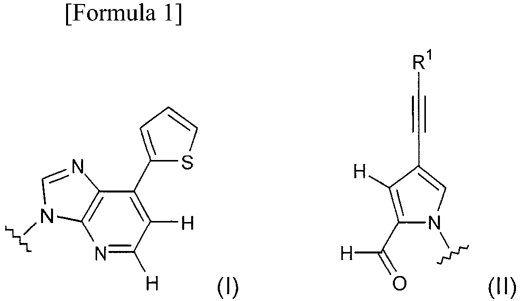
wherein R¹ respectively and independently represents a hydrogen atom or an optionally substituted hydrocarbon group.

2. A single-stranded nucleic acid molecule library comprising a plurality of single-stranded nucleic acid molecules, wherein:
the plurality of single-stranded nucleic acid molecules comprise a Ds/Px-containing single-stranded nucleic acid molecule,
the Ds/Px-containing single-stranded nucleic acid molecule comprises
a pair of primer-binding regions at the 5' end and 3' end thereof, and
a variable region between the pair of primer-binding regions,
the pair of primer-binding regions consist of base sequences which are common among the plurality of single-stranded nucleic acid molecules, and
the variable region comprises
an unnatural base Ds (7-(2-thienyl)-3H-imidazo[4,5-b]pyridine-3-yl) shown in the following general formula (I) and
an unnatural base Px shown in the following general formula (III): 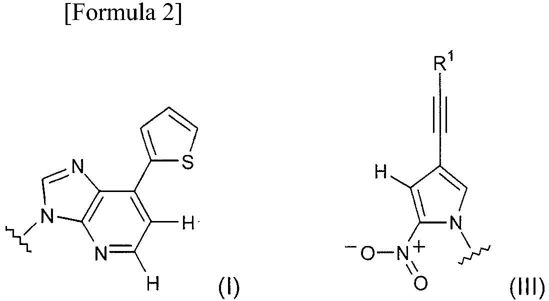
wherein R¹ respectively and independently represents a hydrogen atom or an optionally substituted hydrocarbon group.

3. The single-stranded nucleic acid molecule library of claim 1 or 2, wherein the R¹ represents any one of the following general formulas (IV) to (XXV): 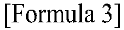 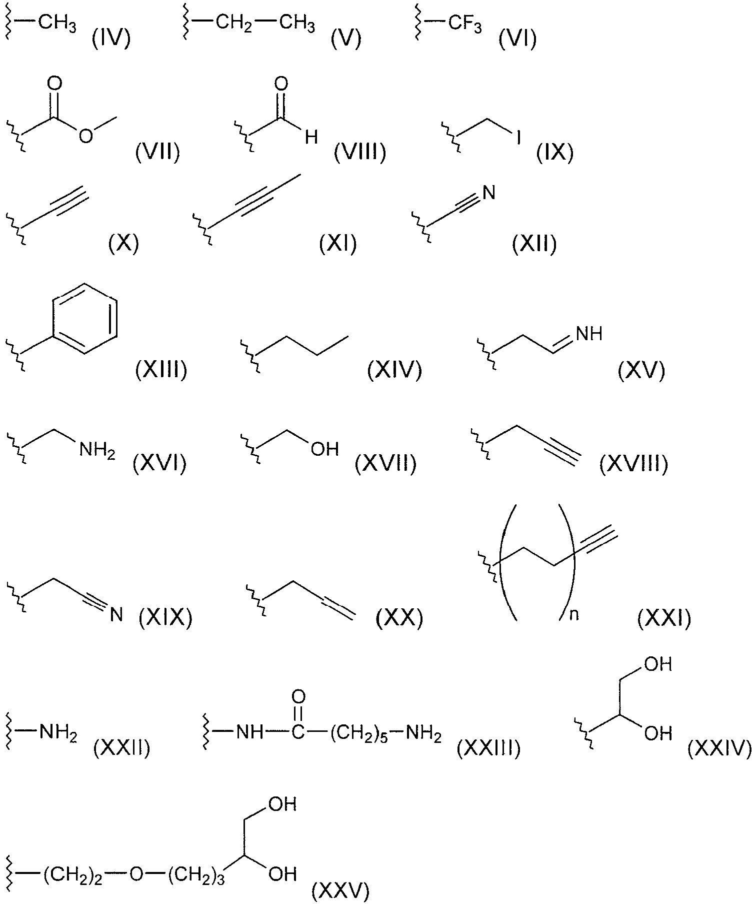 wherein the wave line represents a bond with alkyne, and n represents an integer of one or more.

4. The single-stranded nucleic acid molecule library of claim 1 or 2, wherein the variable region comprises one to ten Ds.

5. The single-stranded nucleic acid molecule library of claim 1 or 2, wherein the variable region comprises one to four Ds.

6. The single-stranded nucleic acid molecule library of claim 1 or 2, wherein the variable region comprises one or more of the Pa' or Px on average.

7. The single-stranded nucleic acid molecule library of claim 1 or 2, wherein the variable region comprises one to three of the Ds, and one to two of the Pa' or Px on average.

8. The single-stranded nucleic acid molecule library of claim 1 or 2, wherein the variable region in the Ds/Pa'-containing single-stranded nucleic acid molecule or the Ds/Px-containing single-stranded nucleic acid molecule comprises:
the Ds at one or more specified positions, and
a random sequence consisting of the Pa' or Px and natural bases except the one or more specified positions.

9. The single-stranded nucleic acid molecule library of claim 8, comprising a plurality of the Ds/Pa'-containing single-stranded nucleic acid molecules or the Ds/Px-containing single-stranded nucleic acid molecules which differ in the specified positions in the variable region.

10. The single-stranded nucleic acid molecule library of claim 1 or 2, wherein the variable region is 10 to 300 bases in length.

11. The single-stranded nucleic acid molecule library of claim 1 or 2, wherein the variable region is 14 to 100 bases in length.

12. The single-stranded nucleic acid molecule library of claim 1 or 2, wherein each of the plurality of single-stranded nucleic acid molecules comprises
a 5'-wing region consisting of natural bases between the primer-binding region at the 5' end and the variable region, and
a 3'-wing region consisting of natural bases between the variable region and the primer-binding region at the 3' end.

13. The single-stranded nucleic acid molecule library of claim 12, wherein the 5'-wing region and/or the 3'-wing region consists of a random sequence composed of natural bases.

14. The single-stranded nucleic acid molecule library of claim 13, wherein the random sequence composed of natural bases is 3 to 20 bases in length.

15. The single-stranded nucleic acid molecule library of claim 12, wherein the 5'-wing region and the 3'-wing region comprise a pair of complementary sequences which are complementary to each other and are capable of forming a stem structure.

16. The single-stranded nucleic acid molecule library of claim 15, wherein the complementary sequences comprise a non-Watson-Crick-type base pair, and/or an insertion sequence and/or a deletion of one or more bases.

17. The single-stranded nucleic acid molecule library of claim 16, wherein the non-Watson-Crick-type base pair comprises a G-T base pair.

18. The single-stranded nucleic acid molecule library of claim 16, wherein the insertion sequence forms a bulge structure or a bulge-out structure.

19. The single-stranded nucleic acid molecule library of claim 18, wherein the bulge structure or bulge-out structure comprises an unnatural base.

20. The single-stranded nucleic acid molecule library of claim 19, wherein the unnatural base is the Ds, the Pa', and/or the Px.

21. The single-stranded nucleic acid molecule library of claim 15, wherein the pair of complementary sequences are 3 to 20 bases in length.

22. A method of producing a single-stranded nucleic acid molecule library, comprising:
an amplification step of amplifying a single-stranded nucleic acid molecule
using, as a template, the single-stranded nucleic acid molecule library of claim 1, and using, as a substrate,
natural nucleotides,
Ds-containing nucleotides which contain the unnatural base Ds, and
Px-containing nucleotides which contain the unnatural base Px.

23. A method of producing a single-stranded nucleic acid molecule library, comprising:
an amplification step of amplifying a single-stranded nucleic acid molecule
using, as a template, the single-stranded nucleic acid molecule library of claim 2, and using, as a substrate,
natural nucleotides,
Ds-containing nucleotides which contain the unnatural base Ds, and
Pa'-containing nucleotides which contain the unnatural base Pa'.

24. A method of producing a nucleic acid aptamer consisting of a single-stranded nucleic acid molecule, comprising:
a complex formation step of mixing the single-stranded nucleic acid molecule library according to claim 2 with a target substance in a solution to allow for formation of a complex between a single-stranded nucleic acid molecule in the single-stranded nucleic acid molecule library and the target substance,
a complex collection step of collecting the complex, and
an amplification step of amplifying the single-stranded nucleic acid molecule comprised in the complex after the complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.

25. The method of claim 24, further comprising a repeating step of repeating the complex formation step to the amplification step once or more than once, using the single-stranded nucleic acid molecule after the amplification step as a new, single-stranded nucleic acid molecule library.

26. A method of producing a nucleic acid aptamer, comprising:
a library synthesis step of synthesizing the single-stranded nucleic acid molecule library of claim 1 by reacting a natural nucleic acid, a Ds-containing nucleic acid which contains the unnatural base Ds, and a Pa'-containing nucleic acid which contains the unnatural base Pa',
a first amplification step of amplifying a single-stranded nucleic acid molecule using, as a template, the single-stranded nucleic acid molecule library after the library synthesis step, and using, as a substrate, natural nucleotides, Ds-containing nucleotides which contain the unnatural base Ds, and Px-containing nucleotides which contain the unnatural base Px,
a complex formation step of mixing the single-stranded nucleic acid molecule after the first amplification step with a target substance in a solution to allow for formation of a complex between the single-stranded nucleic acid molecule and the target substance,
a complex collection step of collecting the complex, and
a second amplification step of amplifying the single-stranded nucleic acid molecule comprised in the complex after the complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.

27. The method of claim 26, further comprising a repeating step of repeating the complex formation step to the second amplification step once or more than once, for the single-stranded nucleic acid molecule after the second amplification step.

28. A method of producing a nucleic acid aptamer, comprising:
a library synthesis step of synthesizing the single-stranded nucleic acid molecule library of claim 1 by reacting a natural nucleic acid, a Ds-containing nucleic acid which contains the unnatural base Ds, and a Pa'-containing nucleic acid which contains the unnatural base Pa',
a first complex formation step of mixing the single-stranded nucleic acid molecule library after the library synthesis step with a target substance in a solution to allow for formation of a complex between a single-stranded nucleic acid molecule in the single-stranded nucleic acid molecule library and the target substance,
a first complex collection step of collecting the complex after the first complex formation step,
a first amplification step of amplifying a single-stranded nucleic acid molecule using, as a template, the single-stranded nucleic acid molecule after the first complex collection step, and using, as a substrate, natural nucleotides, Ds-containing nucleotides which contain the unnatural base Ds, and Px-containing nucleotides which contain the unnatural base Px,
a second complex formation step of mixing the single-stranded nucleic acid molecule after the first amplification step with the target substance in a solution to allow for formation of a complex between the single-stranded nucleic acid molecule and the target substance,
a second complex collection step of collecting the complex after the second complex formation step, and
a second amplification step of amplifying the single-stranded nucleic acid molecule comprised in the complex after the second complex collection step by a nucleic acid amplification method using natural nucleotides, Ds-containing nucleotides which contain the unnatural base Ds, and Px-containing nucleotides which contain the unnatural base Px as a substrate so as to produce a nucleic acid aptamer consisting of the single-stranded nucleic acid molecule.

29. The method of claim 28, further comprising a repeating step of repeating the second complex formation step to the second amplification step once or more than once, for the single-stranded nucleic acid molecule after the second amplification step.

30. A method of determining the base sequence of a single-stranded nucleic acid molecule containing an unnatural base,
wherein the single-stranded nucleic acid molecule containing an unnatural base comprises
a pair of primer-binding regions at the 5' end and 3' end thereof, and
a central region between the pair of primer-binding regions,
wherein the central region comprises an unnatural base Ds (7-(2-thienyl)-3H-imidazo[4,5-b]pyridine-3-yl) shown in the following general formula (I) and an unnatural base Px shown in the following general formula (III),
wherein the method comprises:
a first amplification step of amplifying the single-stranded nucleic acid molecule containing an unnatural base by a nucleic acid amplification method using natural nucleotides as a substrate and using a primer pair which binds to the base sequence of the pair of primer-binding regions, and
a sequencing step of sequencing the base sequence of an amplification product composed only of natural nucleotides which is obtained after the first amplification step: 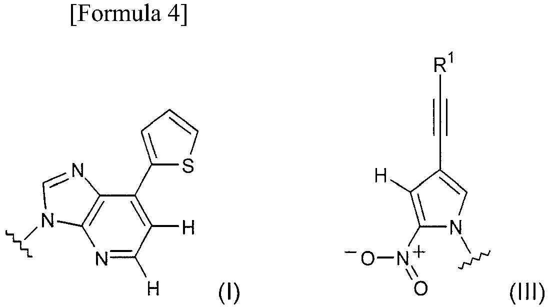
wherein R¹ respectively and independently represents a hydrogen atom or an optionally substituted hydrocarbon group having one or more carbon atoms.

31. The method of claim 30, further comprising: a second amplification step of amplifying the single-stranded nucleic acid molecule containing an unnatural base by a nucleic acid amplification method using, as a substrate, natural nucleotides,
Ds-containing nucleotides which contain the Ds, and
Px-containing nucleotides which contain the Px,
and using a primer pair which are labeled with different labeling molecules and which bind to the base sequences of the pair of primer-binding regions,
a cleavage step of completely or partially cleaving the single-stranded nucleic acid molecule containing an unnatural base and the complementary strand thereof bound to the different labeling molecules which are amplified after the second amplification step at the position(s) of the Px, and
a Px position determining step of determining the position(s) of the Px in the single-stranded nucleic acid molecule containing an unnatural base and the complementary strand thereof by determining the size of single-stranded nucleic acid fragments derived from the single-stranded nucleic acid molecule containing an unnatural base and the complementary strand thereof after the cleavage step based on detection of the labeling molecules.

32. The method of claim 31, further comprising: a Ds/Px position determining step of determining the base positions where both of natural bases A and T are sequenced in the base sequence sequenced in the sequencing step as the positions of the Ds or the Px, and
a Ds position identifying step of identifying, as the position(s) of the Ds, the position(s) other than the position(s) determined as the position(s) of the Px in the Px position determining step, among the positions of the Ds or the Px determined in the Ds/Px position determining step.

33. The method of claim 30, further comprising: a Ds/Px position determining step of determining the base positions where both of natural bases A and T are sequenced in the base sequence sequenced in the sequencing step as the positions of the Ds or the Px,
a synthesis step of synthesizing a plurality of single-stranded nucleic acid molecules which comprise the Ds and/or the Px at the positions of the Ds or the Px determined in the Ds/Px position determining step in multiple or all combinations, wherein the sequence of the single-stranded nucleic acid molecule except the position of the Ds or the Px consists of the base sequence sequenced in the sequencing step,
a complex formation step of mixing the plurality of single-stranded nucleic acid molecules synthesized in the synthesis step with a target substance to allow for formation of a complex between the plurality of single-stranded nucleic acid molecules and the target substance,
a measurement step of measuring complex formation efficiency after the complex formation step, and
a selection step of selecting the base sequence of the single-stranded nucleic acid molecule(s) which shows a higher or the highest complex formation efficiency among the plurality of single-stranded nucleic acid molecules as the base sequence of the single-stranded nucleic acid molecule containing an unnatural base, after the measurement step.

34. The method of any one of claim 30, wherein the single-stranded nucleic acid molecule containing an unnatural base
is derived from the single-stranded nucleic acid molecule library of claim 2, or
is a single-stranded nucleic acid molecule produced by the method of claim 24.

35. The method of claim 31 or 32, wherein the different labeling molecules can be distinguished based on the wavelength of fluorescence thereof.

36. The method of claim 30, wherein the central region comprises one to ten Ds.

37. The method of any one of claims 36, wherein the central region comprises one to four Ds.

38. The method of claim 30, wherein the central region comprises one to three Ds and one to two Px.

39. A nucleic acid aptamer selected from the group consisting of the following (1) to (8):
(1) a nucleic acid aptamer which binds to Interferon gamma (IFN-γ) selected from the group consisting of the following (1-i-a) to (1-iv-d):
(1-i-a) a nucleic acid aptamer comprising the base sequence of SEQ ID NO: 1, wherein the bases shown as n at positions 28 and 34 in the base sequence are an unnatural base Ds shown in the following general formula (I), and the base shown as n at position 22 in the base sequence is an unnatural base Pa' shown in the following general formula (II) or an unnatural base Px shown in the following general formula (III);
(1-i-b) a nucleic acid aptamer comprising a base sequence derived from SEQ ID NO: 1 in which one or multiple bases are deleted, substituted, or added at a position(s) except positions 22, 28, and 34 in SEQ ID NO: 1, wherein the positions in the base sequence corresponding to the bases shown as n at positions 28 and 34 in SEQ ID NO: 1 are the Ds, and the position corresponding to the base shown as n at position 22 in SEQ ID NO: 1 is the Pa' or the Px;
(1-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 2, and the bases shown as n at positions 18 and 24 in the base sequence of SEQ ID NO: 2 are the Ds, and the base shown as n at position 12 in the base sequence of SEQ ID NO: 2 is the Pa' or the Px;
(1-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 2 in which one or multiple bases are deleted, substituted, or added at a position(s) except positions 12, 18, and 24 in SEQ ID NO: 2, wherein the positions in the base sequence in the central region corresponding to the bases shown as n at positions 18 and 24 in SEQ ID NO: 2 are the Ds, and the position in the base sequence in the central region corresponding to the base shown as n at position 12 in SEQ ID NO: 2 is the Pa' or the Px;
(1-ii-a) a nucleic acid aptamer comprising the base sequence of SEQ ID NO: 3, wherein the bases shown as n at positions 27 and 33 in the base sequence are the Ds, and the base shown as n at position 21 in the base sequence is the Pa' or the Px;
(1-ii-b) a nucleic acid aptamer comprising a base sequence derived from SEQ ID NO: 3 in which one or multiple bases are deleted, substituted, or added at a position(s) except positions 21, 27, and 33 in SEQ ID NO: 3, wherein the positions in the base sequence corresponding to the bases shown as n at positions 27 and 33 in SEQ ID NO: 3 are the Ds, and the position in the base sequence corresponding to the base shown as n at position 21 in SEQ ID NO: 3 is the Pa' or the Px;
(1-iii-a) a nucleic acid aptamer comprising the base sequence of SEQ ID NO: 4, wherein the bases shown as n at positions 27 and 33 in the base sequence are the Ds;
(1-iii-b) a nucleic acid aptamer comprising a base sequence derived from SEQ ID NO: 4 in which one or multiple bases are deleted, substituted, or added at a position(s) except positions 27 and 33 in SEQ ID NO: 4, wherein the positions in the base sequence corresponding to the bases shown as n at positions 27 and 33 in SEQ ID NO: 4 are the Ds;
(1-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 5, and the bases shown as n at positions 18 and 24 in the base sequence of SEQ ID NO: 5 are the Ds;
(1-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 5 in which one or multiple bases are deleted, substituted, or added at a position(s) except positions 18 and 24 in SEQ ID NO: 5, wherein the positions in the base sequence in the central region corresponding to the bases shown as n at positions 18 and 24 in SEQ ID NO: 5 are the Ds;
(1-iv-a) a nucleic acid aptamer comprising the base sequence of SEQ ID NO: 6, wherein the base shown as n at position 33 in the base sequence is the Ds, and the base shown as n at position 21 in the base sequence is the Pa' or the Px;
(1-iv-b) a nucleic acid aptamer comprising a base sequence derived from SEQ ID NO: 6 in which one or multiple bases are deleted, substituted, or added at a position(s) except positions 21 and 33 in SEQ ID NO: 6, wherein the position in the base sequence corresponding to the base shown as n at position 33 in SEQ ID NO: 6 is the Ds, and the position in the base sequence corresponding to the base shown as n at position 21 in SEQ ID NO: 6 is the Pa' or the Px;
(1-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 7, and the base shown as n at position 24 in the base sequence of SEQ ID NO: 7 is the Ds, and the base shown as n at position 12 in the base sequence of SEQ ID NO: 7 is the Pa' or the Px; and
(1-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 7 in which one or multiple bases are deleted, substituted, or added at a position(s) except positions 12 and 24 in SEQ ID NO: 7, wherein the position in the base sequence in the central region corresponding to the base shown as n at position 24 in SEQ ID NO: 7 is the Ds, and the position in the base sequence in the central region corresponding to the base shown as n at position 12 in SEQ ID NO: 7 is the Pa' or the Px;
(2) a nucleic acid aptamer which binds to Interferon gamma (IFN-γ) selected from the group consisting of the following (2-i-a) to (2-vi-d):
(2-i-a) a nucleic acid aptamer comprising the base sequence of SEQ ID NO: 8, wherein the bases shown as n at positions 24 and 37 in the base sequence are the Ds;
(2-i-b) a nucleic acid aptamer comprising a base sequence derived from SEQ ID NO: 8 in which one or multiple bases are deleted, substituted, or added at a position(s) except positions 13, 24, 31, and 37 in SEQ ID NO: 8, wherein the positions in the base sequence corresponding to the bases shown as n at positions 24 and 37 in SEQ ID NO: 8 are the Ds;
(2-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 9, and the bases shown as n at positions 15 and 28 in the base sequence of SEQ ID NO: 9 are the Ds;
(2-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 9 in which one or multiple bases are deleted, substituted, or added at a position(s) except positions 4, 15, 22, and 28 in SEQ ID NO: 9, wherein the positions in the base sequence in the central region corresponding to the bases shown as n at positions 15 and 28 in SEQ ID NO: 9 are the Ds;
(2-ii-a) a nucleic acid aptamer comprising the base sequence of SEQ ID NO: 10, wherein the bases shown as n at positions 17, 24, and 37 in the base sequence are the Ds;
(2-ii-b) a nucleic acid aptamer comprising a base sequence derived from SEQ ID NO: 10 in which one or multiple bases are deleted, substituted, or added at a position(s) except positions 13, 17, 24, 31, and 37 in SEQ ID NO: 10, wherein the positions in the base sequence corresponding to the bases shown as n at positions 17, 24, and 37 in SEQ ID NO: 10 are the Ds;
(2-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 11, and the bases shown as n at positions 8, 15, and 28 in the base sequence of SEQ ID NO: 11 are the Ds;
(2-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 11 in which one or multiple bases are deleted, substituted, or added at a position(s) except positions 4, 8, 15, 22, and 28 in SEQ ID NO: 11, wherein the positions in the base sequence in the central region corresponding to the bases shown as n at positions 8, 15, and 28 in SEQ ID NO: 11 are the Ds;
(2-iii-a) a nucleic acid aptamer comprising the base sequence of SEQ ID NO: 12, wherein the bases shown as n at positions 18, 24, and 37 in the base sequence are the Ds;
(2-iii-b) a nucleic acid aptamer comprising a base sequence derived from SEQ ID NO: 12 in which one or multiple bases are deleted, substituted, or added at a position(s) except positions 13, 18, 24, 31, and 37 in SEQ ID NO: 12, wherein the positions in the base sequence corresponding to the bases shown as n at positions 18, 24, and 37 in SEQ ID NO: 12 are the Ds;
(2-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 13, and the bases shown as n at positions 9, 15, and 28 in the base sequence of SEQ ID NO: 13 are the Ds;
(2-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 13 in which one or multiple bases are deleted, substituted, or added at a position(s) except positions 4, 9, 15, 22, and 28 in SEQ ID NO: 13, wherein the positions in the base sequence in the central region corresponding to the bases shown as n at positions 9, 15, and 28 in SEQ ID NO: 13 are the Ds;
(2-iv-a) a nucleic acid aptamer comprising the base sequence of SEQ ID NO: 14, wherein the bases shown as n at positions 24 and 37 in the base sequence are the Ds, and the base shown as n at position 17 in the base sequence is the Pa' or the Px;
(2-iv-b) a nucleic acid aptamer comprising a base sequence derived from SEQ ID NO: 14 in which one or multiple bases are deleted, substituted, or added at a position(s) except positions 13, 17, 24, 31, and 37 in SEQ ID NO: 14, wherein the positions in the base sequence corresponding to the bases shown as n at positions 24 and 37 in SEQ ID NO: 14 are the Ds, and the position in the base sequence corresponding to the base shown as n at position 17 in SEQ ID NO: 14 is the Pa' or the Px;
(2-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 15, and the bases shown as n at positions 15 and 28 in the base sequence of SEQ ID NO: 15 are the Ds, and the base shown as n at position 8 in the base sequence of SEQ ID NO: 15 is the Pa' or the Px;
(2-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 15 in which one or multiple bases are deleted, substituted, or added at a position(s) except positions 4, 8, 15, 22, and 28 in SEQ ID NO: 15, wherein the positions in the base sequence in the central region corresponding to the bases shown as n at positions 15 and 28 in SEQ ID NO: 15 are the Ds, and the position in the base sequence in the central region corresponding to the base shown as n at position 8 in SEQ ID NO: 15 is the Pa' or the Px;
(2-v-a) a nucleic acid aptamer comprising the base sequence of SEQ ID NO: 16, wherein the bases shown as n at positions 24 and 37 in the base sequence are the Ds, and the base shown as n at position 18 in the base sequence is the Pa' or the Px;
(2-v-b) a nucleic acid aptamer comprising a base sequence derived from SEQ ID NO: 16 in which one or multiple bases are deleted, substituted, or added at a position(s) except positions 13, 18, 24, 31, and 37 in SEQ ID NO: 16, wherein the positions in the base sequence corresponding to the bases shown as n at positions 24 and 37 in SEQ ID NO: 16 are the Ds, and the position in the base sequence corresponding to the base shown as n at position 18 in SEQ ID NO: 16 is the Pa' or the Px;
(2-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 17, and the bases shown as n at positions 15 and 28 in the base sequence of SEQ ID NO: 17 are the Ds, and the base shown as n at position 9 in the base sequence of SEQ ID NO: 17 is the Pa' or the Px;
(2-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 17 in which one or multiple bases are deleted, substituted, or added at a position(s) except positions 4, 9, 15, 22, and 28 in SEQ ID NO: 17, wherein the positions in the base sequence in the central region corresponding to the bases shown as n at positions 15 and 28 in SEQ ID NO: 17 are the Ds, and the position in the base sequence in the central region corresponding to the base shown as n at position 9 in SEQ ID NO: 17 is the Pa' or the Px;
(2-vi-a) a nucleic acid aptamer comprising the base sequence of SEQ ID NO: 18, wherein the base(s) shown as n at position(s) 17, 18, 24, and/or 37 in the base sequence are the Ds, the Pa', and/or the Px;
(2-vi-b) a nucleic acid aptamer comprising a base sequence derived from SEQ ID NO: 18 in which one or multiple bases are deleted, substituted, or added at a position(s) except positions 13, 17, 18, 24, 31, and 37 in SEQ ID NO: 18, wherein the positions in the base sequence corresponding to the base(s) shown as n at position(s) 17, 18, 24, and/or 37 in SEQ ID NO: 18 are the Ds, the Pa', and/or the Px;
(2-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 19, and the base(s) shown as n at position(s) 8, 9, 15, and/or 28 in the base sequence of SEQ ID NO: 19 are the Ds, the Pa', and/or the Px; and
(2-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 19 in which one or multiple bases are deleted, substituted, or added at a position(s) except positions 4, 8, 9, 15, 22, and 28 in SEQ ID NO: 19, wherein the position(s) in the base sequence in the central region corresponding to the base(s) shown as n at positions 8, 9, 15, and/or 28 in SEQ ID NO: 19 are the Ds, the Pa', and/or the Px;
(3) a nucleic acid aptamer which binds to C-reactive protein (CRP) selected from the group consisting of the following (3-i-a) to (3-v-b):
(3-i-a) a nucleic acid aptamer comprising the base sequence of SEQ ID NO: 20, wherein the bases shown as n at positions 9 and 20 in the base sequence are the Ds, and the base shown as n at position 32 in the base sequence is the Pa' or the Px;
(3-i-b) a nucleic acid aptamer comprising a base sequence derived from SEQ ID NO: 20 in which one or multiple bases are deleted, substituted, or added at a position(s) except positions 9, 20, and 32 in SEQ ID NO: 20, wherein the positions in the base sequence corresponding to the bases shown as n at positions 9 and 20 in SEQ ID NO: 20 are the Ds, and the position in the base sequence corresponding to the base shown as n at position 32 in SEQ ID NO: 20 is the Pa' or the Px;
(3-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 21, and the base shown as n at position 2 in the base sequence of SEQ ID NO: 21 is the Ds, and the base shown as n at position 14 in the base sequence of SEQ ID NO: 21 is the Pa' or the Px;
(3-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 21 in which one or multiple bases are deleted, substituted, or added at a position(s) except positions 2 and 14 in SEQ ID NO: 21, wherein the positions in the base sequence in the central region corresponding to the base shown as n at position 2 in SEQ ID NO: 21 is the Ds, and the position in the base sequence in the central region corresponding to the base shown as n at position 14 in SEQ ID NO: 21 is the Pa' or the Px;
(3-ii-a) a nucleic acid aptamer comprising the base sequence of SEQ ID NO: 22, wherein the base shown as n at position 20 in the base sequence is the Ds, and the base shown as n at position 32 in the base sequence is the Pa' or the Px;
(3-ii-b) a nucleic acid aptamer comprising a base sequence derived from SEQ ID NO: 22 in which one or multiple bases are deleted, substituted, or added at a position(s) except positions 20 and 32 in SEQ ID NO: 22, wherein the position in the base sequence corresponding to the base shown as n at position 20 in SEQ ID NO: 22 is the Ds, and the position in the base sequence corresponding to the base shown as n at position 32 in SEQ ID NO: 22 is the Pa' or the Px;
(3-iii-a) a nucleic acid aptamer comprising the base sequence of SEQ ID NO: 23, wherein the bases shown as n at positions 9 and 20 in the base sequence are the Ds, and the base shown as n at position 31 in the base sequence is the Pa' or the Px;
(3-iii-b) a nucleic acid aptamer comprising a base sequence derived from SEQ ID NO: 23 in which one or multiple bases are deleted, substituted, or added at a position(s) except positions 9, 20, and 31 in SEQ ID NO: 23, wherein the positions in the base sequence corresponding to the bases shown as n at positions 9 and 20 in SEQ ID NO: 23 are the Ds, and the position in the base sequence corresponding to the base shown as n at position 31 in SEQ ID NO: 23 is the Pa' or the Px;
(3-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 24, and the base shown as n at position 2 in the base sequence of SEQ ID NO: 24 is the Ds, and the base shown as n at position 13 in the base sequence of SEQ ID NO: 24 is the Pa' or the Px;
(3-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 24 in which one or multiple bases are deleted, substituted, or added at a position(s) except positions 2 and 13 in SEQ ID NO: 24, wherein the position in the base sequence in the central region corresponding to the base shown as n at position 2 in SEQ ID NO: 24 is the Ds, and the position in the base sequence in the central region corresponding to the base shown as n at position 13 in SEQ ID NO: 24 is the Pa' or the Px;
(3-iv-a) a nucleic acid aptamer comprising the base sequence of SEQ ID NO: 25, wherein the base shown as n at position 14 in the base sequence is the Ds, and the base shown as n at position 26 in the base sequence is the Pa' or the Px;
(3-iv-b) a nucleic acid aptamer comprising a base sequence derived from SEQ ID NO: 25 in which one or multiple bases are deleted, substituted, or added at a position(s) except positions 14 and 26 in SEQ ID NO: 25, wherein the position in the base sequence corresponding to the base shown as n at position 14 in SEQ ID NO: 25 is the Ds, and the position in the base sequence corresponding to the base shown as n at position 26 in SEQ ID NO: 25 is the Pa' or the Px;
(3-v-a) a nucleic acid aptamer comprising the base sequence of SEQ ID NO: 26, wherein the base shown as n at positions 14 in the base sequence is the Ds, and the base shown as n at position 25 in the base sequence is the Pa' or the Px; and
(3-v-b) a nucleic acid aptamer comprising a base sequence derived from SEQ ID NO: 26 in which one or multiple bases are deleted, substituted, or added at a position(s) except positions 14 and 25 in SEQ ID NO: 26, wherein the position in the base sequence corresponding to the base shown as n at position 14 in SEQ ID NO: 26 is the Ds, and the position in the base sequence corresponding to the base shown as n at position 25 in SEQ ID NO: 26 is the Pa' or the Px;
(4) a nucleic acid aptamer which binds to Thrombin selected from the group consisting of the following (4-i-a) to (4-vi-d):
(4-i-a) a nucleic acid aptamer comprising the base sequence of SEQ ID NO: 27, wherein the bases shown as n at positions 16 and 22 in the base sequence are the Ds;
(4-i-b) a nucleic acid aptamer comprising a base sequence derived from SEQ ID NO: 27 in which one or multiple bases are deleted, substituted, or added at a position(s) except positions 16 and 22 in SEQ ID NO: 27, wherein the positions in the base sequence corresponding to the bases shown as n at positions 16 and 22 in SEQ ID NO: 27 are the Ds;
(4-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 28, and the bases shown as n at positions 6 and 12 in the base sequence of SEQ ID NO: 28 are the Ds;
(4-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 28 in which one or multiple bases are deleted, substituted, or added at a position(s) except positions 6 and 12 in SEQ ID NO: 28, wherein the positions in the base sequence in the central region corresponding to the bases shown as n at positions 6 and 12 in SEQ ID NO: 28 are the Ds;
(4-ii-a) a nucleic acid aptamer comprising the base sequence of SEQ ID NO: 29, wherein the bases shown as n at positions 10 and 16 in the base sequence are the Ds, and the base shown as n at position 28 in the base sequence is the Pa' or the Px;
(4-ii-b) a nucleic acid aptamer comprising a base sequence derived from SEQ ID NO: 29 in which one or multiple bases are deleted, substituted, or added at a position(s) except positions 10, 16, and 28 in SEQ ID NO: 29, wherein the positions in the base sequence corresponding to the bases shown as n at positions 10 and 16 in SEQ ID NO: 29 are the Ds, and the position in the base sequence corresponding to the base shown as n at position 28 in SEQ ID NO: 29 is the Pa' or the Px;
(4-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 30, and the bases shown as n at positions 1 and 7 in the base sequence of SEQ ID NO: 30 are the Ds, and the base shown as n at position 19 in the base sequence of SEQ ID NO: 30 is the Pa' or the Px;
(4-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 30 in which one or multiple bases are deleted, substituted, or added at a position(s) except positions 1, 7, and 19 in SEQ ID NO: 30, wherein the positions in the base sequence in the central region corresponding to the bases shown as n at positions 1 and 7 in SEQ ID NO: 30 are the Ds, and the position in the base sequence in the central region corresponding to the base shown as n at position 19 in SEQ ID NO: 30 is the Pa' or the Px;
(4-iii-a) a nucleic acid aptamer comprising the base sequence of SEQ ID NO: 31, wherein the bases shown as n at positions 26 and 41 in the base sequence are the Ds;
(4-iii-b) a nucleic acid aptamer comprising a base sequence derived from SEQ ID NO: 31 in which one or multiple bases are deleted, substituted, or added at a position(s) except positions 26 and 41 in SEQ ID NO: 31, wherein the positions in the base sequence corresponding to the bases shown as n at positions 26 and 41 in SEQ ID NO: 31 are the Ds;
(4-iii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 32, and the bases shown as n at positions 18 and 33 in the base sequence of SEQ ID NO: 32 are the Ds;
(4-iii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 32 in which one or multiple bases are deleted, substituted, or added at a position(s) except positions 18 and 33 in SEQ ID NO: 32, wherein the positions in the base sequence in the central region corresponding to the bases shown as n at positions 18 and 33 in SEQ ID NO: 32 are the Ds;
(4-iv-a) a nucleic acid aptamer comprising the base sequence of SEQ ID NO: 33, wherein the bases shown as n at positions 16 and 24 in the base sequence are the Ds, and the base shown as n at position 35 in the base sequence is the Pa' or the Px;
(4-iv-b) a nucleic acid aptamer comprising a base sequence derived from SEQ ID NO: 33 in which one or multiple bases are deleted, substituted, or added at a position(s) except positions 16, 24, and 35 in SEQ ID NO: 33, wherein the positions in the base sequence corresponding to the bases shown as n at positions 16 and 24 in SEQ ID NO: 33 are the Ds, and the position in the base sequence corresponding to the base shown as n at position 35 in SEQ ID NO: 33 is the Pa' or the Px;
(4-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 34, and the bases shown as n at positions 10 and 18 in the base sequence of SEQ ID NO: 34 are the Ds, and the base shown as n at position 29 in the base sequence of SEQ ID NO: 34 is the Pa' or the Px;
(4-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 34 in which one or multiple bases are deleted, substituted, or added at a position(s) except positions 10, 18, and 29 in SEQ ID NO: 34, wherein the positions in the base sequence in the central region corresponding to the bases shown as n at positions 10 and 18 in SEQ ID NO: 34 are the Ds, and the position in the base sequence in the central region corresponding to the base shown as n at position 29 in SEQ ID NO: 34 is the Pa' or the Px;
(4-v-a) a nucleic acid aptamer comprising the base sequence of SEQ ID NO: 35, wherein the bases shown as n at positions 17 and 24 in the base sequence are the Ds, and the bases shown as n at position 36 and 39 in the base sequence are the Pa' or the Px;
(4-v-b) a nucleic acid aptamer comprising a base sequence derived from SEQ ID NO: 35 in which one or multiple bases are deleted, substituted, or added at a position(s) except positions 17, 24, 36, and 39 in SEQ ID NO: 35, wherein the positions in the base sequence corresponding to the bases shown as n at positions 17 and 24 in SEQ ID NO: 35 are the Ds, and the positions in the base sequence corresponding to the bases shown as n at position 36 and 39 in SEQ ID NO: 35 are the Pa' or the Px;
(4-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 36, and the bases shown as n at positions 9 and 16 in the base sequence of SEQ ID NO: 36 are the Ds, and the bases shown as n at position 28 and 31 in the base sequence of SEQ ID NO: 36 are the Pa' or the Px;
(4-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 36 in which one or multiple bases are deleted, substituted, or added at a position(s) except positions 9, 16, 28, and 31 in SEQ ID NO: 36, wherein the positions in the base sequence in the central region corresponding to the bases shown as n at positions 9 and 16 in SEQ ID NO: 36 are the Ds, and the positions in the base sequence in the central region corresponding to the bases shown as n at positions 28 and 31 in SEQ ID NO: 36 are the Pa' or the Px;
(4-vi-a) a nucleic acid aptamer comprising the base sequence of SEQ ID NO: 37, wherein the bases shown as n at positions 29 and 38 in the base sequence are the Ds;
(4-vi-b) a nucleic acid aptamer comprising a base sequence derived from SEQ ID NO: 37 in which one or multiple bases are deleted, substituted, or added at a position(s) except positions 29 and 38 in SEQ ID NO: 37, wherein the positions in the base sequence corresponding to the bases shown as n at positions 29 and 38 in SEQ ID NO: 37 are the Ds;
(4-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 38, and the base shown as n at position 19 in the base sequence of SEQ ID NO: 38 is the Ds; and
(4-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 38 in which one or multiple bases are deleted, substituted, or added at a position(s) except position 19 in SEQ ID NO: 38, wherein the position in the base sequence in the central region corresponding to the bases shown as n at position 19 in SEQ ID NO: 38 is the Ds;
(5) a nucleic acid aptamer which binds to Interleukin-6 (IL-6) selected from the group consisting of the following (5-i-a) to (5-ii-d):
(5-i-a) a nucleic acid aptamer comprising the base sequence of SEQ ID NO: 39, wherein the bases shown as n at positions 25 and 34 in the base sequence are the Ds, and the bases shown as n at positions 13 and 20 in the base sequence are the Pa' or the Px;
(5-i-b) a nucleic acid aptamer comprising a base sequence derived from SEQ ID NO: 39 in which one or multiple bases are deleted, substituted, or added at a position(s) except positions 13, 20, 25, and 34 in SEQ ID NO: 39, wherein the positions in the base sequence corresponding to the bases shown as n at positions 25 and 34 in SEQ ID NO: 39 are the Ds, and the positions in the base sequence corresponding to the bases shown as n at positions 13 and 20 in SEQ ID NO: 39 are the Pa' or the Px;
(5-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 40, and the bases shown as n at positions 14 and 23 in the base sequence of SEQ ID NO: 40 are the Ds, and the bases shown as n at positions 2 and 9 in the base sequence of SEQ ID NO: 40 are the Pa' or the Px;
(5-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 40 in which one or multiple bases are deleted, substituted, or added at a position(s) except positions 2, 9, 14, and 23 in SEQ ID NO: 40, wherein the positions in the base sequence in the central region corresponding to the bases shown as n at positions 14 and 23 in SEQ ID NO: 40 are the Ds, and the positions in the base sequence in the central region corresponding to the bases shown as n at positions 2 and 9 in SEQ ID NO: 40 are the Pa' or the Px;
(5-ii-a) a nucleic acid aptamer comprising the base sequence of SEQ ID NO: 41, wherein the bases shown as n at positions 17 and 33 in the base sequence are the Ds, and the base shown as n at position 38 in the base sequence is the Pa' or the Px;
(5-ii-b) a nucleic acid aptamer comprising a base sequence derived from SEQ ID NO: 41 in which one or multiple bases are deleted, substituted, or added at a position(s) except positions 17, 33, and 38 in SEQ ID NO: 41, wherein the positions in the base sequence corresponding to the bases shown as n at positions 17 and 33 in SEQ ID NO: 41 are the Ds, and the position in the base sequence corresponding to the base shown as n at position 38 in SEQ ID NO: 41 is the Pa' or the Px;
(5-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 42, and the bases shown as n at positions 1 and 17 in the base sequence of SEQ ID NO: 42 are the Ds, and the base shown as n at position 22 in the base sequence of SEQ ID NO: 42 is the Pa' or the Px;
(5-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 42 in which one or multiple bases are deleted, substituted, or added at a position(s) except positions 1, 17, and 22 in SEQ ID NO: 42, wherein the positions in the base sequence in the central region corresponding to the bases shown as n at positions 1 and 17 in SEQ ID NO: 42 are the Ds, and the position in the base sequence in the central region corresponding to the base shown as n at position 22 in SEQ ID NO: 42 is the Pa' or the Px; and
(6) a nucleic acid aptamer which binds to Interleukin-8 (IL-8) selected from the group consisting of the following (6-i-a) to (6-vii-d):
(6-i-a) a nucleic acid aptamer comprising the base sequence of SEQ ID NO: 43, wherein the base shown as n at position 26 in the base sequence is the Ds, and the base shown as n at position 17 in the base sequence is the Pa' or the Px;
(6-i-b) a nucleic acid aptamer comprising a base sequence derived from SEQ ID NO: 43 in which one or multiple bases are deleted, substituted, or added at a position(s) except positions 17 and 26 in SEQ ID NO: 43, wherein the position in the base sequence corresponding to the base shown as n at position 26 in SEQ ID NO: 43 is the Ds, and the position in the base sequence corresponding to the base shown as n at position 17 in SEQ ID NO: 43 is the Pa' or the Px;
(6-i-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 44, and the base shown as n at position 12 in the base sequence of SEQ ID NO: 44 is the Ds, and the base shown as n at position 3 in the base sequence of SEQ ID NO: 44 is the Pa' or the Px;
(6-i-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 44 in which one or multiple bases are deleted, substituted, or added at a position(s) except positions 3 and 12 in SEQ ID NO: 44, wherein the position in the base sequence in the central region corresponding to the base shown as n at position 12 in SEQ ID NO: 44 is the Ds, and the position in the base sequence in the central region corresponding to the base shown as n at position 3 in SEQ ID NO: 44 is the Pa' or the Px;
(6-ii-a) a nucleic acid aptamer comprising the base sequence of SEQ ID NO: 45, wherein the base shown as n at position 26 in the base sequence is the Ds, and the base shown as n at position 17 in the base sequence is the Pa' or the Px;
(6-ii-b) a nucleic acid aptamer comprising a base sequence derived from SEQ ID NO: 45 in which one or multiple bases are deleted, substituted, or added at a position(s) except positions 17 and 26 in SEQ ID NO: 45, wherein the position in the base sequence corresponding to the base shown as n at position 26 in SEQ ID NO: 45 is the Ds, and the position in the base sequence corresponding to the base shown as n at position 17 in SEQ ID NO: 45 is the Pa' or the Px;
(6-ii-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 46, and the base shown as n at position 12 in the base sequence of SEQ ID NO: 46 is the Ds, and the base shown as n at position 3 in the base sequence of SEQ ID NO: 46 is the Pa' or the Px;
(6-ii-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 46 in which one or multiple bases are deleted, substituted, or added at a position(s) except positions 3 and 12 in SEQ ID NO: 46, wherein the position in the base sequence in the central region corresponding to the base shown as n at position 12 in SEQ ID NO: 46 is the Ds, and the position in the base sequence in the central region corresponding to the bases shown as n at position 3 in SEQ ID NO: 46 is the Pa' or the Px;
(6-iii-a) a nucleic acid aptamer comprising the base sequence of SEQ ID NO: 47, wherein the bases shown as n at positions 26 and 37 in the base sequence are the Ds, and the base shown as n at position 17 in the base sequence is the Pa' or the Px;
(6-iii-b) a nucleic acid aptamer comprising a base sequence derived from SEQ ID NO: 47 in which one or multiple bases are deleted, substituted, or added at a position(s) except positions 17, 26, and 37 in SEQ ID NO: 47, wherein the positions in the base sequence corresponding to the bases shown as n at positions 26 and 37 in SEQ ID NO: 47 are the Ds, and the position in the base sequence corresponding to the base shown as n at position 17 in SEQ ID NO: 47 is the Pa' or the Px;
(6-iii-c) a nucleic acid aptamer comprising the base sequence of SEQ ID NO: 48, wherein the base shown as n at position 25 in the base sequence is the Ds, and the base shown as n at position 16 in the base sequence is the Pa' or the Px;
(6-iii-d) a nucleic acid aptamer comprising a base sequence derived from SEQ ID NO: 48 in which one or multiple bases are deleted, substituted, or added at a position(s) except positions 16 and 25 in SEQ ID NO: 48, wherein the position in the base sequence corresponding to the base shown as n at position 25 in SEQ ID NO: 48 is the Ds, and the position in the base sequence corresponding to the base shown as n at position 16 in SEQ ID NO: 48 is the Pa' or the Px;
(6-iv-a) a nucleic acid aptamer comprising the base sequence of SEQ ID NO: 43, wherein the bases shown as n at positions 17 and 26 in the base sequence are the Ds;
(6-iv-b) a nucleic acid aptamer comprising a base sequence derived from SEQ ID NO: 43 in which one or multiple bases are deleted, substituted, or added at a position(s) except positions 17 and 26 in SEQ ID NO: 43, wherein the positions in the base sequence corresponding to the bases shown as n at positions 17 and 26 in SEQ ID NO: 43 are the Ds;
(6-iv-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 44, and the bases shown as n at positions 3 and 12 in the base sequence of SEQ ID NO: 44 are the Ds;
(6-iv-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 44 in which one or multiple bases are deleted, substituted, or added at a position(s) except positions 3 and 12 in SEQ ID NO: 44, wherein the positions in the base sequence in the central region corresponding to the bases shown as n at positions 3 and 12 in SEQ ID NO: 44 are the Ds;
(6-v-a) a nucleic acid aptamer comprising the base sequence of SEQ ID NO: 45, wherein the bases shown as n at positions 17 and 26 in the base sequence are the Ds;
(6-v-b) a nucleic acid aptamer comprising a base sequence derived from SEQ ID NO: 45 in which one or multiple bases are deleted, substituted, or added at a position(s) except positions 17 and 26 in SEQ ID NO: 45, wherein the positions in the base sequence corresponding to the bases shown as n at positions 17 and 26 in SEQ ID NO: 45 are the Ds;
(6-v-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 46, and the bases shown as n at positions 3 and 12 in the base sequence of SEQ ID NO: 46 are the Ds;
(6-v-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 46 in which one or multiple bases are deleted, substituted, or added at a position(s) except positions 3 and 12 in SEQ ID NO: 46, wherein the positions in the base sequence in the central region corresponding to the bases shown as n at positions 3 and 12 in SEQ ID NO: 46 are the Ds;
(6-vi-a) a nucleic acid aptamer comprising the base sequence of SEQ ID NO: 49, wherein the bases shown as n at positions 17 and 30 in the base sequence are the Ds, and the base shown as n at position 23 in the base sequence is the Pa' or the Px;
(6-vi-b) a nucleic acid aptamer comprising a base sequence derived from SEQ ID NO: 49 in which one or multiple bases are deleted, substituted, or added at a position(s) except positions 17, 23, and 30 in SEQ ID NO: 49, wherein the positions in the base sequence corresponding to the bases shown as n at positions 17 and 30 in SEQ ID NO: 49 are the Ds, and the position in the base sequence corresponding to the base shown as n at position 23 in SEQ ID NO: 49 is the Pa' or the Px;
(6-vi-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 50, and the bases shown as n at positions 3 and 16 in the base sequence of SEQ ID NO: 50 are the Ds, and the base shown as n at position 9 in the base sequence of SEQ ID NO: 50 is the Pa' or the Px; and
(6-vi-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 50 in which one or multiple bases are deleted, substituted, or added at a position(s) except positions 3, 9, and 16 in SEQ ID NO: 50, wherein the positions in the base sequence in the central region corresponding to the bases shown as n at positions 3 and 16 in SEQ ID NO: 50 are the Ds, and the position in the base sequence in the central region corresponding to the base shown as n at position 9 in SEQ ID NO: 50 is the Pa' or the Px;
(7) a nucleic acid aptamer that binds to HMGB1 protein selected from the group consisting of the following (7-a) to (7-d):
(7-a) a nucleic acid aptamer comprising the base sequence of SEQ ID NO: 135, wherein the bases shown as n at positions 22 and 33 in the base sequence are the Ds;
(7-b) a nucleic acid aptamer comprising a base sequence derived from SEQ ID NO: 135 in which one or multiple bases are deleted, substituted, or added at a position(s) except positions 22 and 33 in SEQ ID NO: 135, wherein the positions in the base sequence corresponding to the bases shown as n at positions 22 and 33 in SEQ ID NO: 135 are the Ds;
(7-c) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 134, and the bases shown as n at positions 12 and 23 in the base sequence of SEQ ID NO: 134 are the Ds; and
(7-d) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 134 in which one or multiple bases are deleted, substituted, or added at a position(s) except positions 12 and 23 in SEQ ID NO: 134, wherein the positions in the base sequence in the central region corresponding to the bases shown as n at positions 12 and 23 in SEQ ID NO: 134 are the Ds;
(8) a nucleic acid aptamer which binds to Interferon gamma (IFN-γ) selected from the group consisting of the following (8-i-a) to (8-xii-b):
(8-i-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 213, and the bases shown as n at positions 10 and 26 in the base sequence of SEQ ID NO: 213 are the Ds, and the base shown as n at position 16 in the base sequence of SEQ ID NO: 213 is the Pa', the Px, or the T base;
(8-i-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 213 in which one or multiple bases are deleted, substituted, or added at a position(s) except positions 10, 16, and 26 in SEQ ID NO: 213, wherein the positions in the base sequence in the central region corresponding to the bases shown as n at positions 10 and 26 in SEQ ID NO: 213 are the Ds, and the position in the base sequence in the central region corresponding to the base shown as n at position 16 in SEQ ID NO: 213 is the Pa', the Px, or the T base;
(8-ii-a) the nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 214, wherein the base shown as n at position 2 in the base sequence of SEQ ID NO: 214 is the Pa', the Px, or the T base, the base shown as n at position 16 in the base sequence of SEQ ID NO: 214 is the Ds, the Pa', or the Px, the base shown as n at position 26 in the base sequence of SEQ ID NO: 214 is the Pa' or the Px, the base shown as n at position 31 in the base sequence of SEQ ID NO: 214 is the Ds;
(8-ii-b) the nucleic acid aptamer comprising a 5' region, a central region, and a 3' region in this order from the 5' end, wherein the 5' region and 3' region comprise a pair of base sequences which are complementary to each other, and are capable of forming a stem structure, and wherein the central region comprises a base sequence derived from SEQ ID NO: 214 in which one or multiple bases are deleted, substituted, or added at a position(s) except positions 2, 16, 26, and 31 in SEQ ID NO: 214, and wherein the position in the base sequence in the central region corresponding to the base shown as n at position 2 in SEQ ID NO: 214 is the Pa', the Px, or the T base, the position in the base sequence in the central region corresponding to the base shown as n at position 16 in SEQ ID NO: 214 is the Ds, the Pa', or the Px, the position in the base sequence in the central region corresponding to the base shown as n at position 26 in SEQ ID NO: 214 is the Pa' or the Px, and the position in the base sequence in the central region corresponding to the base shown as n at position 31 in SEQ ID NO: 214 is the Ds;
(8-iii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 215, and the bases shown as n at positions 12 and 18 in the base sequence of SEQ ID NO: 215 are the Ds or the base A, and the base shown as n at position 18 in the base sequence of SEQ ID NO: 215 is the Pa', the Px, or the T base;
(8-iii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 215 in which one or multiple bases are deleted, substituted, or added at a position(s) except positions 12, 18, and 28 in SEQ ID NO: 215, wherein the positions in the positions in the base sequence in the central region corresponding to the bases shown as n at positions 12 and 28 in SEQ ID NO: 215 are the Ds or the base A, and the position in the base sequence in the central region corresponding to the base shown as n at position 18 in SEQ ID NO: 215 is the Pa', the Px, or the T base;
(8-iv-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of any of SEQ ID NOs: 216 to 221, 227, and 228, and the bases shown as n at positions 12 and 18 in the base sequence of any of SEQ ID NOs: 216 to 221, 227, and 228 are the Ds, and the base shown as n at position 18 in the base sequence of any of SEQ ID NOs: 216 to 221, 227, and 228 is the Pa' or the Px;
(8-iv-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from any of SEQ ID NOs: 216 to 221, 227, and 228 in which one or multiple bases are deleted, substituted, or added at a position(s) except positions 12, 18, and 28 in any of SEQ ID NOs: 216 to 221, 227, and 228, wherein the positions in the base sequence in the central region corresponding to the bases shown as n at positions 12 and 28 in any of SEQ ID NOs: 216 to 221, 227, and 228 are the Ds, and the position in the base sequence in the central region corresponding to the base shown as n at position 18 in any of SEQ ID NOs: 216 to 221, 227, and 228 is the Pa' or the Px;
(8-v-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 222, and the bases shown as n at positions 12 and 28 in the base sequence of SEQ ID NO: 222 are the Ds, and the bases shown as n at position 16 and 18 in the base sequence of SEQ ID NO: 222 is the Pa' or the Px;
(8-v-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 222 in which one or multiple bases are deleted, substituted, or added at a position(s) except positions 12, 16, 18, and 28 in SEQ ID NO: 222, wherein the positions in the base sequence in the central region corresponding to the bases shown as n at positions 12 and 28 in SEQ ID NO: 222 are the Ds, and the positions in the base sequence in the central region corresponding to the bases shown as n at positions 16 and 18 in SEQ ID NO: 222 are the Pa' or the Px;
(8-vi-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 223, and the bases shown as n at positions 12 and 28 in the base sequence of SEQ ID NO: 223 are the Ds, and the bases shown as n at position 18 and 34 in the base sequence of SEQ ID NO: 223 are the Pa' or the Px;
(8-vi-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 223 in which one or multiple bases are deleted, substituted, or added at a position(s) except positions 12, 18, 28, and 34 in SEQ ID NO: 223, wherein the positions in the base sequence in the central region corresponding to the bases shown as n at positions 12 and 28 in SEQ ID NO: 223 are the Ds, and the positions in the base sequence in the central region corresponding to the bases shown as n at positions 18 and 34 in SEQ ID NO: 223 are the Pa' or the Px;
(8-vii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 224, and the bases shown as n at positions 12 and 28 in the base sequence of SEQ ID NO: 224 are the Ds, and the bases shown as n at position 18 and 38 in the base sequence of SEQ ID NO: 224 are the Pa' or the Px;
(8-vii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 224 in which one or multiple bases are deleted, substituted, or added at a position(s) except positions 12, 18, 28, and 38 in SEQ ID NO: 224, wherein the positions in the base sequence in the central region corresponding to the bases shown as n at positions 12 and 28 in SEQ ID NO: 224 are the Ds, and the positions in the base sequence in the central region corresponding to the bases shown as n at positions 18 and 38 in SEQ ID NO: 224 are the Pa' or the Px;
(8-viii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 225, and the bases shown as n at positions 12 and 28 in the base sequence of SEQ ID NO: 225 are the Ds, and the bases shown as n at position 18, 34, and 38 in the base sequence of SEQ ID NO: 225 are the Pa' or the Px;
(8-viii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 225 in which one or multiple bases are deleted, substituted, or added at a position(s) except positions 12, 18, 28, 34, and 38 in SEQ ID NO: 225, wherein the positions in the base sequence in the central region corresponding to the bases shown as n at positions 12 and 28 in SEQ ID NO: 225 are the Ds, and the positions in the base sequence in the central region corresponding to the bases shown as n at positions 18, 34, and 38 in SEQ ID NO: 225 are the Pa' or the Px;
(8-ix-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 226, and the bases shown as n at positions 12 and 28 in the base sequence of SEQ ID NO: 226 are the Ds, and the bases shown as n at position 16, 18, 34 and 38 in the base sequence of SEQ ID NO: 226 are the Pa' or the Px;
(8-ix-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 226 in which one or multiple bases are deleted, substituted, or added at a position(s) except positions 12, 16, 18, 28, 34, and 38 in SEQ ID NO: 226, wherein the positions in the base sequence in the central region corresponding to the bases shown as n at positions 12 and 28 in SEQ ID NO: 226 are the Ds, and the positions in the base sequence in the central region corresponding to the bases shown as n at positions 16, 18, 34, and 38 in SEQ ID NO: 226 are the Pa' or the Px;
(8-x-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 229, and the bases shown as n at positions 11 and 27 in the base sequence of SEQ ID NO: 229 are the Ds, and the base shown as n at position 17 in the base sequence of SEQ ID NO: 229 is the Pa' or the Px;
(8-x-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 229 in which one or multiple bases are deleted, substituted, or added at a position(s) except positions 11, 17, and 27 in SEQ ID NO: 229, wherein the positions in the base sequence in the central region corresponding to the bases shown as n at positions 11 and 27 in SEQ ID NO: 229 are the Ds, and the position in the base sequence in the central region corresponding to the base shown as n at position 17 in SEQ ID NO: 229 is the Pa' or the Px;
(8-xi-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 230, and the bases shown as n at positions 11 and 27 in the base sequence of SEQ ID NO: 230 are the Ds, and the base shown as n at position 15 and 17 in the base sequence of SEQ ID NO: 230 are the Pa' or the Px;
(8-xi-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 230 in which one or multiple bases are deleted, substituted, or added at a position(s) except positions 11, 17, and 27 in SEQ ID NO: 230, wherein the positions in the base sequence in the central region corresponding to the bases shown as n at positions 11 and 27 in SEQ ID NO: 230 are the Ds, and the positions in the base sequence in the central region corresponding to the bases shown as n at positions 15 and 17 in SEQ ID NO: 230 are the Pa' or the Px;
(8-xii-a) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises the base sequence of SEQ ID NO: 231, and the bases shown as n at positions 8 and 24 in the base sequence of SEQ ID NO: 231 are the Ds, and the base shown as n at position 14 in the base sequence of SEQ ID NO: 231 is the Pa' or the Px;
(8-xii-b) a nucleic acid aptamer comprising a 5' region, a central region, and a 3' region from the 5' end in this order, wherein the 5' region and 3' region comprise a pair of base sequences which are complementary to each other and are capable of forming a stem structure, and wherein the central region comprises a base sequence derived from SEQ ID NO: 231 in which one or multiple bases are deleted, substituted, or added at a position(s) except positions 8, 14, and 24 in SEQ ID NO: 231, wherein the positions in the base sequence in the central region corresponding to the bases shown as n at positions 8 and 24 in SEQ ID NO: 231 are the Ds, and the position in the base sequence in the central region corresponding to the base shown as n at position 14 in SEQ ID NO: 231 is the Pa' or the Px; 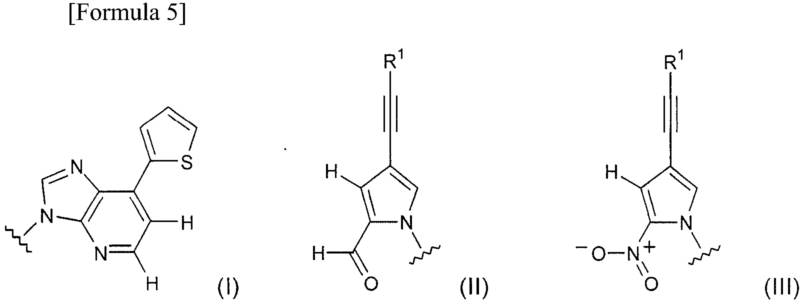
[wherein R¹ respectively and independently represents a hydrogen atom or an optionally substituted hydrocarbon group having one or more carbon atoms].

40. The nucleic acid aptamer of claim 39, wherein the pair of base sequences comprise a non-complementary base pair, and/or an insertion sequence and/or a deletion of one or more bases.

41. The nucleic acid aptamer of claim 40, wherein the non-complementary base pair comprises a G-T base pair.

42. The nucleic acid aptamer of claim 40 or 41, wherein the insertion sequence forms a bulge structure or a bulge-out structure.

43. The nucleic acid aptamer of claim 42, wherein the bulge structure or bulge-out structure comprises an unnatural base.

44. The nucleic acid aptamer of claim 43, wherein the unnatural base is the Ds, the Pa', and/or the Px.

45. The nucleic acid aptamer of claim 39, wherein the pair of base sequences consist of SEQ ID NOs: 51 and 52; SEQ ID NOs: 53 and 54; SEQ ID NOs: 55 and 56; SEQ ID NOs: 57 and 58 (wherein the base shown as n at position 9 in SEQ ID NO: 57 is the Ds); SEQ ID NOs: 59 and 60 (GGTCATGTGACAAGCAGC); SEQ ID NOs: 61 and 62; SEQ ID NOs: 63 and 64; SEQ ID NOs: 65 and 66; SEQ ID NOs: 67 and 68; SEQ ID NOs: 69 and 70; SEQ ID NOs: 71 and 72; SEQ ID NOs: 73 and 74 (wherein the base shown as n at position 3 in SEQ ID NO: 74 is the Ds); SEQ ID NO: 75 and SEQ ID NO: 76; SEQ ID NOs: 77 and 78; SEQ ID NOs: 79 and 80; SEQ ID NOs: 81 and 82; SEQ ID NOs: 79 and 83 (wherein the base shown as n at position 7 in SEQ ID NO: 83 is the Ds); SEQ ID NOs: 84 and 85; SEQ ID NOs: 86 and 87; SEQ ID NO: 232 (GCGCTTT) and SEQ ID NO: 233 (AAAGCGC); SEQ ID NO: 234 (GCGTT) and SEQ ID NO: 235 (AACGC); SEQ ID NOs: 239 and 240; or SEQ ID NOs: 241 and 242.

46. The nucleic acid aptamer of claim 39, comprising a mini-hairpin sequence at the 5' end and/or 3' end thereof.

47. The nucleic acid aptamer of claim 39, wherein the Interferon gamma (IFN-γ) is glycosylated.

48. The nucleic acid aptamer of claim 39, wherein the R¹ represents any one of the following general formulas (IV) to (XXV): 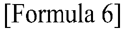 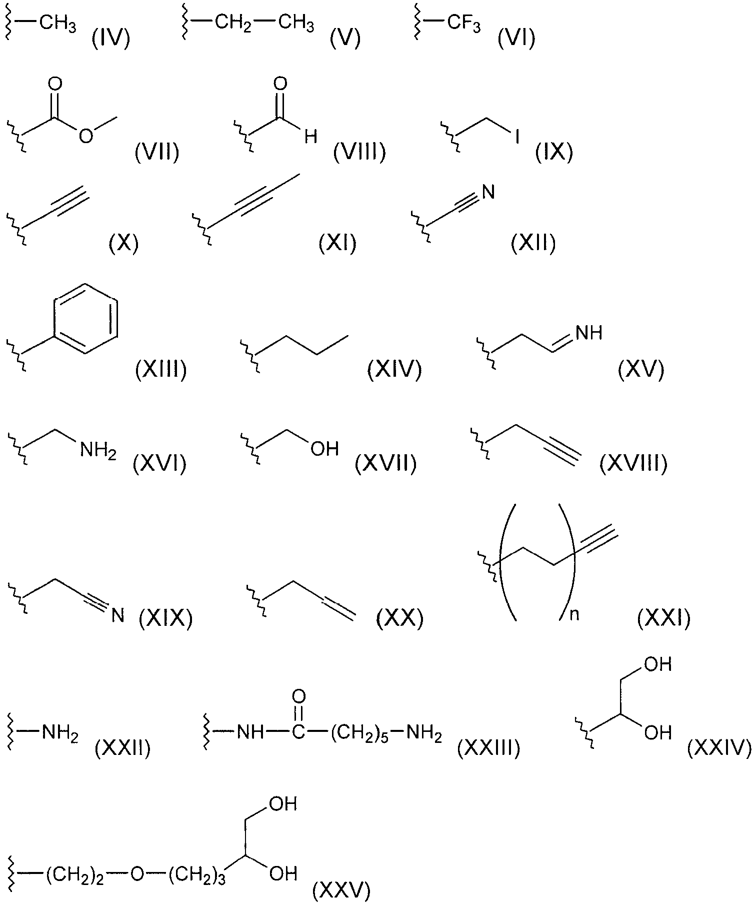 wherein the wave line represents a bond with alkyne, and n represents an integer of one or more.

49. A kit for detecting Interferon gamma (IFN-γ), C-reactive protein (CRP), Thrombin, Interleukin-6 (IL-6), or Interleukin-8 (IL-8), or HMGB1, comprising the nucleic acid aptamer of claim 39.

50. A method for detecting Interferon gamma (IFN-γ), C-reactive protein (CRP), Thrombin, Interleukin-6 (IL-6), Interleukin-8 (IL-8), or HMGB1, comprising:
a detection step of detecting Interferon gamma (IFN-γ), C-reactive protein (CRP), Thrombin, Interleukin-6 (IL-6), Interleukin-8 (IL-8), or HMGB1 with the nucleic acid aptamer of claim 39.

51. The kit of claim 49 or the method of claim 50, for detecting an inflammation or an inflammatory disease, or evaluating an inflammatory level thereof.

52. The kit or method of claim 51, comprising the nucleic acid aptamer of the item (3) or comprising use of the nucleic acid aptamer of the item (3), wherein the inflammatory disease is an infection, collagen disease, cardiovascular disease, or tumor, or wherein the inflammation is associated with tissue damage, necrosis, or surgery.

53. The kit of claim 49 comprising the nucleic acid aptamer of the item (1), (2), or (8), or the method of claim 50 comprising using the nucleic acid aptamer of the item (1), (2), or (8), for evaluating the activation level of T cells and/or NK cells in an immunotherapy, or for detecting an infection of Mycobacterium tuberculosis.

54. A pharmaceutical composition comprising the nucleic acid aptamer of claim 39.

55. The pharmaceutical composition of claim 54 for anti-inflammation.

56. The pharmaceutical composition of claim 54 for use in apheresis, comprising the nucleic acid aptamer of the item (1), (2), or (8) as an active ingredient.

57. The pharmaceutical composition of claim 54 for suppressing or inhibiting blood clotting, comprising the nucleic acid aptamer of the item (4) as an active ingredient.

58. The pharmaceutical composition of claim 54 for treating rheumatism, comprising the nucleic acid aptamer of the item (5) as an active ingredient.

59. The pharmaceutical composition of claim 54 for treating cancer, comprising the nucleic acid aptamer of the item (6) as an active ingredient.

60. A method of treating a disease, comprising a step of administering the nucleic acid aptamer of claim 39 to a subject.

61. The method of claim 60, which is for treating an inflammatory disease.

62. The method of claim 60, comprising a step of removing Interferon gamma (IFN-γ) from blood by apheresis using the nucleic acid aptamer of the item (1), (2), or (8).

63. The method of claim 60, comprising administering the nucleic acid aptamer of the item (4) to the subject for suppressing or inhibiting blood clotting.

64. The method of claim 60 for treating rheumatism, comprising administering the nucleic acid aptamer of the item (5) to the subject.

65. The method of claim 60 for treating cancer, comprising administering the nucleic acid aptamer of the item (6) to the subject.

66. The method of claim 60 for treating an inflammatory disease, an autoimmune disease, cancer, a cardiovascular disease, or a neurological disease, comprising administering the nucleic acid aptamer of the item (7) to the subject.
